(19) Europäisches Patentamt
European Patent Office
Office européen des brevets

(11) Publication number: **0 150 966**
**B1**

(12) # EUROPEAN PATENT SPECIFICATION

(45) Date of publication of patent specification: **12.07.89**

(21) Application number: **85300381.2**

(22) Date of filing: **21.01.85**

(51) Int. Cl.⁴: **C 07 D 493/04,**
C 07 D 491/04,
C 07 D 495/04,
C 07 D 311/92,
C 07 D 311/94,
A 61 K 31/335, A 61 K 31/47

(54) **Benzopyran derivatives, compositions containing them and processes for their preparation.**

(30) Priority: **26.01.84 GB 8402047**
**01.02.84 GB 8402577**

(43) Date of publication of application:
**07.08.85 Bulletin 85/32**

(45) Publication of the grant of the patent:
**12.07.89 Bulletin 89/28**

(84) Designated Contracting States:
**AT BE CH DE FR GB IT LI LU NL SE**

(56) References cited:
**GB-A-1 389 827**
**GB-A-2 022 078**
**GB-A-2 028 316**
**GB-A-2 035 312**
**LU-A- 57 806**

**The file contains technical information submitted after the application was filed and not included in this specification**

(73) Proprietor: **FISONS plc**
**Fison House Princes Street**
**Ipswich Suffolk IP1 1QH (GB)**

(72) Inventor: **Dicker, Ian Douglas**
**36 Main Street Normanton on Soar**
**Loughborough Leicestershire (GB)**
Inventor: **Gould, Kenneth John**
**3A Turvey Lane**
**Long Whatton Leicestershire (GB)**
Inventor: **Suschitzky, John Louis**
**73 Kirkstone Drive**
**Loughborough Leicestershire (GB)**

(74) Representative: **Craig, Christopher Bradberry et al**
**Fisons plc 12 Derby Road**
**Loughborough Leicestershire LE11 0BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 150 966 B1**

**Description**

This invention relates to new benzopyrans, processes for their production and compositions containing them.

Benzodipyran dicarboxylic acid derivative having anti-allergic properties are disclosed in UK Patent No 1230087 and Luxembourg Patent No 57806. Certain benzodipyran derivatives substituted by tetrazolyl groups and having anti-allergic properties are disclosed in UK Patent No. 1389827. Various pyranoquinoline dicarboxylic acids which also have anti-allergic activity are disclosed in UK Patent Nos. 2022078B and 2035312B. UK Patent Application No 2028316A discloses a number of thiopyranobenzopyrans having similar utility. We have now found a new group of benzopyran derivatives which have advantageous properties over compounds specifically disclosed in the above patent specifications.

According to the invention there are provided benzopyrans of formula I,

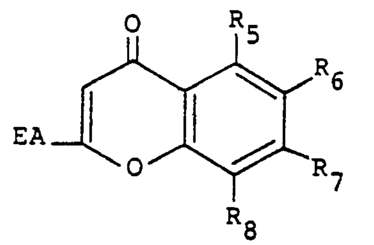

I

wherein an adjacent pair of $R_5$, $R_6$, $R_7$ and $R_8$ represent the chain $—X—CR_{12}R_{13}—CR_{14}R_{15}—Y—$ in which the chain is substituted by $—A_1E_1$,

X represents $CR_{10}R_{11}$ or a single bond,

Y represents O, S, $NR_{20}$ or $CH_2$, and (a) $R_{14}$ and $R_{20}$ together form a single bond and optionally $R_{10}$ and $R_{12}$ together form a single bond, or (b) $R_{12}$ and $R_{14}$ together form a single bond, or (c) $R_{10}$, $R_{12}$ and $R_{14}$ each represent hydrogen; and

the remainder of $R_{10}$, $R_{11}$, $R_{12}$, $R_{13}$, $R_{14}$ and $R_{15}$, which may be the same or different, independently represent hydrogen, alkyl C1 to 6, $NR_{21}COR_{22}$, CN or $C_nH_xF_{(2n+1-x)}$; in addition, $R_{10}$ and $R_{11}$, together with the carbon atom to which they are attached, may represent C=O or $C=N—OR_{23}$; also $R_{13}$ and $R_{15}$ may form the chain —CH=CH—CH=CH— which is substituted by $A_1E_1$,

A and $A_1$, which may be the same or different, represent a single bond, $(CH_2)_m$ or phenylene,

n represents an integer from 1 to 4 inclusive,

x represents 0 or an integer from 1 to 2n inclusive,

m represents an integer from 1 to 4 inclusive,

when $R_{20}$ does not form a single bond with $R_{14}$, $R_{20}$ may represent hydrogen, alkyl C1 to 6 or alkyl C1 to 6 substituted by phenyl,

$R_{21}$, $R_{22}$, and $R_{23}$, which may be the same or different, independently represent hydrogen or alkyl C1 to 6;

the remainder of $R_5$, $R_6$, $R_7$ and $R_8$, which may be the same or different, each independently represent hydrogen, hydroxy or alkyl C1 to 6,

E and $E_1$, which may be the same or different, independently represent —COOH, or 5H-tetrazolyl, provided that when the chain $—X—CR_{12}R_{13}=CR_{14}R_{15}—Y$

i) represents a chain $—CO—CR_{13}=CR_{15}—Y—$ in which Y is O or S and A and $A_1$ each represent a single bond, then Y is bonded to $R_7$, $R_5$ and $R_{13}$ each represent hydrogen, $R_8$ represents propyl and $R_{15}$ represents $E_1$, in which $E_1$, instead of being as defined above, represents 5H-tetrazolyl or $CONR_{24}R_{25}$, wherein $R_{24}$ and $R_{25}$, which may be the same or different, represent hydrogen or alkyl C1 to 6;

ii) represents a chain $—CO—CR_{13}=CR_{15}—NR_{20}—$, in which one of $R_{13}$ and $R_{15}$ represents $E_1$, then $E_1$, instead of being as defined above, represents hydrogen, and

iii) represents a chain $—CR_{11}=CR_{13}—CR_{15}=N—$, then at least one of $R_{11}$, $R_{13}$ or $R_{15}$, which may be the same or different, represents $NR_{21}COR_{22}$, CN or $C_nH_xF_{(2n+1-x)}$, and pharmaceutically acceptable derivatives thereof as hereinafter defined.

According to the invention there is also provided a process for the production of a benzopyran of formula I, or a pharmaceutically acceptable derivative thereof as hereinafter defined, which comprises

(a) cyclising a compound of formula II, or a suitable derivative thereof,

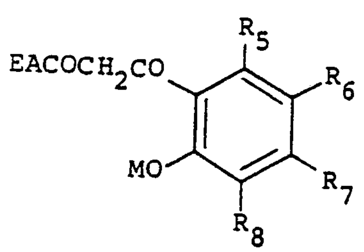

II

in which M represents a hydrogen atom or an alkali metal,

and E, A, $R_5$, $R_6$, $R_7$ and $R_8$ are as defined above,

(b) producing a compound of formula I, in which at least one of E and $E_1$ represents —COOH, by hydrolysing a compound of formula I in which the corresponding E, or $E_1$ represents a group hydrolysable to a —COOH group,

(c) producing a compound of formula I, in which at least one of E and $E_1$ represents 5-tetrazolyl, by reacting a compound of formula I, in which the corresponding E or $E_1$ represents —CN, with an azide in a solvent which is inert under the reaction conditions,

(d) producing a compound of formula I, in which $E_1$ represents —$CONR_{24}R_{25}$, by reacting a compound of formula I in which $E_1$ represents —COL, wherein L is a good leaving group,

with a compound of formula III,

$$R_{24}R_{25}NH \qquad III$$

in which $R_{24}$ and $R_{25}$ are as defined above, or

(e) producing a compound of formula I, in which at least one of $R_{10}$, $R_{11}$, $R_{12}$, $R_{13}$, $R_{14}$ and $R_{15}$ represents $C_nH_xF_{(2n+1-x)}$,

by reacting a compound of formula I, or a derivative thereof, in which the corresponding $R_{10}$, $R_{11}$, $R_{12}$, $R_{13}$, $R_{14}$ or $R_{15}$ represents $L_1$,

wherein $L_1$ is a halogen atom,

with a compound $C_nH_xF_{(2n+1-x)}L_2$, in which n and x are as defined above and $L_2$ represents chlorine, bromine or iodine,

and if necessary or desired converting the compound of formula I to a pharmaceutically acceptable derivative thereof as hereinafter defined or vice versa.

The cyclisation of process (a) may be carried out by heating, or under basic or neutral conditions. It is however preferred to carry out the cyclisation in the presence of an acid, e.g. gaseous or aqueous HCl, and in a solvent which is inert under the reaction conditions, e.g. ethanol or dioxan. The reaction may be carried out at from about 20° to 150°C. When E in the compound of formula I represents —COOH, E in the compound of formula II preferably represents a carboxylic acid ester, e.g. an ethyl or methyl ester.

Groups hydrolysable to a —COOH group include carboxylic acid amides, nitriles, carboxylic halides and preferably carboxylic acid esters. The conditions of the hydrolysis depend on the nature of the group, but may be carried out using conventional techniques, for example under mildly basic conditions, e.g. using sodium carbonate, sodium hydroxide, sodium bicarbonate; or under acidic conditions, e.g. hydrogen bromide in acetic acid. For carboxylic acid esters, we prefer to carry out the hydrolysis under basic conditions, e.g. using sodium hydroxide in an alkanol, e.g. methanol. The hydrolysis may be carried out at a temperature of from about −5° to 120°C depending on the compound and reagents used.

Suitable solvents which are inert under the reaction conditions of reaction (c) include those in which both reagents are soluble, e.g. N,N-dimethylformamide. Other solvents which may be mentioned include dimethylsulphoxide, tetrahydrofuran, diethylglycol and ethyl methyl glycol. The reaction is preferably carried out at a temperature of from about 20° to 130°C for from about 1 to 20 hours. The azide used in the reaction is preferably ammonium or an alkali metal azide, e.g. sodium or lithium azide, but other azides, e.g. aluminium azide or the azides of nitrogen containing bases, e.g. mono-, di-, tri-, and tetra-methylammonium, anilinium, morpholinium and piperadinium azides, may also be used if desired. Where an azide other than that of an alkali metal is used, this azide may be prepared in the reaction mixture by double decomposition. The reaction may, if desired, be carried out in the presence of an electron acceptor, e.g. aluminium chloride, boron trifluoride, ethyl sulphonic acid or benzene sulphonic acid. As an alternative to the reaction conditions set out above the reaction may be carried out using hydrazoic acid (hydrogen azide) at a temperature of from about 20° to 150° in a suitable solvent, under greater than atmospheric pressure. When an azide other than hydrazoic acid is used, e.g. sodium azide, the product of the reaction will be the corresponding salt. This salt may be readily converted to the free acid by treatment with strong acid, e.g. hydrochloric acid.

The reaction of process (d) is preferably carried out in a solvent which is inert to the reaction conditions, e.g. diethyl ether, tetrahydrofuran, halogenated hydrocarbons such as dichloroethane and dichloromethane, or 1,4-dioxan. Good leaving groups that L may represent include halide, especially chloride, and $OCOCF_3$. The reaction is preferably carried out at a temperature of from about 0° to 120°C for from about 0.5 hours to 10 hours. The reaction is preferably carried out under anhydrous conditions under an inert atmosphere of, for example, nitrogen or argon. The reaction is preferably carried out in the presence of a proton acceptor, for example an excess of the compound of formula III or a non-nucleophilic proton acceptor, e.g. pyridine.

Process (e) may be carried out in a solvent which is inert under the reaction conditions, e.g. hexamethylphosphoric triamide or dimethylformamide. The reaction may be carried out on elevated temperature of from about 50° to 200°C, and may, if desired, be carried out in an inert atmosphere and under pressure. The reaction is preferably carried out in the presence of a copper catalyst (Ullmann reaction). We prefer the groups $L_1$ and $L_2$, which may be the same or different, to be selected from bromine and iodine.

Compounds of formula II and derivatives thereof, may be prepared by reacting a carbanion derived from the corresponding compound of formula IV, or a derivative thereof,

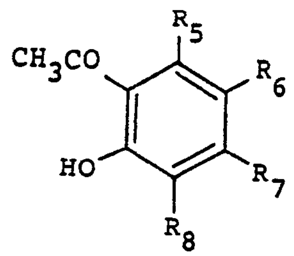

IV

in which $R_5$, $R_6$, $R_7$ and $R_8$ are as first defined above,

with a compound of formula V,

$$ECOL_3 \qquad V$$

in which $L_3$ represents a good leaving group and E is as defined above.

The reaction is preferably carried out in a solvent which is inert to the reaction conditions, e.g. an alkanol such as ethanol, or dimethylformamide. Carbanions of the compound of formula IV may be formed by treating the compound of formula IV with a strong, non-nucleophilic base, for example a metal alkoxide such as potassium t-butoxide or sodium ethoxide, or a metal hydride, for example sodium hydride. Preferably an excess, for example four equivalents of base for each equivalent of the compound of formula IV is used. Good leaving groups that $L_3$ may represent include alkoxy, e.g. ethoxy. The reaction is preferably carried out at a temperature of from about 20° to 120° under an inert atmosphere and under anhydrous conditions.

The compounds of formula I in which at least one of E and $E_1$ represents a carboxylic ester may be made by processes analogous to those described under process (a) and, where appropriate, process (e).

The starting materials for process (c), i.e. compounds of formula I in which at least one of E and $E_1$ and $E_2$ represents —CN, may be prepared by reacting the compound of formula I in which the corresponding E or $E_1$ represents —$CONH_2$ with a dehydrating reagent. The reaction is preferably carried out using at least two molar equivalents of a dehydrating agent, e.g. $POCl_3$, per —$CONH_2$ group. The reaction may, if desired, be carried out in the presence of a proton acceptor, e.g. triethylamine. The reaction may be carried out in the presence of a solvent, e.g. N,N-dimethylformamide, dimethylsulphoxide, pyridine, benzene or hexamethyl phosphoric triamide, or an excess of the dehydrating agent may be used as the reaction medium. The reaction may be carried out at a temperature of from about 0° to 200°C depending on the dehydrating agent used. When phosphorous oxychloride is used a temperature of from about 0° to 100°C is preferred.

The compounds of formula I in which E or $E_1$ represents $CONH_2$ may be made in a conventional manner known *per se* from the corresponding carboxylic acid ester, e.g. by reaction of the ester with ammonia in an alkanol or dialkyl formamide solvent at a temperature of 0° to 120°C.

When L represents halide, the compounds of formula I in which E, $E_1$ or $E_2$ represents COL may be prepared by reacting the carboxylic acid with, for example, the appropriate thionyl halide or phosphoroyloxyhalide, under conventional conditions known *per se*. When L represents $OCOCF_3$, the mixed anhydride may be prepared from the corresponding carboxylic acid derivatives by conventional methods, e.g. by reaction with trifluoroacetic anhydride.

The compounds of formula III, IV and V are either known compounds or may be made from known compounds using for example techniques such as those described in the Examples.

The compounds of formula I and the intermediates therefor may be recovered from their reaction mixtures using conventional methods.

The process described above may produce the compound of formula I or a derivative thereof. It is also within the scope of this invention to treat any derivative so produced to liberate the free compound of formula I, or to convert one derivative into another.

Pharmaceutically acceptable derivatives of the compounds of formula I are pharmaceutically acceptable salts and, when any one of E or $E_1$ represents a carboxylic acid group, pharmaceutically acceptable esters or amides of the carboxylic acid groups.

Pharmaceutically acceptable salts of the compounds of formula I include ammonium, alkali metal (e.g. sodium, potassium and lithium) and alkaline earth metal (e.g. calcium or magnesium) salts, and salts with suitable organic bases, e.g. salts with hydroxylamine, lower alkylamines such as methylamine or ethylamine, with substituted lower alkylamines, e.g. hydroxy substituted alkylamines such as tris(hydroxymethyl)methylamine, or with simple monocyclic nitrogen heterocyclic compounds, e.g. piperidine or morpholine. Suitable esters include simple lower alkyl esters, e.g. the ethyl ester, esters derived from alcohols containing basic groups, e.g. di-lower alkyl amino substituted alkanols such as the beta-(diethylamino)ethyl ester, and acyloxy alkyl esters, e.g. a lower acyloxy-lower alkyl ester such as the pivaloyloxymethyl ester, or a bis-ester derived from a di-hydroxy compound, e.g. a di(hydroxy-lower alkyl)ester, e.g.

the bis-2-oxapropan-1,3-diyl ester. The pharmaceutically acceptable acid addition salts of the basic esters, e.g. the hydrochloride, the hydrobromide, the oxalate, the maleate or the fumarate salts may also be used. The esters (when a —COOH group is present) may be made by conventional techniques, e.g. esterification or transesterification. The amides (when a —COOH group is present) may be, for example, unsubstituted or mono- or di- C 1 to 6 alkyl amides and may be made by conventional techniques, e.g. reaction of an ester of the corresponding acid with ammonia or an appropriate amine.

The compounds of formula I and pharmaceutically acceptable salts, and, when E or $E_1$ is a —COOH group, pharmaceutically acceptable esters and amides thereof are useful because they possess pharmacological activity in animals; in particular they are useful because they inhibit the release and/or action of pharmacological mediators which result from the *in vivo* combination of certain types of antibody and specific antigen, e.g. the combination of reaginic antibody with specific antigen (see Example 27 of British Patent Specification No 1,292,601). The new compounds have also been found to interfere with reflex pathways in experimental animals and man, and in particular those reflexes associated with lung function. In man, both subjective and objective changes which result from the inhalation of specific antigen by sensitised subjects are inhibited by prior administration of the new compounds. Thus the new compounds are indicated for use in the treatment of reversible airway obstruction and/or to prevent the secretion of excess mucus. The new compounds are thus indicated for the treatment of allergic asthma, so-called "intrinsic" asthma (in which no sensitivity to extrinsic antigen can be demonstrated), bronchitis, coughs and the nasal and bronchial obstructions associated with common colds. The new compounds may also be of value in the treatment of other conditions in which antigen-antibody reactions or excess mucus secretion are responsible for, or are an adjunct to, disease, for example, hay fever; certain eye conditions, e.g. trachoma; alimentary allergy, e.g. urticaria and atopic eczema; and gastrointestinal conditions, for example gastrointestinal allergy, especially in children, e.g. milk allergy, or ulcerative colitis.

For the above mentioned uses the doses administered will, of course, vary with the compound employed, the mode of administration and the treatment desired. However, in general, satisfactory results are obtained when the compounds are administered at the dosage of from 0.001 to 50 mg per kg of animal body weight in the test set out in Exmaple 27 of British Patent Specification No 1,292,601. For man the indicated total daily dosage is in the range of from 0.01 mg to 1,000 mg, preferably from 0.01 mg to 200 mg and more preferably from 1 mg to 60 mg, which may be administered in divided doses from 1 to 6 times a day or in sustained release form. Thus unit dosage forms suitable for administration (by inhalation or oesophageally) comprise from 0.01 mg to 50 mg, preferably 0.01 mg to 20 mg, and more preferably from 0.01 mg to 10 mg, of the compound preferably admixed with a solid or liquid pharmaceutically acceptable diluent, carrier or adjuvant.

The compounds of formula I, and pharmaceutically acceptable salts, and, when E or $E_1$ is a —COOH group, pharmaceutically acceptable esters and amides thereof, have the advantage that they are more efficacious in certain pharmacological models, or are longer acting than compounds of similar structure to the compounds of formula I. Furthermore the compounds of formula I, and pharmaceutically acceptable salts, and, when E or $E_1$ is a —COOH group, pharmaceutically acceptable esters and amides thereof, are advantageous in that they are more efficacious in interfering with reflex pathways and in inhibiting the secretion of mucus than are compounds of similar structure to the compounds of formula I.

According to the invention there is also provided the use of compounds of formula I to make a pharmaceutical formulation for the treatment of asthma.

$R_5$, $R_6$, $R_7$, $R_8$, $R_{10}$, $R_{11}$, $R_{12}$, $R_{13}$, $R_{14}$, $R_{15}$, $R_{20}$, $R_{21}$, $R_{22}$, $R_{23}$, $R_{24}$ and $R_{25}$, when they represent alkyl C1 to 6, may represent for example methyl, ethyl or propyl.

The chain $—X—CR_{12}R_{13}—CR_{14}R_{15}—Y—$ may be formed between any adjacent pair of $R_5$, $R_6$, $R_7$ and $R_8$. However, we prefer those compounds in which the chain is formed between $R_6$ and $R_7$, particularly those compounds in which —X— is bonded to $R_6$ and —Y— is bonded to $R_7$.

Chains $—X—CR_{12}R_{13}—CR_{14}R_{15}—Y—$ that may be specifically mentioned include:

$—COCH{=}C(E_1)—O—$ in which $E_1$ represents 5-tetrazolyl or $CONR_{24}R_{25}$;

$COCH{=}C(C_6H_4COOH)—O—$;

$COCH{=}C((CH_2)_mCOOH)—O—$;

$COCR_{13}{=}CR_{15}—O—$, in which $R_{13}$ and $R_{15}$ together form the chain $—CH{=}CH—CH{=}CH—$, the chain $—CH{=}CH—CH{=}CH—$ being substituted by COOH;

$—CH_2CH_2CH(COOH)—O—$;

$—CH_2CH(COOH)CH_2CH_2—$;

$—CH_2CH_2CH(COOH)CH_2—$;

$—C(C_nH_xF_{(2n+1-x)}){=}CH—C(COOH){=}N—$;

$—C(NR_{21}COR_{22}){=}CH—C(COOH){=}N—$;

$—C({=}NOR_{23})—CH{=}C(COOH)—NR_{20}—$;

$—CO—CH{=}CH—NR_{20}—$;

$—C(CN){=}CH—C(COOH){=}N—$;

$—CH{=}C(COOH)—CO—S—$; and

$—CR_{13}{=}CR_{15}—Y—$, in which one of $R_{13}$ and $R_{15}$ represents —COOH and the other represents hydrogen and Y represents O, S or $NR_{20}$.

We prefer compounds of formula I in which $R_5$ represents hydrogen or hydroxy.

We prefer compounds of formula I in which $R_8$ represents alkyl C1 to 6, in particular propyl.

Particular groups that $C_nH_xF_{(2n+1-x)}$ may represent include those in which x is zero, for example $CF_3$, $C_2F_5$ and $C_3F_7$. Other groups that may be mentioned include $CHF_2$ and $CH_2CF_3$.

We particularly prefer compounds of formula I in which X represents C=O.

We prefer compounds of formula I in which $NR_{21}COR_{22}$ represents $NHCOR_{22}$.

We prefer compounds of formula I in which X represents $C=N—OR_{23}$.

When $R_{13}$ and $R_{15}$ form the chain —CH=CH—CH=CH— which is substituted by $A_1E_1$, the corresponding vicinal pair of $R_{12}$ and $R_{14}$ preferably represents a single bond, such that the chain —CH=CH—CH=CH— and the carbon atoms to which it is attached forms a six-membered carbocyclic aromatic ring. We further prefer the chain —CH=CH—CH=CH— to be substituted by a group $E_1$, preferably COOH.

We prefer compounds in which A represents a single bond.

We prefer compounds of formula I in which E represents —COOH.

We prefer compounds of formula I which bear a single group $E_1$. We particularly prefer compounds in which one of $R_{13}$ or $R_{15}$ represents $E_1$, particularly —COOH.

As a preferred group of compounds we provide the compounds of formula I in which

an adjacent pair of $R_5$, $R_6$, $R_7$ and $R_8$ represents the chain $—CO—CR_{12}R_{13}CR_{14}R_{15}—O—$,

$R_{12}$ and $R_{14}$ together form a single bond,

one of $R_{13}$ and $R_{15}$ represents $A_1E_1$ and the other represents hydrogen, or $R_{13}$ and $R_{15}$ together form a chain —CH=CH—CH=CH—, the chain being substituted by $A_1E_1$,

A represents a single bond,

$A_1$ represents a single bond, phenylene or $(CH_2)_m$ and E, $E_1$, the remainder of $R_5$, $R_6$, $R_7$, $R_8$, m and the provisos are as defined above.

As a first specific group of compounds, we provide compounds of formula I in which

$R_6$ and $R_7$ together form the chain $—COCH=CE_1—O—$, in which —O— is attached to $R_7$.

$R_5$ represents hydrogen,

$R_8$ represents propyl,

A represents a single bond,

E represents —COOH or a 5H-tetrazolyl group,

$E_1$ represents $—CONR_{24}R_{25}$ or a 5H-tetrazolyl group

$R_{24}$ and $R_{25}$, which may be the same or different represent hydrogen or alkyl C1 to 6.

As a second specific group of compounds we provide compounds of formula I in which

an adjacent pair of $R_5$, $R_6$, $R_7$, and $R_8$ represents the chain $—COCH=C(A_1E_1)—O—$,

A represents a single bond,

$A_1$ represents phenylene,

E and $E_1$ each represent —COOH,

the remainder of $R_5$, $R_6$, $R_7$ and $R_8$ represent hydrogen or alkyl C1 to 6.

As a further group of preferred compounds, we provide the compounds of formula I in which

an adjacent pair of $R_5$, $R_6$, $R_7$ and $R_8$ represents the chain $—CHR_{10}—CHR_{12}—CHR_{14}—Y—$,

Y represents O or $—CHR_{16}$,

one of $R_{10}$, $R_{12}$, $R_{14}$ and $R_{16}$ represents —COOH and the remainder represent hydrogen,

A represents a single bond,

E represents —COOH,

the remainder of $R_5$, $R_6$, $R_7$ and $R_8$ which may be the same or different, independently represent hydrogen or alkyl C1 to 6.

As a yet further group of preferred compounds, we provide the compounds of formula I in which

an adjacent pair of $R_5$, $R_6$, $R_7$ and $R_8$ represents a chain $—CR_{10}R_{11}—CR_{12}R_{13}—CR_{14}R_{15}—NR_{20}—$,

in which the chain is substituted by $—A_1E_1$, wherein either (a) $R_{10}$ and $R_{12}$ together form a single bond and $R_{14}$ and $R_{20}$ together form a single bond, or (b) $R_{12}$ and $R_{14}$ together form a single bond,

the remainder of $R_{10}$, $R_{11}$, $R_{12}$, $R_{13}$, $R_{14}$ and $R_{15}$, which may be the same or different independently represent hydrogen, alkyl C1 to 6, $NHCOR_{22}$, CN or $C_nH_xF_{(2n+1-x)}$; in addition $R_{10}$ and $R_{11}$, together with the carbon atom to which they are attached, may represent C=O or $C=NOR_{23}$,

A and $A_1$ each represent a single bond,

when $R_{20}$ does not form a single bond with $R_{14}$, $R_{20}$ may represent hydrogen, alkyl C1 to 6 or alkyl C1 to 6 substituted by phenyl,

n, x, $R_{21}$, $R_{22}$ are as first defined above,

the remainder of $R_5$, $R_6$, $R_7$ and $R_8$, which may be the same or different, each independently represent hydrogen or hydroxy,

E and $E_1$ represent —COOH, save that when the chain represents $—COCR_{13}=CR_{15}—NR_{20}$, in which one of $R_{13}$ and $R_{15}$ represents $E_1$ and the other represents hydrogen or alkyl C1 to 6, then $E_1$ represents hydrogen, and

provided that when the chain represents $—CR_{11}=CR_{13}—CR_{15}=N—$, then at least one of $R_{11}$, $R_{13}$ or $R_{15}$, which may be the same or different represents $NHCOR_{22}$, CN or $C_nH_xF_{(n+1-x)}$.

As a third specific group of compounds, we provide compounds of formula I in which

$R_6$ and $R_7$ together represent the chain $—C(C_nH_xF_{(n+1-x)})=CR_{13}—CR_{15}=N—$,

one of $R_{13}$ and $R_{15}$ is —COOH and the other is hydrogen,

$R_5$ represents hydrogen
$R_8$ represents alkyl C1 to 6,
A represents a single bond,
E represents —COOH,
n and x are as first defined above.

As a group of preferred compounds we also provide the compounds of formula I in which an adjacent pair of $R_5$, $R_6$, $R_7$ and $R_8$ represents $CR_{13}=CR_{15}-Y-$,
wherein one of $R_{13}$ and $R_{15}$ represents COOH and the other represents hydrogen,
Y represents O, S or $NR_{20}$,
$R_{20}$ represents hydrogen or alkyl Cl to 6,
A represents a single bond,
E represents COOH.

According to our invention we also provide a pharmaceutical composition comprising (preferably less than 80%, and more preferably less than 50% by weight) of a compound of formule I, or a pharmaceutically acceptable salt, or where E is a —COOH group, ester or amide thereof, in combination with a pharmaceutically acceptable adjuvant, diluent or carrier. Examples of suitable adjuvants, diluents or carriers are:— for tablets, capsules and dragees; microcrystalline cellulose, calcium phosphate, diatomaceous earth, a sugar such as lactose, dextrose or mannitol, talc, stearic acid, starch, sodium bicarbonate and/or gelatin; for suppositories, natural or hardened oils or waxes; and for inhalation compositions, coarse lactose. The compound of formula I, or the pharmaceutically acceptable salt, or where E or $E_1$ is a —COOH group, ester or amide thereof, preferably is in a form having a mass median diameter of from 0.01 to 10 microns. The compositions may also contain suitable preserving, stabilising and wetting agents, solubilisers, sweetening and colouring agents and flavourings. The compositions may, if desired, be formulated in sustained release form. We prefer compositions which are designed to be inhaled.

The invention is illustrated by the following Examples, in which temperatures are in degrees centigrade.

Example 1

10-Propyl-2,8-bis(1H-tetrazol-5-yl)-4H,6H-benzo[1,2-b:5,4-b']dipyran-4,6-dione

a) 4,6-Dioxo-10-propyl-4H,6H-benzo[1,2-b:5,4-b']dipyran-2,8-dicarboxamide

Diethyl 4,6-dioxo-10-propyl-4H,6H-benzo[1,2-b:5,4-b']dipyran-2,8-dicarboxylate (2 g, 5 mmol) was dissolved in ethanol (100 ml) and ammonia gas passed through the solution for 2 hours. The flow of ammonia was then stopped and the reaction mixture stirrd for a further hour. The resulting suspension was treated with ethanolic HCl and water and then filtered to give the sub-title compound as a.buff coloured solid on recrystallisation from methylene chloride.

b) 4,6-Dioxo-10-propyl-4H,6H-benzo[1,2-b:5,4-b']dipyran-2,8-dicarbonitrile

The product of step a) (7.5 g, 21.9 mmol) was slowly added to a solution of $POCl_3$ (6.12 ml, 65.65 mmol) in dimethylformamide (500 ml) at 0°. The mixture was heated to 60° and held at that temperature for 42 hours. The reaction mixture was then cooled, poured into water, stirred for 1.5 hours, filtered, dried and recrystallised from acetone to give the sub-title compound as a dark pink solid.

c) 4,6-Dioxo-10-propyl-4H,6H-benzo[1,2-b:5,4-b']dipyran-2,8-di-(5H-tetrazole)

The product of step b) (300 mg, 0.9 mmol), ammonium chloride (0.138 g, 2.5 mmol) and sodium azide (0.168 g, 2.58 mmol) were added slowly with stirring to dry dimethylformamide (50 ml). The temperature of the suspension was raised to 60° over 2 hours and kept at that temperature for a further 2.5 hours.The reaction mixture was then cooled and poured into water (150 ml) to form a clear solution. Dilute HCl was then added until the suspension was acidic. The resulting precipitate was filtered off, washed with water and chloroform, and dried to give the sub-title product.

d) Disodium 10-propyl-2,8-bis(1H-tetrazol-5-yl)-4H,6H-benzo[1,2-b:5,4-b']dipyran-4,6-dione

To the product of step c) (3.5795 g, 8.9 mmol) in water (20 ml) was added with stirring 1.5132 g of sodium hydrogen carbonate. The resulting solution was filtered, evaporated to half its volume, triturated with acetone (40 ml), left to stand, filtered and the precipitate washed with acetone. The solid was then dissolved in sterile water, filtered and freeze dried to give the disodium salt of the title compound.

Analysis

| | | | |
|---|---|---|---|
| Found: | C, 36.80; | H, 2.48; | N, 19.80 |
| 21.5% water Requires: | C, 36.81; | H, 2.29; | N, 20.15 |

Example 2

4,6-Dioxo-10-propyl-8-(1H-tetrazol-5-yl)-4H,6H-benzo[1,2-b:5,4-b']dipyran-2-carboxylate

a) Ethyl 8-aminocarbonyl-4,6-dioxo-10-propyl-4H,6H-benzo[1,2-b:5,4-b']dipyran-2-carboxylate

Ethyl 4,6-dioxo-10-propyl-8-carboxy-4H,6H-benzo[1,2-b:5,4-b']dipyran-2-carboxylate (6.0 g, 16.13 mmol), thionyl chloride (1.50 ml, 20.5 mmoles) dry dimethylformamide (0.5 ml) and dry dichloroethane (100 ml) were stirred and heated unde reflux for 1 hour.

The cooled reaction mixture was evaporated to dryness to afford a pale orance solid. This was suspended in dry dimethylformamide (50 ml) with stirring and cooled in an ice water bath. A solution of ammonia in dry dimethylformamide (1.8% w/v; 30.5 ml, 32.3 mmol) was added dropwise over 15 mins, followed by stirring at this temperature for 2 hours. The cooling bath was removed and stirring continued for 3 hours. Further ammonia in dimethylformamide (2 ml) was added at room temperature and stirring continued overnight.

The resulting suspension was diluted to 1200 ml with ethyl acetate and stirring continued for 1 hour. Water (200 ml) was then added and the near complete solutions separated. The aqueous phase which had been made basic with $NaHCO_3$ was extracted with further ethyl acetate, and the combined organic layers were washed with sat. brine, filtered and separated. The organic phase was dried quickly with $Na_2SO_4$, washing the desiccant well with ethyl acetate, and evaporated to afford a semi-solid residue. Trituration with ether (200 ml) and filtration afforded the sub-title product (5.24 g) off white plates from ethanol, mp 229—31°.

Analysis

| | | | |
|---|---|---|---|
| $C_{19}H_{17}NO_7$ requires: | C, 61.45; | H, 4.62; | N, 3.77 |
| found: | C, 61.73; | H, 4.77; | N, 3.75 |

b) Ethyl 8-cyano-4,6-dioxo-10-propyl-4H,6H-benzo[1,2-b:5,4-b']dipyran-2-carboxylate

Phosphoryl chloride (1.50 ml, 16.18 mmole) was added dropwise to stirred dimethylformamide (65 ml) with ice-cooling over 10 mins. The product of step a) (4.0 g, 10.75 mmol) was added in one portion and the suspension heated at 50—55°C for 30 mins. The cooled reaction mixture was poured into vigorously stirred brine (3 l) and the precipitated solid collected by filtration, dissolved in ethyl acetate (400 ml) washed with sat. brine, dried ($MgSO_4$) and evaporated to afford a fawn-coloured solid 3.77 g, which was recrystallised from 60—80° petroleum ether (75 ml) evaporating to 110 ml to afford the sub-title product as cream coloured plates, 2.52 g after vacuum-drying at 70°. mp 182—4°.

Analysis

| | | | |
|---|---|---|---|
| $C_{19}H_{15}NO_6$ requires: | C, 64.59; | H, 4.28; | N, 3.96 |
| found: | C, 64.49; | H, 4.01; | N, 4.40 |

c) Ethyl 4,6-dioxo-10-propyl-8-(1H-tetrazol-5-yl)-4H,6H-benzo[1,2-b:5,4-b']dipyran-2-carboxylate

A mixture of the product of step b) (2.34 g, 6.63 mole), ammonium chloride (0.44 g, 8.22 mmol), sodium azide (0.53 g, 8.15 mmole) and dry dimethylformamide (45 ml) was heated on a steam bath for 2.75 hours then allowed to cool overnight, and poured with vigorous stirring into sat. brine (1.5l) and dil. HCl (1 l). The precipitated solid was collected by filtration, washed well with water, then ether and vacuum-dried over $P_2O_5$. The product was Soxhlet extracted with acetone, evaporating to a final volume of 140 ml. On cooling the product separated as a tan coloured solid, 1.53 g, after vacuum drying at 70°C.

Analysis

| | | | |
|---|---|---|---|
| $C_{19}H_{16}N_4O_6$ requires: | C, 57.57; | H, 4.07; | N, 14.14 |
| Found: | C, 57.18; | H, 4.08; | N, 13.97 |

d) Disodium 4,6-dioxo-10-propyl-8-(1H-tetrazol-5-yl)-4H,6H-benzo[1,2-b:5,4-b']dipyran-2-carboxylate

Sodium hydroxide (12.62 ml of 0.1 M solution) was added over 30 mins to a stirred suspension of the product of step c) (0.25 g) in methanol (25 ml) whilst heating under reflux.

The reaction mixture was then filtered and evaporated to form a yellow semi-solid residue. This solid was dissolved in methanol, filtered and the product precipitated with ethyl. The resulting yellow solid was dissolved in sterile water, filtered and freeze-dried to afford the sub-title product as a yellow solid 1.288 g.

Analysis

| | | | | |
|---|---|---|---|---|
| Found: | C, 44.07; | H, 3.40; | N, 12.02; | $H_2O$, 10.3 |
| $C_{17}H_{10}N_4Na_2O_6$ requires for 10.3% $H_2O$: | C, 44.42; | H, 3.32; | N, 12.18 | |

## Example 3

8-Aminocarbonyl-4,6-dioxo-10-propyl-4H,6H-benzo[1,2-b:5,4-b']dipyran-8-carboxylic acid

a) 8-Aminocarbonyl-4,6-dioxo-10-propyl-4H,6H-benzo[1,2-b:5,4-b']dipyran-2-carboxylic acid

Sodium hydroxide solution (0.1M, 41.3 ml) was added with stirring to a refluxing suspension of the amide from Example 1(a) (1.53 g) in methanol (125 ml). When the addition was complete the resulting solution was cooled, added to water (1L) and hydrochloric acid (dil. 50 ml) added to give a precipitate. Recrystallisation from acetone:water gave the title compound as off white needles (0.895 g), mp 322—4° (decomp.).

b) Sodium 8-aminocarbonyl-4,6-dioxo-10-propyl-4H,6H-benzo[1,2-b:5,4-b']dipyran-2-carboxylate

The acid amide from step a) (0.85 g) and sodium bicarbonate (0.206 g) were stirred in water (50 ml) at room temperature until a clear, yellow solution was attained. Filtration, followed by freeze drying gave the sodium salt of the title compound as a pale yellow solid.

$C_{17}H_{12}NNaO_7$,5%$H_2O$

| | | | |
|---|---|---|---|
| Requires: | C, 52.72; | H, 3.23; | N, 3.65 |
| Found: | C, 53.10; | H, 3.64; | N, 3.63 |

Example 4

8-(N,N-Dimethylcarbonylamino)-4,6-dioxo-10-propyl-4H,6H-benzo[1,2-b:5,4-b']dipyran-2-carboxylate

a) Ethyl 8-[N,N-dimethylcarbonylamino]-4,6-dioxo-10-propyl-4H,6H-benzo[1,2-b:5,4-b]dipyran-2-carboxylate

To a stirred solution of 33% w/v dimethylamine in methanol (25 ml) in dry ether, (100 ml), was added, over 5 minutes, portions of finely powdered 4-nitrophenyl 4,6-dioxo-10-propyl-4H,6H-benzo[1,2-b:5,4-b']-dipyran-2,8-dicarboxylate, (15 g). The mixture was kept under reflux for 30 minutes then volatiles were removed.

The residue was dissolved in ethanol, (40 ml) treated with conc. hydrochloric acid (1 ml) and heated under reflux for 10 minutes then evaporated. The residue was extracted into chloroform and then washed with water, dried ($MgSO_4$), filtered and evaporated. Recovered material was dissolved in hot ethyl acetate and crystallised from an ethyl acetate/60—80° petrol mixture to give, sub-title material, (3.7 g) m.p. 270°(d).

b) 8-(N,N-Dimethylcarbonylamino)-4,6-dioxo-10-propyl-4H,6H-benzo[1,2-b:5,4-b']dipyran-2-carboxylic acid

The product of Step a) (3.6 g) and sodium bicarbonate, (1.2 g) were treated with aqueous methanol and heated under reflux for 30 minutes to give a clear solution. The alcohol was evaporated off and the concentrate was diluted with water, filtered then acidified with dilute hydrochloric acid. A precipitate was obtained and was filtered off, washed with water and dried to give the sub-title compound as a cream powder, (2.2 g), m.p. 267—70°(d).

c) Sodium 8-(N,N-dimethylcarboxamido)-4,6-dioxo-10-propyl-4H,6H-benzo[1,2-b:5,4-b']dipyran-2-carboxylate

The product of Step b) (2.2 g) and sodium bicarbonate, (0.5 g) were mixed and dissolved in water, (6 ml). The resulting solution was filtered and then added dropwise to anhydrous acetone. A precipitate was obtained which was filtered off and crystallised from a methanol/acetone/water mixture and dried *in vacuo* to give the sodium salt of the title compound as a grey powder (1.3 g).

Analysis

| | | | | |
|---|---|---|---|---|
| Found: | C, 51.54; | (H, 3.92); | N, 3.05; | $H_2O$, 9.63% (unencapsulated), |
| $C_{19}H_{16}N NaO_7,3H_2O$ requires: | C, 51.00; | (H, 4.92); | N, 3.13; | $H_2O$, 12%. |

Example 5

8-(4-Carboxyphenyl)-4,6-dioxo-10-propyl-4H,6H-benzo[1,2-b:5,4-b']dipyran-2-carboxylic acid

a) Ethyl 6-acetyl-7-(4-methoxycarbonylbenzoyloxy)-4-oxo-8-propyl-4H-1-benzopyran-2-carboxylate

Ethyl 6-acetyl-7-hydroxy-4-oxo-8-propyl-4H-[3,2-b]benzopyran-2-carboxylate (1.5 g, 4.7 mmol) and p-methoxycarbonylbenzoyl chloride (975 mg, 4.9 mmol) in dry pyridine (20 ml) were heated on a steam bath for 4 hours. The mixture was then poured into concentrated hydrochloric acid and extracted with ethyl acetate (3 times). The organic layers were washed with 2M hydrochloric acid, water, dilute sodium bicarbonate solution (3 times) and water (twice), dilute sodium bicarbonate solution (3 times) and water (twice), and then dried to yield on removal of solvent a pale yellow solid. Recrystallisation from ethanol gave the sub-title compound as off white crystals, 1.86 g.

$^1$NMR ($CDCl_3$), δ: 8.62 (s, 1H); 8.27 (ABq, 4H); 7.9 (s, 1H); 4.60 (q, 2H); 4.00 (s, 3H); 3.00 (t, 2H); 2.64 (s, 3H), 1.7 (m, 2H); 1.51 (t, 3H); 1.02 (t, 3H).

b) 8-(4-Carboxyphenyl)-4,6-doxo-10-propyl-4H,6H-benzo[1,2-b:5,4-b']dipyran-2-carboxylic acid, mixed dimethyl and methyl ethyl esters.

The product of step a) (2 g, 4M mmol) and sodium hydride (50% oil suspension, 1.4 g, 29 mmole) were stirred under nitrogen at room temperature in 1,4-dioxan for 0.5 hours, then heated under reflux for a further 5 hours.

The product mixture was poured into methanolic hydrochloric acid (200 ml) and heated under reflux for 1 hour. The yelow solution was then poured into water and extracted with ethyla cetate (3 times). The combined organics were washed with water (twice), dried (anhydrous sodium sulphate) and evaporated to give a grey solid which was refluxed with methanol (500 ml) for 15 minutes. The suspension was allowed to cool to room temperature and grey solid filtered off and dried over phosphorus pentoxide to yield 930 mg of a 5:2 mixture of the sub-title dimethyl and methyl ethyl esters. Concentration of the mother liquors gave a further 320 mg of the desired product.

c) (4-Carboxyphenyl)-4,6-dioxo-10-propyl-4H,6H-benzo[1,2-b:5,4-b']dipyran-2-carboxylic acid

The diester of step b) (175 mg, 0.39 mmol) was heated under reflux in acetic acid (20 ml) and concentrated hydrochloric acid (5 ml). After 8 hours further concentrated hydrochloric acid (3 ml) was added and reflux continued overnight. The mixture was allowed to cool and stand at room temperature for 24 hours, then the greyish solid filtered off and washed to give the title product, 155 mg, mp 250°.

$^1$HNMR ($(CD_3)_2SO$), δ: 8.55 (s, 1H); 8.20 (ABq, 4H); 7.21 (s, 1H); 6.96 (s, 1H); 3.22 (m, 2H); 1.8 (m, 2H); 1.03 (t, 3H).

Elemental analysis: $C_{23}H_{16}O_8$

| | | |
|---|---|---|
| Requires: | C, 65.71; | H, 3.84%; |
| Found: | C, 65.56; | H, 3.81%. |

d) Disodium 8-(4-Carboxyphenyl)-4,6-dioxo-10-propyl)-4H,6H-benzo[1,2-b:5,4-b']dipyran-2-carboxylate

The diacid from step c) (920 mg, 2.19 mmol) was treated with sodium bicarbonate (368 mg; 4,38 mmol) in water (25 ml). Addition of acetone precipitated a pale red brown solid which was filtered off, dissolved in water, filtered through a millipore filter (0.5 micron) and freeze dried to give the disodium salt of the title compound, 997 mg.

Elemental analysis: $C_{23}H_{14}Na_2O_8.4H_2O$

| | | | |
|---|---|---|---|
| Requires: | C, 51.49; | H, 4.13; | Na, 8.57%; |
| Found: | C, 51.38; | H, 2.59; | Na, 8.55. |

Example 6

3-(8-Carboxy-4,6-dioxo-10-propyl-4H,6H-benzo[1,2-b:5,4-b']dipyran-2-(propanoic acid

a) Ethyl 6-acetyl-7-hydroxy-4-oxo-8-propyl-4H-1-benzopyran-2-carboxylate

4,6-Dioxo-10-propyl-4H,6H-benzo[1,2-b:5,4-b']dipyran-2,8-dicarboxylic acid (3.44 g) and sodium bicarbonate (3.4 g) were heated together in ethanol (25 ml) and water (25 ml) under reflux with mechanical stirring for five hours. Water (300 ml) was added and the mixture was washed with ether. The mixture was acidified and extracted with ether. The ether extracts were combined washed with water and brine then dried and evaporated to leave a pale yellow solid (2.3 g). The solid was dissolved in dry ethanol (100 ml) and heated under reflux for two hours during which time hydrogen chloride gas was passed through the solution. The cooled solution was concentrated in vacuo and the resultant solid crystallised from ethanol to afford prisms 1.54 g. Recrystallisation from ethyl acetate/ether gave the sub-title compund 1.06 g mp 129—131°.

NMR $CDCl_3$, δ: 12.9 (s, 1H), 8.55 (s, 1H), 7.0 (s, 1H), 2.75 (s, 3H).

b) Ethyl (6-Acetyl-2-ethoxycarbonyl-4-oxo-8-propyl-(4H-1-benzo-pyran-7-yl)succinate

Ethyl 6-acetyl-7-hydroxy-4-oxo-8-propyl-4H-1-benzopyran-2-carboxylate (5 g) in dry dimethylformamide (100 ml) was added dropwise to an ether washed suspension of sodium hydride (0.8 g of 50% in oil) stirred in dry dimethylformamide (200 ml) under nitrogen. After addition was complete the mixture was stirred for a further thirty minutes. A solution of ethyl succinyl chloride (2.37 ml) in dry dimethylformamide (50 ml) was added dropwise to the red solution and the mixture then heated at 60° for two hours. The mixture was poured onto water (500 ml) containing concentrated hydrochloric acid (50 ml) and ether extracted. The combined extracts were washed with dil. hydrochloric acid, water, saturated sodium bicarbonate solution and water then dried and evaporated. Crystallisation from cyclohexane gave the sub-title compound as colourless prisms 4.9 g mp 76—78°.

NMR ($CDCl_3$), δ: 8.52 (s, 1H), 7.12 (s, 1H), 2.63 (s, 3H), 1.45 (t, 3H), 1.30 (t, 3H), 1.0 (t, 3H), 4.48 (q, 2H), 4.20 (q, 2H), 2.9 (m, 2H), 1.7 (m, 2H).

c) Ethyl 3-(8-ethoxycarbonyl-4,6-dioxo-10-propyl-4H,6H-benzo[1,2-b:5,4-b']dipyran-2)propanoate

A solution of ethyl (6-acetyl-2-ethyoxycarbonyl-4-oxo-8-4H-1-benzopyran-7-yl) succinate (10.9 g) in dry dioxan (200 ml) was added dropwise to a stirred suspension of ether washed sodium hydride (2.75 g of 50% in oil) in dry dioxan (500 ml) under a nitrogen atmosphere. After addition was complete ethanol (one drop) was added and the mixture heated to 80° and stirred at this temperature for four hours. After cooling the mixture was diluted with water (500 ml) acidified with dilute hydrochloric acid and extracted with chloroform. The combined extracts were washed with dilute hydrochloric acid and water then dried and evaporated to leave an oil. The oil was dissolved in ethanol (300 ml) conc. hydrochloric acid (5 ml) added and the mixture heated under reflux for three hours. The mixture was concentrated in vacuo and the residue crystallised from ethanol to afford the sub-title compound as buff prisms 3.4 g mp 131—133°.

| | | |
|---|---|---|
| CHN: Theory: | C, 64.48; | H, 5.65 |
| Found: | C 64.34; | H, 5.84 |

d) Disodium 3-(8-carboxy-4,6-dioxo-10-propyl-4H,6H-benzo[1,2-b:5,4-b']dipyran-2)propanoate

Ethyl 8-ethoxycarbonyl-4,6-dioxo-10-propyl-4H,6H-benzo[1,2-b:5,4-b']dipyran-2-propanoate (1 g) was heated in methanol (200 ml) under reflux. Sodium hydroxide solution (43.5 ml of 0.1016M) was added dropwise over two hours. The mixture was heated for a further two hours then the mixture concentrated in vacuo to 2 ml. Acetone (500 ml) was added and the precipitate collected. The solid was dissolved in water and dilute hydrochloric acid added. After ten minutes the product was collected. Purification by reverse phase HPLC gave 340 mg of the title compound.

| | | |
|---|---|---|
| CHN: Found: | C, 57.18; | H, 4.99 |
| Req. for 61.7% $H_2O$: | C, 57.18; | H, 4.78 |

The diacid (340 mg) was stirred in distilled water (40 ml) with sodium bicarbonate (144 mg). When complete solution was attained the solution was freeze dried to leave the disodium salt of the title compound, as a fine white solid 348 mg.

NMR (($(CD_3)_2SO$, δ) 8.44 (s, 1H), 6.68 (s, 1H), 6.20 (s, 1H), 3.09 (t, 2H), 2.88 (t, 2H), 2.35 (t, 2H), 1.70 (m, 2H), 0.91 (t, 3H).

| | | |
|---|---|---|
| CHN: Found: | C, 42.93; | H, 4.92 |
| Req. for 61.7% $H_2O$: | C, 42.93; | H, 5.06. |

Example 7

4,6-Dioxo-12-propyl-4H,6H-[1]-benzopyrano[3,2-g][1]-benzopyran-2,8-dicarboxylic acid

a) 4-[4-Acetyl-3-hydroxy-2-propylphenoxy]isophthalic acid

A solution of dimethyl 4-bromoisophthalate (2.73 g) and 2,4-dihydroxy-3-propylphenylethanone (1.94 g) in nitrobenzene (20 ml) was heated at 140° under nitrogen for 3 hours with copper powder (200 mg) and potassium carbonate (2.7 g). The solution was cooled and a solution of sodium hydroxide (1.6 g) in water (8 ml) and ethanol (22 ml) was added. The mixture was heated under reflux for one hour. The cooled mixture was poured onto ice-water and washed with dichloromethane. The aqueous solution was acidified with hydrochloric acid and the precipitate collected. Crystallisation from 20% aqueous ethanol gave the sub-title compound as a brown solid 0.8 g mp 280—283°.

b) 7-Acetyl-6-hydroxy-5-propylxanthane-2-carboxylic acid

4-[4-Acetyl-3-hydroxy-2-propylphenoxy]isophthalic acid (0.8 g) was dissolved in sulpholane (10 ml) and heated to 80°. Polyphosphoric acid (10 ml) was added and the solution stirred for 4 hours. The mixture was poured onto water and the precipitate collected to afford the sub-title compound as a pale brown solid 0.65 g, mp >300°.

c) Ethyl 7-acetyl-6-hydroxy-5-propylxanthane-2-carboxylate

7-Acetyl-6-hydroxy-5-propylxanthane-2-carboxylic acid (3.4 g) was dissolved in ethanolic hydrogen chloride (250 ml) and the solution heated under reflux for 3 hrs. The mixture was concentrated *in vacuo* and the residue taken up in water, neutralised with sodium carbonate solution and extracted with chloroform. The solution was dried and evaporated to leave the sub-title compound as a brown solid which was crystallised from ethanol as pale brown plates 1.7 g mp 200—201°.

d) Diethyl 4,6-dioxo-12-propyl-4H-6H[1]benzopyrano[3,2-g][1]benzopyran-2,8-dicarboxylate

A solution of ethyl 7-acetyl-6-hydroxy-5-propylxanthane-2-carboxylate (3.68 g) in ethanol (20 ml) was added to a solution of sodium ethoxide prepared from ethanol (80 ml) and sodium (2 g). After stirring for 5 mins. diethyl oxalate (4 ml) was added. The mixture was stirred for 1 hr to produce a bright yellow suspension. The mixture was poured onto ice-water and acidified with hydrochloric acid and extracted with ethyl acetate. The ethyl acetate was dried and concentrated in vacuo. The residue was taken up in ethanolic hydrogen chloride and left at room temperature for 16 hrs. The mixture was diluted with water and extracted with dichloromethane. The dichloromethane was washed with sodium bicarbonate solution and brine then dried and evaporated to give a pale brown solid 3.5 g mp 195—210°. Crystallisation from toluene (twice) gave the sub-title compound as colourless plates 1.1 g mp 218—220°.

e) 4,6-Dioxo-12-propyl-4H-6H-[1]benzopyrano[3,2-g][1]benzopyran-2,8-dicarboxylic acid

Diethyl 4,6-dioxo-12-propyl-4H-6H-[1]benzopyrano[3,2-g][1]benzopyran-2,8-dicarboxylate[2.75 g] was stirred in conc. sulphuric acid (90 ml) and the mixture heated keeping the temperature between 70 and 80° for three hours. The cooled mixture was poured onto ice-water and the precipitate collected and washed with water. Crystallisation from dimethylformamide produced the sub-title compound as pale yellow prisms 1.9 g mp >300°.

f) Disodium 4,6-dioxo-12-propyl-4H-6H-[1]benzopyrano[3,2-g][1]benzopyran-2,8-dicarboxylate

4,6-Dioxo-12-propyl-4H-6H-[1]benzopyrano[3,2-g][1]benzopyran-2,8-dicarboxylic acid (1.9 g) was suspended in water (50 ml) and sodium bicarbonate (770 mg) added portionwise over 30 mins. The mixture was stirred for a further ten minutes then filtered. Acetone was added to precipitate the product which was collected and washed with acetone. The solid was dissolved in water, filtered and the solution freeze dried to leave the disodium salt of the title compound as a white powder 860 mg.

Analysis

| | | |
|---|---|---|
| Found: | C, 47.77; | H, 2.39 |
| Req for 16.3% $H_2O$: | C, 48.17; | H, 2.31—4.10 |

$H_2O$ 16.3% weight loss by thermogravimetric analysis (TGA) at 200°.

Example 8

6,7,8,9-Tetrahydro-4-oxo-10-propyl-4H-naphtho[2,3-b]pyran-2,7-dicarboxylic acid

a) Ethyl 6-methoxy-1-oxo-1,2,3,4-tetrahydronaphthalene-2-carboxylate

A solution of 6-methoxy-1-oxo-1,2,3,4-tetrahydronaphthalene (3 g) in tetrahydrofuran (30 ml) was added slowly to a solution of diethyl carbonate (5.85 ml) in tetrahydrofuran (150 ml) containing a suspension of sodium hydride (50% in oil, 3.28 g) over a period of 30 minutes. The mixture was treated at reflux with stirring, for 4 hours. Acetic acid (6 ml) then dilute HCl (20 ml) were added dropwise, and the bulk of the tetrahydrofuran removed by evaporation. The residue was taken up in ether (100 ml), washed with saturated brine and evaporated to give the sub-title compound as an orange oil (4.4 g).

b) Ethyl 6-methoxy-1,2,3,4-tetrahydronaphthalene-2-carboxylate

The ketoester from step a) (2.6 g) was mixed with palladium on charcoal catalyst (10%, 0.25 g) suspended in acetic acid (10 ml) and added to acetic acid (10 ml) containing 10 drops of perchloric acid. The mixture was then hydrogenated at 3 atmospheres pressure at room temperature for 18 hours. The mixture was filtered to remove catalyst, evaporated to a residue, the residue dissolved in ethyl acetate, washed, separated and dried to give on evaporation the sub-title compound as an orange oil, bp 130—40°,/0.03 mm.

c) Ethyl 7-acetyl-6-hydroxy-1,2,3,4-tetrahydronaphthalene-2-carboxylate

The methoxy ester from step b) (1.73 g) was heated under reflux with hydrobromic acid (48% aqueous solution, 8 ml) for 2 hours and then evaporated to dryness, to give an orange oil. This oil was dissolved in boron trifluoride:acetic acid complex (15 ml) and heated on a steam bath. After 1.25 hours the reaction mixture was poured into saturated brine (400 ml), the pH adjusted to about 5 by the addition of saturated aqueous sodium bicarbonate and the mixture stirred overnight. Ethyl acetate was added, the organic layer separated, washed with saturated brine, dried and evaporated to give the sub-title compound (1.89 g), colourless solid from pentane, mp 73—5°.

d) Ethyl 7-acetyl-6-prop-2-enyloxy-1,2,3,4-tetrahydronaphthalene-2-carboxylate

The product from step c) (42.6 g), 3-bromopropene (17.5 ml) and potassium carbonate (27 g) were heated at 55° with stirring in dimethylformamide (250 ml) containing a one crystal of potassium iodide. After 24 hours, the reaction mixture was cooled, poured into iced brine (4 l) and the pale yellow precipitate formed collected by filtration. The preciptate was washed, dried and recrystallised from petrol (bp 30—40°) to give the sub-title compound as pale yellow crystals, 27.0 g.

e) 7-acetyl-6-hydroxy-5-prop-2-enyl-1,2,3,4-tetrahydronaphthalene-2-carboxylate

The product from step d) (0.5 g) was heated under reflux under an atmosphere of nitrogen in N-methylpyrrolidone (4 ml) for 1.5 hours. The reaction mixture was then poured into saturated brine (250 ml), to give a precipitate which on filtration, washing and drying gave the sub-title compound as an orange powder, 0.49 g.

f) Ethyl-7-acetyl-6-hydroxy-5-propyl-1,2,3,4-tetrahydronaphthalene-2-carboxylate

The product of step e) (0.49 g) was hydrogenated at atmospheric pressure at room temperature in ethanol (100 ml) with palladium on charcoal (5% 50 mg) as catalyst. Filtration to remove catalyst, followed by evaporation of the filtrate gave the sub-title compound as a colourless solid, 0.43 g.

g) Diethyl 6,7,8,9-tetrahydro-4-oxo-10-propyl-4H-naphtho[2,3-b]pyran-2,7-dicarboxylate

The product of step f) (0.42 g) was added to a stirred solution of diethyl oxalate (0.56 ml) and sodium ethoxide (from 0.16 g sodium) in ethanol (25 ml). The mixture was heated at reflux for 40 minutes then cooled to room temperature then ethanolic HCl added (15 ml). The mixture was then heated for an hour, the solvent removed by evaporation and the residue separated between ethyl acetate and water. The organic layer on washing, drying and evaporation gave the sub-title compound as an oil which crystallised on standing, (0.58 g), mp 89.5—90.5°.

h) 6,7,8,9-Tetrahydro-4-oxo-10-propyl-4H-naphtho[2,3-b]pyran-2,7-dicarboxylic acid

The product of step g) (2.3 g), glacial acetic acid (30 ml) and concentrated hydrochloric acid (15 ml) were heated at reflux for 1 hour, then cooled and filtered to give the title compound, as a white powder (1.88 g) mp 271—2°.

i) Disodium 6,7,8,9-tetrahydro-4-oxo-10-propyl-4H-naphtho[2,3-b]pyran-2,7-dicarboxylate

The product of step h) (1.758 g) and sodium bicarbonate (0.885 g) were stirred in water (5 ml) at room temperature until complete dissolution occurred. The solution was filtered and then poured into acetone (400 ml) with vigarous stirring. The precipitate that formed was dissolved in water (20 ml) and freeze dried, to give the disodium salt of the title compound as a colourless solid, (1.654 g).

| | | | |
|---|---|---|---|
| $C_{18}H_{16}Na_2O_6,5H_2O$ requires: | C, 46.13; | H, 4.24; | Na, 10.9% |
| Found: | C, 46.56; | H, 5.64; | Na, 9.91% |

## Example 9

6,7,8,9-Tetrahydro-5-hydroxy-4-oxo-10-propyl-4H-naphtho[2,3-b]pyran-2,7-dicarboxylic acid

(a) Diethyl 6,7,8,9-Tetrahydro-5-nitro-4-oxo-10-propyl-4H-naphtho[2,3-b]pyran-2,7-dicarboxylate

The diester from Example 10 (g) (1 g) was dissolved in concentrated sulphuric acid (5 ml) with stirring and cooled to 0°. Fuming nitric acid (0.3 ml) was added dropwise over 10 minutes, the ice path was then removed and the reaction mixture was stirred at room temperature for 30 minutes. The mixture was then poured into the water, vigorously stirred and the resulting precipitate collected by filtration. Recrystallisation from petrol (bp 60—80°)/acetone gave the sub-title compound as colourless needles. (0.9 g), mp 164—6°.

(b) Diethyl 5-amino-6,7,8,9-tetrahydro-4-oxo-10-propyl-4H-naphtho[2,3-b]pyran-2,7-dicarboxylate

The diester from step (a) (7.62 g) was dissolved in hot ethyl acetate (500 ml). The resulting solution was cooled to room temperature and hydrogenated at atmospheric pressure in the presence of palladium on charcoal (5%, 0.75 g). After 90 hours, the reaction mixture was filtered to remove catalyst and the filtrate evaporated to give the sub-title product as orange needles (4.81 g), from petrol (bp 60—80°/acetone), mp 102—103°.

(c) Diethyl 6,7,8,9-tetrahydro-5-hydroxy-4-oxo-10-propyl-4H-naphtho[2,3-b]pyran-2,7-dicarboxylate

The amine from step (b) (4.65 g) was dissolved in sulphuric acid 50%, (60 ml) at room temperature and then cooled to 0°.

A solution of sodium nitrite (1.20 g) in water (8 ml) was added dropwise over 30 minutes, whilst maintaining the internal temperature between 2—4°. The cooled reaction mixture was then added in 1 ml portions to sulphuric acid solution (20%, 190 ml) containing urea (9.3 g) maintained at a temperature of 140°. The sulphuric acid solution was maintained at this temperature for a further 2 hours after the addition was complete, then cooled and poured into water (21). The resulting precipitate was filtered off and esterified with ethanolic HCL, evaporated to dryness to give the sub-title compound as yellow needles (3.40 g), from ethanol, mp 107—9°.

(d) 6,7,8,9-Tetrahydro-5-hydroxy-4-oxo-10-propyl-4H-naphtho[2,3-b]pyran-2,7-dicarboxylic acid

The product of step (c) (1.20 g), glacial acetic acid (15 ml) and concentrated hydrochloric acid (9 ml) were heated with stirring at reflux for 4.5 hours. The mixture was cooled and the title product collected by filtration as a yellow solid (1.00 g) mass spec. m/e 346.

(e) Disodium 6,7,8,9-Tetrahydro-5-hydroxy-4-oxo-10-propyl-4H-naphtho[2,3-b]pyran-2,7-dicarboxylate

A solution of sodium bicarbonate (0.5 g) in water (10 ml) was added to a stirred suspension of the diester from step (d) (1.065 g) in water (5 ml) to give a clear solution. The solution was evaporated to dryness and the residue dissolved in methanol (50 ml). The methanol solution was poured into ether to give a pale yellow solid, which was dissolved in water and freeze-dried to give the disodium salt of the title product (1.09 g).

| | | | |
|---|---|---|---|
| $C_{18}H_{16}Na_2O_7$, 11.79%$H_2O$ requires: | C 48.86, | H 4.58, | $H_2O$ 11.79% |
| Found: | C 48.86, | H 4.94, | $H_2O$ 11.79% |

### Example 10

6,7,8,9-Tetrahydro-5-hydroxy-4-oxo-10-propyl-4H-naphtho[2,3-b]pyran-2,8-dicarboxylic acid

The title acid was prepared from the corresponding diethyl ester, mp 306—308° (dec.).

The disodium salt of the title acid was prepared by the method Example 9(e), uv spectrum, λmax. (ε) 206, (22750); 256, (13500); 270; 360.

### Example 11

7,8,9,10-Tetrahydro-1-oxo-5-propyl-1H-naphtho[2,1-b]pyran-3,8-dicarboxylic acid

(a) Diethyl 7,8,9,10-tetrahydro-1-oxo-15-propylnaphtho[2,1-b]pyran-3,8-dicarboxylate

Ethyl 5-acetyl-6-hydroxy-7-propyl-1,2,3,4-tetrahydronaphthalene-2-carboxylate (1.6 g) (isolated as a by-product from a large scale preparation of Example 8 (f) was reacted with diethyl oxalate (4 ml) and sodium ethoxide in ethanol by the method of Example 8 (g). Work up gave the sub-title product as off-white needles (1.48 g), mp 107.5—109°.

(b) 7,8,9,10-Tetrahydro-1-oxo-5-propyl-1H-naphtho(2,1-b]pyran-3,8-dicarboxylic acid

The diester from step (a) (1.16 g) glacial acetic acid (15 ml) and concentrated hydrochloric acid (10 ml) were heated at reflux for 3 hours, allowed to cool and the resulting precipitate collected by filtration, to give the title compound as a colourless solid, (1.067 g), mp 305—306 (dec).

(c) Disodium 7,8,9,10-Tetrahydro-1-oxo-5-propyl-1H-naphtho[2,1-b]pyran-3,8-dicarboxylate

The diacid (1.002 g) from step (b) and sodium bicarbonate (0.505 g) were stirred in water (10 ml) at room temperature until a clear solution was obtained. Filtration the solution followed by freeze-drying gave the disodium salt of the title compound, as a colourless solid (1.036 g).

u.v. spectrum: λmax (ε) 207, (17529); 243, (17711); 323, (5660).

### Example 12

4-Oxo-10-propyl-6-(trifluoromethyl))-4H-pyrano[3,2-g]quinoline-2,8-dicarboxylic acid, disodium salt

a) 2-Ethyl 8-methyl 6-bromo-4-oxo-10-propyl-4H-pyrano[3,2-g]quinoline-2,8-dicarboxylate

A solution of 2-ethyl 8-methyl 4,6-dioxo-10-propyl-4H-pyrano[3,2-g]quinoline-2,8-dicarboxylate (40 g, 0.108 m) and phosphorus oxybromide (62 g, 0.216 M) in 1,2-dichloroethane (2.5 L) was heated and stirred at reflux for 3 hours. The mixture was cooled, filtered, and methanol (1L) was added. The solution was evaporated to dryness and the residue was triturated with petroleum ether (40—60°) to give the sub-title compound as orange needles, 21.8 g (45%) mp 177—8°.

b) 2-Ethyl 8-methyl-4-oxo-10-propyl-6(trifluoromethyl)-4H-pyrano[3,2,g]quinoline-2,8-dicarboxylate

The product of step a) (12.5 g, 28 mmol) was stirred with trifluoromethyl iodide (50 g, 25 mmol) and activated copper powder (50 g, prepared by precipitation from cupric sulphate with zinc) in hexamethylphosphoric triamide (200 ml) in a nitrogen atmosphere at 110° in a pressure vessel for 3 hours. The mixture

was allowed to cool and was diluted with water (1L) and the solid was separated. Dichloromethane was added to the solid and undissolved copper was filtered off. The organic filtrate was dried, filtered, and the filtrate was evaporated to dryness leaving a brown solid. This solid was chromatographed on silica eluting with ether/petroleum ether 60—80° (3:1). Evaporation of the eluent gave a yellow solid 2.5 g (20%) mp 172—3°.

c) 4-Oxo-10-propyl-6-(trifluoromethyl)-4H-pyrano[3,2-g] quinoline-2,8-dicarboxylic acid

A solution of the product of step b) (2.4 g, 55 mmol) in boiling methanol (300 ml) was stirred at reflux while N/10 aqueous sodium hydroxide solution (110 ml) was added. After the addition the solution was heated at reflux for 30 minutes and was then poured into a large volume of acetone. The solid obtained was filtered off, dissolved in water, and the resulting solution was acidified with dilute HCl. The solid obtained was filtered off, washed well with water and was sucked dry. The product was crystallised from ethanol to give pale yellow needles, 1.6 g (73%) mp 248—9°.

Analysis

| | | | |
|---|---|---|---|
| Found: | C, 53.4; | H, 2.9; | N, 3.43% |
| $C_{18}H_{12}F_3NO_6$ requires with 2.54% water: | C, 53.3; | H, 2.59; | N, 3.45% |

d) Disodium 4-oxo-10-propyl-6-(trifluoromethyl)-4H-pyrano[3,2-g]quinoline-2,8-dicarboxylate

The product of step c) (1.466 g, 3.711 mmol) was added to a solution of sodium hydrogen carbonate (0.6235 g, 7.422 mmol) in water (50 ml). The resulting solution was filtered through a glass filter and the filtrate was freeze-dried. The solid obtained was dried *in vacuo* to give the disodium salt of the title compound, 1.5 g (97%).

Analysis

| | | | |
|---|---|---|---|
| Found: | C, 42.33; | H, 2.09; | N, 2.66% |
| $C_8H_{10}F_3NNa_2O_6$ requires with 13.2% water: | C, 42.7; | H, 1.97; | N, 2.77% |

Example 13

6-(Heptafluoropropyl)-4-oxo-10-propyl-4H-pyrano[3,2-g]quinoline-2,8-dicarboxylic acid

a) 2-Ethyl 8-methyl 6-(heptafluoropropyl)-4-oxo-10-propyl-4H-pyrano[3,2-g]quinoline-2,8-dicarboxylate

Activated copper powder (10 g) was washed several times with 0.02 molar ethylendiaminetetracetic acid solution and water, then filtered, washed with water and dried. The dry copper powder (10 g) the quinoline product of Example 12(a), (10 g), perfluoropropyl iodide (25 g) and hexamethylphosphoric triamide (100 ml) were placed in a 100 ml sealed tube and heated at 110° for 24 hours. The cooled mixture was poured into water (500 ml) and the mixture filtered. The residue was washed off the filter with methylene chloride leaving behind the copper. The methylene chloride was washed with water, dried and evaporated to leave a brown solid 11.1 g. Column chromatography using $SiO_2$ with $CH_2Cl_2$: toluene 9:1 as eluant produced a faster running product 2.5 g (21%). The product was recrystallised from acetone to leave the sub-title product as pale yellow plates 1.80 g mp 144—145.5°.

b) Disodium 6-(heptafluoropropyl)-4-oxo-10-propyl-4H-pyrano[3,2-g]quinoline-2,8-dicarboxylate

The diester product of step a) (1.79 g) was dissolved in methanol (50 ml) and heated under reflux. Sodium hydroxide solution (6.66 ml of 1 M) was added dropwise and heating continued for 30 minutes. The solvent was removed *in vacuo* and the product precipitated from methanol (50 ml) with ether (700 ml). A second precipitation from water (20 ml) with acetone (1000 ml) gave, after scratching and allowing to stand, a fine yellow powder. The powder was dissolved in water, filtered and then freeze-dried to leave the disodium salt of the title compound, as an ochre powder, 1.30 g.

Analysis

| | | | |
|---|---|---|---|
| Found: | C, 39.74; | H, 2.08; | N, 2.28 |
| Required for 10.62% $H_2O$: | C, 39.77 | H, 1.65—2.82; | N, 2.32 |

Example 14

4-Oxo-6-(pentafluoroethyl)-10-propyl-4H-pyrano[3,2-g]quinoline-2,8-dicarboxylic acid

a) 2-Ethyl 8-methyl-4-oxo-6-(pentafluoroethyl)-10-propyl-4H-pyrano[3,2-g]quinoline-2,8-dicarboxylate

MP 136—139° was prepared using the method of Example 13a), but substituting perfluoroethyl iodide for the perfluoropropyl iodide.

Analysis

| | | | |
|---|---|---|---|
| Theory: | C, 54.21; | H, 3.72; | N, 2.87 |
| Found: | C, 54.00; | H, 3.79; | N, 2.85 |

b) Disodium 4-oxo-6-(pentafluoroethyl)-10-propyl-4H-pyrano[3,2-g]quinoline-2,8-dicarboxylate was prepared by the method of Example 13b) but using the product of step a) of this Example as starting material.

Analysis

| | | | |
|---|---|---|---|
| Theory for 10.27 $H_2O$: | C, 41.85; | H, 2.99; | N, 2.57; |
| Found: | C, 41.64; | H, 2.79; | N, 2.52; |

Example 15

6-(Difluoromethyl)-4-oxo-10-propyl-4H-pyrano[3,2-g]quinoline-2,8-dicarboxylic acid

a) Diethyl 6-formyl-4-oxo-10-propyl-4H-pyrano[3,2-g]quinoline-2,8-dicarboxylate

A mixture of diethyl 6-methyl-4-oxo-10-propyl-4H-pyrano[3,2-g]quinoline-2,8-dicarboxylate (19 g), selenium dioxide (20.99 g), and glacial acetic acid (950 ml) was heated on a steam bath with stirring for 2 hours. The resulting suspension was cooled, filtered and poured into brine (8 l). The resulting precipitate was filtered off and the crude product dissolved in $CH_2Cl_2$, filtered and evaporated to dryness to give 20.5 g of the crude product (20.5 g). This product was chromatographed using 25:1 $CH_2Cl_2$: ethyl acetate as the solvent, yielding 13.95 g of required product (71%).

b) Diethyl 6-(difluoromethyl)-4-oxo-10-propyl-4H-pyrano[3,2-g]quinoline-2,8-dicarboxylate

To a solution of diethylaminosulphur trifluoride (0.6 ml) in dichloromethane (30 ml) at 25° was added dropwise a solution of the product of step (a) (2.0 g) in dichloromethane (20 ml). The mixture was stirred at 25° for 3 hours. A further quantity of diethylaminosulphur trifluoride (0.06 ml) was added and the mixture stirred at room temperature for a further 2 hours. The resulting mixture was poured into water and extracted with ethyl acetate. The ethyl acetate extracts were washed with brine, dried and evaporated to leave an orange solid 1.9 g. Chromatography on $SiO_2$, ethyl acetate/petroleum ether 1:2, followed by dry column $SiO_2$ toluene/ethyl acetate 1:9) produced 65% of the sub-title compound, 1.37 g.

NMR, UV and IR spectra were consistent with the required structure.

c) Disodium 6-(difluoromethyl)-4-oxo-10-propyl-4H-pyrano[3,2-g]quinoline-2,8-dicarboxylate.

To a stirred solution of the product of step b) (1.31 g) in methanol (200 ml) under reflux was added sodium hydroxide solution (12.09 ml of 0.5M) dropwise. Heating was continued for 30 mins then the solvent evaporated. The residue was dissolved in water (6 mls) and this solution added dropwise to acetone (800 mls) with stirring. The precipitate was collected, washed with acetone and then dissolved in sterile water. The solution was filtered and then freeze-dried to leave the disodium salt of the title compound as an ochre powder 1.138 g.

Analysis

| | | | |
|---|---|---|---|
| Found: | C, 45.42; | H, 2.78; | N, 2.71; |
| Required for 11.20% $H_2O$: | C, 45.66; | H, 2.34—3.55; | N, 2.95; |

Example 16

6,9-Dihydro-4,6-dioxo-10-propyl-4H,6H-pyrano[3,2-g]quinoline-2-carboxylic acid

(a) Ethyl 4,6-dioxo-10-propyl-4H,6H-pyrano[3,2-g]quinoline-2-carboxylate

6-Acetyl-4-chloro-7-hydroxy-8-propylquinoline (9.64 g) and diethyl oxalate (43.4 ml) in dry dimethylformamide (150 ml) were added to ether washed 50% sodium hydride (7.71 g) in dry dimethylformamide (150 ml) under nitrogen with stirring. After stirring at room temperature for 5 hrs the whole was poured into ethyl acetate and treated with aqueous acetic acid. The organic layer was separated, washed well with water and dried. The solvent was evaporated to leave a diketone which was dissolved in glacial acetic acid (100 ml) containing a few drops of concentrated sulphuric acid and heated under reflux for 24 hours. The reaction mixture was poured into water and extracted with ethyl acetate. Some insoluble material was filtered off to give 1.3 g of product.

The ethyl acetate was washed well with water, dried and the solvent evaporated to leave 1.2 g of product. Both product were shown by thin layer chromatography to be identical and were combined. Total yield, 2.5 g.

A recrystallisation from ethanol gave the sub-title compound, mp 296° dec.

Analysis:

| | | | |
|---|---|---|---|
| Found: | C, 65.8%; | H, 5,4%; | N, 4.03%; |
| $C_{18}H_{17}NO_5$ Required: | C, 66.1%; | H, 5.2%; | N, 4.3%; |

(b) 6,9-Dihydro-4,6-dioxo-10-propyl-4H,6H-pyrano[3,2-g]quinoline-2-carboxylic acid

The product of step (a) (1.92 g) in methanol (100 ml) was treated dropwise with 0.1 M sodium hydroxide solution (58.7 ml) with stirring under reflux. The reaction mixture was heated under reflux with stirring for a further 0.5 hour after addition, cooled, filtered and the filtrate acidified with aqueous glacial acetic acid. The precipitated product was collected by filtration, washed well with water and dried to give 1.37 g of green solid. This was suspended in water (100 ml), treated with sodium bicarbonate (0.385 g) and filtered to remove some green tar. The filtrate was acidified with glacial acetic acid and the precipitated

product collected by filtration, washed well with water and dried to give 1.16 g of the title product, mp 324—327° dec.

Analysis

| | | | |
|---|---|---|---|
| Found: | C, 63.88%; | H, 4.67%; | N, 4.38%; |
| $C_{16}H_{13}NO_5$ Required: | C, 64.2%; | H, 4.35%; | N, 4.68%; |

(c) Sodium 6,9-dihydro-4,6-dioxo-10-propyl-4H,6H-pyrano[3,2-g]-quinoline-2-carboxylate

The product of step (b) (0.833 g) and sodium bicarbonate (0.234 g) were stirred in water (100 ml) until complete solution was obtained. The whole was filtered and the filtrate freeze-dried to give 0.851 g of the sodium salt of the title compound.

Analysis:

| | | | |
|---|---|---|---|
| Found: | C, 53.53%; | H, 4.51%; | N, 3.9%; |
| $C_{16}H_{12}NNaO_5.2H_2O$ Required: | C, 53.78%; | H, 4.48%; | N, 3.92%; |

Example 17

6,9-Dihydro-4,6-dioxo-9-ethyl-10-propyl-4H,6H-pyrano-[3,2-g]quinoline-2-carboxylic acid

(a) 6,9-Dihydro-4,6-dioxo-9-ethyl-10-propyl-4H,6H-pyrano[3,2-g]-quinoline-2-carboxylic acid

Diethyl 6,9-dihydro-4,6-dioxo-9-ethyl-10-propyl-4H,6H-pyrano-[3,2-g]quinoline-2,8-dicarboxylate (13.5 g) and sodium bicarbonate (10.6 g) in ethanol (500 ml) and water (100 ml) were refluxed for 5 hours. The reaction mixture was poured into water (2 l), acidified, and the product which was the crude diacid obtained by filtration and drying *in vacuo*. The crude diacid was recrystallised from boiling dimethylformamide to give 4.9 g of the sub-title monoacid, mp 302° dec.

Analysis:

| | | | |
|---|---|---|---|
| Found: | C, 65.5%; | H, 5.5%; | N, 4.5%; |
| $C_{18}H_{17}NO_5$ Required: | C, 66.0%; | H, 5.2%; | N, 4.3%; |

(b) Sodium 6,9-dihydro-4,6-dioxo-9-ethyl-10-propyl-4H,6H-pyrano-[3,2-g]quinoline-2-carboxylate

The product of step (a) (4.736 g) and sodium bicarbonate (1.22 g) in water (150 ml) were stirred together until complete solution was obtained. The solution was filtered and the filtrate freeze-dried to give 5.05 g of the sodium salt of the title compound.

Analysis:

| | | | |
|---|---|---|---|
| Found: | C, 56.0%; | H, 5.2%; | N, 3.4%; |
| $C_{18}H_{16}NNaO_5.2H_2O$ Requires: | C, 56.1%; | H, 5.2%; | N, 3.6%; |

Example 18

6,9-dihydro-4,6-dioxo-9-phenylmethyl-10-propyl-4H-pyrano[3,2-g]quinoline-2-carboxylic acid

(a) N-[2,4-diacetyl-5-hydroxyphenyl]formamide

A mixture of formic acid (98%, 50 ml) and 1-[5-acetyl-2-amino-4-hydroxyphenyl]ethanone (5.0 g) was heated at reflux for 2 hours. The solution was allowed to cool and the crystalline material was filtered off and dried *in vacuo* at 80° to give the required amide, 5.2 g (90%) mp 193—4°.

| | | | |
|---|---|---|---|
| Found: | C, 59.66; | H, 4.99; | N, 6.2%; |
| $C_{11}H_{11}NO_4$ requires: | C, 59.7; | H, 4.97; | N, 6.3%; |

(b) 6-Acetyl-7-hydroxy-1-phenylmethyl-4(1H)-quinolinone

A suspension of pre-washed sodium hydride (0.87 g, 0.036 mmol) was stirred at 0° in dry dimethylformamide under nitrogen while N-[2,4-diacetyl-5-hydroxyphenyl]formamide (4.0 g, 18 mmol) was added in small portions. After the addition was complete the mixture was allowed to stir at 0° for 30 minutes, then benzyl bromide (3.1 g, 18 mmol) was added all at once. The mixture was stirred for 6 hours during which time the temperature was allowed to rise to 10°. The dark brown solution was poured into a large excess of water, the mixture was acidified to approximately pH 4.0, and was stirred overnight. The off-white solid was filtered off, washed well with water and was dried. The solid was crystallised as white needles 4.8 g (90%) mp 246—7°.

| | | | |
|---|---|---|---|
| Found: | C, 73.78; | H, 5.08; | N, 5.09%; |
| $C_{18}H_{15}NO_3$ requires: | C, 73.7; | H, 5.1; | N, 4.78%; |

(c) 6-Acetyl-1-phenylmethyl-7-(prop-2-enyloxy)-4(1H)-quinoline

A mixture of 6-acetyl-7-hydroxy-1-phenylmethyl-4(1H)-quinolinone (6.3 g, 21.5 mmol), anhydrous potassium carbonate (2.97 g, 21.5 mmol) and 3-bromopropene (2.86 g, 2.36 mmol) was stirred at room

temperature in dry dimethylformamide (100 ml) for 18 hours. The mixture was poured into a large excess of water and was stirred rapidly for 1 hour. The solid produced was filtered off and washed well with water. The yellow solid was dried to give the required quinolone as a yellow powder 6.2 g (87%) mp 139—140°.

Mass and n.m.r. spectra confirmed the structure.

(d) 6-Acetyl-7-hydroxy-1-phenylmethyl-8-(prop-2-enyl)-4(1H)-quinolinone

A solution of 6-acetyl-1-phenylmethyl-7-(prop-2-enyloxy)-4(1H)-quinolinone (6.0 g, 18 mmol) in N,N-diethylbenzeneamine (40 ml) was heated at reflux for 2 hours under nitrogen. The solution was cooled and was poured into a large volume of rapidly stirred petroleum ether (40—60°). A buff solid was produced which was filtered off and dried *in vacuo* to give the required quinolinone, 5.5 g (91%) mp 123—4°.

(e) 6-Acetyl-7-hydroxy-1-phenylmethyl-8-propyl-4(1H)-quinolinone

A solution of 6-acetyl-7-hydroxy-1-phenylmethyl-8-(prop-2-enyl)-4(1H)-quinolinone (5.0 g, 15 mmol) in ethanol (250 ml) was hydrogenated at room temperature and atmospheric pressure over 5% Palladium on charcoal catalyst (0.5 g) until hydrogen uptake was complete (45 minutes). The catalyst was removed by filtration and the filtrate was evaporated to dryness leaving the required quinolinone as a green oil, 4.2 g (84%).

(f) Ethyl 6,9-dihydro-4,6-dioxo-9-phenylmethyl-10-propyl-4H-pyrano[3,2-g]quinoline-2-carboxylate

A solution of 6-acetyl-7-hydroxy-1-phenylmethyl-8-propyl-4(1H)-quinolinone (3.0 g, 8.9 mmol) in ethanol (100 ml) was added to a solution of sodium ethoxide (from sodium, 1.15 g) in ethanol (150 ml). The mixture was stirred for 10 minutes then diethyl oxalate (3.6 g, 24.6 mmol) was added all at once and the mixture was heated at reflux for 30 minutes. The solution was poured into a large excess of water and the resulting solution was acidified to pH 4.0 with dilute HCl. The mixture was extracted with chloroform and the extracts were washed with water, dried over $MgSO_4$, filtered and the filtrate was evaporated to dryness. The residue was dissolved in ethanol (40 ml) containing ethanolic HCl (1 ml) and the solution was heated at reflux for 30 minutes. The solution was cooled and the solid obtained was filtered off and was crystallised from ethanol to give the required pyranoquinoline as pale yellow prisms, 1.9 g (50%) m.p. 207—8°.

| | | | |
|---|---|---|---|
| Found: | C 69.1; | H, 5.55; | N, 3.21%; |
| $C_{25}H_{23}NO_5$ requires: | C, 68.9; | H, 5.7; | N, 3.21%; |

with 1 mole $H_2O$.

(g) 6,9-Dihydro-4,6-dioxo-9-phenylmethyl-10-propyl-4H-pyrano[3,2-g]quinoline-2-carboxylic acid

A suspension of ethyl 6,9-dihydro-9-phenylmethyl-10-propyl-4H-pyrano[3,2-g]quinoline-2-carboxylate (1.8 g, 4.6 mmol) in 0.1 N aqueous sodium hydroxide solution (46.9 ml) containing ethanol (10 ml) was heated at reflux for 6 hours. The mixture was cooled, filtered and the filtrate was acidified and filtered. The solid obtained was dried *in vacuo* at 80° to give the required acid as a yellow powder 0.6 g (32%) mp > 250°.

Mass and n.m.r. spectra confirmed the structure.

(h) Sodium 6,9-dihydro-4,6-dioxo-9-phenylmethyl-10-propyl-4H-pyrano[3,2-g]quinoline-2-carboxylate

6,9-Dihydro-4,6-dioxo-9-phenylmethyl-10-propyl-4H-pyrano[3,2-g]quinoline-2-carboxylic acid (0.4237 g, 1.09 mmol) was dissolved in a solution of sodium bicarbonate (0.0915 g, 0.00109 M) in water (50 ml). The resulting solution was filtered and freeze-dried. The solid obtained was dried *in vacuo* at 80° to give the required sodium salt as a yellow powder, 0.43 g (96%) m.p. > 250°.

| | | | |
|---|---|---|---|
| Found: | C, 59.6; | H, 4.03; | N, 3.14% |
| $C_{28}H_{18}NNaO_5$ requires with 10.75% water: | C, 59.9; | | N, 3.04% |

Example 19

9-Ethyl-7,9-dihydro-6-methoxyimino-4-oxo-10-propyl-4H,6H-pyrano[3,2-g]quinoline-2,8-dicarboxylic acid

(a) Diethyl 9-ethyl-7,9-dihydro-6-methoxyimino-4-oxo-10-propyl-4H,6H-pyrano[3,2-g]quinoline-2,8-dicarboxylic acid

Methoxamine hydrochloride (3.9 g) in ethanol (100 ml) was added dropwise with stirring to a refluxing suspension of diethyl 9-ethyl-7,9-dihydro-6-methoxyimino-4-oxo-10-propyl-4H,6H-pyrano[3,2-g]quinoline-2,8-dicarboxylate (20 g) in ethanol (600 ml). After one hour, the suspension was cooled, evaporated and the resulting solid purified by preparative high performance liquid chromatography (HPLC), first using 30% ethyl acetate:70% petrol (bp 60—80°), as eluant, then dichloromethane:ethyl acetate 25:1, to give the sub-title product (2.5 g) mp 205—7°.

(b) Disodium 9-ethyl-7,9-dihydro-6-methoxyimino-4-oxo-10-propyl-4H,6H-pyrano[3,2-g]quinoline-2,8-dicarboxylate

Sodium hydroxide solution (0.1 M, 105 ml) was added dropwise over 1.25 hour to a refluxing solution of the diester of step (a) (2.45 g) in methanol (350 ml). The solution was then cooled, evaporated, the resulting solid dissolved in water and freeze-dried to give the disodium salt of the title compound (1.79 g).

| | | | |
|---|---|---|---|
| $C_{20}H_{18}N_2Na_2O_7.4.5H_2O$ requires: | C, 45.72; | H, 5.17; | N, 5.33%; |
| Found: | C, 45.56; | H, 4.57; | N, 5.35%; |

Example 20

6-Methoxyimino-4-oxo-10-propyl-4H,6H-benzo[1,2-b:5,4-b']dipyran-2,8-dicarboxylic acid

a) Diethyl 6-methoxyimino-4-oxo-10-propyl-4H,6H-benzo[1,2-b:5,4-b']dipyran-2,8-dicarboxylate

A mixture of diethyl 4,6-dioxo-10-propyl-4H,6H-benzo[1,2-b:5,4-b']dipyran-2,8-dicarboxylate (2.0 g, 5.0 mmole) and methoxyamine hydrochloride (0.42 g, 5 mmole) in dry ethanol (40 ml) was heated under reflux with stirring during 2 hours. After cooling to room temperature, a yellow crystalline solid was collected by filtration and air-dried. Recrystallisation from ethanol, followed by column chromatography on silica (100 g), eluting with a mixture of 3:1 petroleum ether-ether, and finally recrystallisation from 60—80° petroleum ether afforded the sub-title compound as a pale yellow crystalline solid (0.22 g, 21%), mp 175—9°.

| | | | |
|---|---|---|---|
| $C_{22}H_{23}NO_8$ requires: | C, 61.53%; | H, 5.40%; | N, 3.26%; |
| Found: | C, 61,44%; | H, 5.39%; | N, 3.40% |

b) Disodium 6-methoxyimino-4-oxo-10-propyl-4H,6H-benzo[1,2-b:5,4-b']dipyran-2,8-dicarboxylate

An aqueous solution of sodium hydroxide (10.1 ml, 1.0 M) was added dropwise over 3.75 hours to a stirred solution of the product of step a) (2.20 g, 5.12 mmole) in pure methanol (150 ml), maintained at reflux temperature. The resulting solution was filtered and evaporated to afford a semi-solid residue, which was taken into a minimum of water (9 ml) and added to stirred pure acetone (1500 ml). The product was collected by filtration and dried *in vacuo* at 60° to afford the required disodium salt as a pale yellow solid (2.08 g, 85%).

| | | | |
|---|---|---|---|
| $C_{18}H_{13}NNa_2O_8.3.5\ H_2O$ requires: | C, 45.00%; | H, 2.91%; | N, 13.12%; |
| Found: | C, 44.71%; | H, 2.95%; | N, 13.51%; |

Example 21

6-(N-Methyl-N-acetylamino)-4-oxo-10-propyl-4H-pyrano[3,2-g]quinoline-2,8-dicarboxylic acid

(a) Diethyl 6-(N-methyl-N-acetylamino)-4-oxo-10-propyl-4H-pyrano[3,2-g]quinoline-2,8-dicarboxylate

A solution of diethyl 6-(N-methylamino)-4-oxo-10-propyl-4H-pyrano[3,2-g]quinoline-2,8-dicarboxylate (1.25 g) in acetic acid (25 ml) and acetic anhydride (25 ml) was refluxed for 12 hours, then the solution was poured into water, extracted with ethyl acetate and the extract washed with sodium bicarbonate solution, dried and evaporated to give an oil. The oil crystallised from ethanol to give the required amide, 0.56 g (41%) mp 164—5°C.

| | | | |
|---|---|---|---|
| Found: | C, 63.77; | H, 6.00; | N, 6.42%; |
| $C_{24}H_{26}N_2O_7$ requires: | C, 63.42; | H, 5.75; | N, 6.17%; |

(b) Disodium 6-(N-methyl-N-acetylamino)-4-oxo-10-propyl-4H-pyrano[3,2-g]quinoline-2,8-dicarboxylate

0.10M Sodium hydroxide solution (21.85 ml) was slowly added to a stirred refluxing solution of diethyl 6-(N-methyl-N-acetylamino)-4-oxo-10-propyl-4H-pyrano[3,2-g]quinoline-2,8-dicarboxylate (0.497 g), in ethanol (50 ml). After 2.5 hours the solvent was evaporated and water was added (30 ml). The disodium salt was precipitated by the addition of acetone (1 l.) filtered off and redissolved in a little water. The solution was freeze-dried to give the disodium salt of the title compound as a light green fluffy solid 0.39 g (73%).

| | | | |
|---|---|---|---|
| Found: | C, 47.88; | H, 3.59; | N, 5.52%; |
| $C_{20}H_{16}N_2Na_2O_7$. 11.8% water required: | C, 47.88; | H, 4.50; | N, 5.58%; |

Example 22

6-Cyano-4-oxo-10-propyl-4H-pyrano[3,2-g]quinoline-2,8-dicarboxylic acid

a) 2-(4-Acetyl-3-hydroxy-2-propylphenylamino)-4-oxo-1-pentanoic acid

4-Acetyl-3-hydroxy-2-propylaniline (38.9 g) and 4-oxo-1-pentanoic acid (21 g) were finely powdered together then heated on a steam bath until a melt was obtained. After 15 minutes further heating, the mass solidified. The mass was then cooled, triturated with ether and the powdered product was collected and dried to yield the sub-title compound 22.6 g, mp 145—147°.

b) Ethyl 6-acetyl-7-hydroxy-4-methyl-8-propylquinoline-2-carboxylate

Polyphosphoric acid (200 ml) was heated with stirring on a steam bath and the pentanoic acid derivative of step (a) (finely powdered, 22.0 g) added in portions to the hot acid. After about 45 minutes further heating and stirring the warm mixture was poured into water (3 l) and stirred vigorously for 10 minutes. Ethyl acetate extraction, drying of the organic fraction and evaporation gave a gum. The gum was suspended in ethanolic HCl and refluxed for 1 hour. Evaporation gave the sub-title compound, 8.0 g, mp 154—155° from propan-2-ol.

c) Diethyl 6-methyl-4-oxo-10-propyl-4H-pyrano[3,2-g]quinoline-2,8-dicarboxylate

Sodium (2.925 g) was dissolved in dry ethanol (380 ml) and the product of step (b) (8 g) was added together with diethyl oxalate (8.7 ml). After 2 hours at reflux, the mixture was cooled and acidified with gaseous HCl, until the suspension was yellow and the pH about 2. The mixture was then refluxed 30 minutes, cooled and evaporated. The residue obtained was washed with dilute sodium bicarbonate solution, dried to give the sub-title compound, 7.8 g from ethanol, mp 183—4°.

d) Diethyl 6-cyano-4-oxo-10-propyl-4H-pyrano[3,2-g]quinoline-2,8-dicarboxylate

The diester from step (c) (3.97 g), selenium dioxide (4.0 ml) and glacial acetic acid (200 ml) were heated on a steam bath for 2 hours with stirring, to give a solution. The cooled solution was evaporated to give a residue, extracted into chloroform, filtered and the filtrate evaporated to give 6.3 g of the aldehyde as an orange solid.

The crude aldehyde (6.3 g) was dissolved in formic acid (99%, 40 ml), treated with sodium formate (1.12 g) and hydroxylamine hydrochloride (11 ml) and stirred on a steam bath for 6 hours. A further portion of hydroxylamine hydrochloride (0.38 g) and sodium formate (0.56 g) was added and the reaction heated for a further 3 hours. The reaction mixture was cooled, poured into water and the sub-title compound isolated as a green-yellow solid, 1.15 g from ethanol, mp 207—209°.

e) Disodium 6-cyano-4-oxo-10-propyl-4H-pyrano[3,2-g]quinoline-2,8-dicarboxylate

Sodium hydroxide solution (0.2 M, 24.1 ml) was added dropwise to a suspension of the diester from step (d) (0.9837 g) suspended in refluxing methanol (50 ml), over a period of 3 hours. The mixture was refluxed for a further 2 hours, cooled and evaporated to a residue. The residue was dissolved in water (5 ml), precipitated with acetone, the precipitate redissolved in water (15 ml) and the resulting solution freeze-dried to give the disodium salt of the title compound as a green powder, 0.4 g.

Nmr ($D_2O$) δ, 0.9 (3H, t), 1.8 (2H, m), 3.5 (2H, t), 6.9 (1H, s). 8.4 (2H, s).

Example 23

5-Oxo-9-propyl-5H-furo[3,2-g][1]-benzopyran-2,7-dicarboxylic acid

(a) 1-[2-Hydroxy-4-(2-propenyloxy)-3-propylphenyl]ethanone

1-(2,4-Dihydroxy-3-propylphenyl)ethanone (19.4 g), allyl bromide (10.5 g), and anhydrous potassium carbonate (15.2 g) were stirred in dry acetone (200 ml) under reflux for 17 hours. The reaction mixture was cooled, poured into water and the precipitated product extracted with ether, which was washed with water and dried. The solvent was evaporated to leave an orange solid which was recrystallised from 30—40° petroleum ether to give the sub-title product as 19.9 g of orange needles, m.p. 47—49°.

Analysis:

| | | |
|---|---|---|
| Found: | C, 71.85%; | H, 7.70%; |
| $C_{14}H_{18}O_3$ Requires: | C, 71.8%; | H, 7.69%; |

(b) 1-[2,4-Dihydroxy-5-(2-propenyl)-3-propylphenyl]ethanone

The product of step (a) (16.6 g) in N,N-diethylbenzeneamine (120 ml) was heated at reflux for 5 hours. The solvent was evaporated under reduced pressure and the residue, which solidified on cooling, was recrystallised from 60—80° petroleum ether to give 14.3 g of the sub-title product as fluffy needles, mp 87—88°.

Analysis:

| | | |
|---|---|---|
| Found: | C, 71.84%; | H, 7.78%; |
| $C_{14}H_{18}O_3$ Required: | C, 71.8%; | H, 7.69%; |

(c) E & Z-1-[2,4-Dihydroxy-5-(1-propenyl)-3-propyl)phenyl ethanone

The product of step (b) (12 g) and potassium *t*-butoxide (18.7 g) were stirred at room temperature in dry dimethylsulphoxide (200 ml) under nitrogen for 17 hours at room temperature and then 30—35° in an oil bath for 40 hours. The reaction mixture was poured into water, acidified and extracted with ether. The ethereal extract was washed well with water and dried. The solvent was evaporated to leave 12 g of residue, which was shown to be a mixture of the desired E & Z isomers by nmr spectroscopy.

The two isomers were separated by high pressure liquid chromatography to give the E isomer, mp 114—116°.

Analysis:

| | | |
|---|---|---|
| Found: | C, 71.95%; | H, 7.9%; |
| $C_{14}H_{18}O_3$ Requires: | C, 71.8%; | H, 7.69%; |

The Z isomer had mp 58—60°

Analysis:

| | | |
|---|---|---|
| Found: | C, 71.5%; | H, 7.71%; |
| $C_{14}H_{18}O_3$ Requires: | C, 71.8% | H, 7.69%; |

(d) Ethyl 7-hydroxy-4-oxo-6-(1-propenyl)-8-propyl-4H-1)benzopyran-2-carboxylate (mixture of E & Z isomers)

The mixture of E and Z isomers from step (c) above (1.17 g) and diethyl oxalate (1.7 ml) were dissolved in dry dimethylformamide (20 ml) and added to ether washed 50% sodium hydride (0.96%) suspended in dry dimethylformamide (20 ml) with stirring under nitrogen. The reaction mixture was stirred for 4 hours at

room temperature, poured into dilute hydrochloric acid and extracted with ethyl acetate, which was then washed with water and dried. The solvent was evaporated, the residue treated with ethanol previously saturated with hydrogen chloride gas, and left to stand for 10 min. The reaction mixture was poured into water and the precipitated product collected by filtration, and dried to give 1.38 g of product, mp 182—185°.

Analysis:

| | | |
|---|---|---|
| Found: | C, 67.9%; | H, 6.47%; |
| $C_{18}H_{20}O_5$ Requires: | C, 68.4%; | H, 6.33%; |

(e) Ethyl 6-formyl-7-hydroxy-4-oxo-8-propyl-4H-1-benzopyran-2-carboxylate

The mixture of isomers from step (d) (6.3 g) was dissolved in dry ethanol (400 ml) and cooled to −60°C to precipitate the starting materials as a fine solid. The suspension was treated with ozonised oxygen gas at a rate of 25 1/hour for 1 hour with stirring. The reaction mixture, while still at −60°C, was flushed with nitrogen for 15 mins and dimethyl sulphide (2 ml) was added. The reaction mixture was stirred at −10°C for 1 hour, ice-bath temperature for 1 h and finally room temperature for 1 hour, poured into water, and extracted with ethyl acetate which was washed with water and dried. The solvent was evaporated and the residue recrystallised from ethanol to give 3.8 g of product, mp 137—138°.

Analysis:

| | | |
|---|---|---|
| Found: | C, 62.8%; | H, 5.4%; |
| $C_{16}H_{16}O_6$ Requires: | C, 63.2%; | H, 5.26%; |

(f) Triethyl 2,3-dihydro-3-hydroxy-5-oxo-9-propyl-5H-furo[3,2-g]-[1]-benzopyran-2,2,7-tricarboxylate

The product from step (f) (2.6 g), diethyl bromomalonate (2.28 g) and potassium carbonate (1.75 g) were heated under reflux in methyl ethyl ketone (65 ml) with stirring for 0.75 hour. The reaction mixture was cooled, poured into water and extracted with ethyl acetate, which was then washed with brine and dried. The solvent was evaporated and the residue triturated with 30—40° petroleum ether to give 3.2 g of product. A recrystallisation from aqueous ethanol gave material, mp 131—133°.

Analysis:

| | | |
|---|---|---|
| Found: | C, 60.0%; | H, 5.49%; |
| $C_{23}H_{26}O_{10}$ Requires: | C, 59.7%; | H, 5.63%; |

(g) 5-Oxo-9-propyl-5H-furo[3,2-g][1]-benzopyran-2,7-dicarboxylic acid

The product of step (f) (2.7 g) was heated under reflux in concentrated hydrochloric acid (20 ml), water (20 ml) and glacial acetic acid (50 ml) for 6 hours with stirring. The reaction mixture was allowed to cool overnight and the solid which had precipitated was collected by filtration. A recrystallisation from glacial acetic acid gave 1.64 g of product, mp 316—318° dec.

Analysis:

| | | |
|---|---|---|
| Found: | C, 58.15%; | H, 3.8%; |
| $C_{16}H_{12}O_7$ 0.75 $H_2O$ Requires: | C, 58.2%; | H, 3.77%; |

(h) Disodium 5-oxo-9-propyl-5H-furo[3,2-g][1]-benzopyran-2,7-dicarboxylate

The product from step (g) (1.0356 g) and sodium bicarbonate (0.528 g) in water (200 ml) were stirred until a clear solution was formed. The solution was filtered and the filtrate freeze-dried to give 1.13 g of pale yellow solid.

Analysis:

| | | |
|---|---|---|
| Found: | C, 48.57%; | H, 2.94%; |
| $C_{16}H_{10}Na_2O_7$ Requires: | C, 48.22%; | H, 3.0% |
| | 9.53% water | |

Example 24

Disodium 5-oxo-9-propyl-5H-thieno[3,2-g][1]-benzopyran-2,7-dicarboxylate

(a) O-[4-Acetyl-3-hydroxy-6-(1-propenyl)-2-propylphenyl]-N,N-dimethylthiocarbamate (mixture of E & Z isomers)

E & Z-1-[2,4-Dihydroxy-5-(1-propenyl)-3-propylphenyl]ethanone (19.35 g), potassium carbonate (13.2 g) and dimethylthiocarbamoyl chloride (11.2 g) were heated under reflux in dry acetone (155 ml) for 6 hours. The reaction mixture was cooled, poured into water and extracted into ether, which was washed with brine and then dried. The solvent was evaporated and the residue eluted down a silica gel column

using ether/petroleum ether (2:3) as eluant to give the product as 24.8 g of an oily solid.

Analysis:

| | |
|---|---|
| Found: | C, 63.54%; H, 7.17%; N, 4.33%; S, 10.1%; |
| $C_{17}H_{23}NO_3S$ Requires: | C, 63.6% H, 7.17%; N, 4.36%; S, 9.97%; |

(b) S-[4-Acetyl-3-hydroxy-6-(1-propenyl)-2-propylphenyl]-N,N-dimethylthiocarbamate (mixture of E & Z isomers)

The product of step (a) (5.0 g) was added to diphenyl ether (70 ml) under reflux with stirring. The reaction mixture was heated under reflux with stirring for 5 mins and the excess diphenyl ether evaporated under reduced pressure. The residue was eluted down a silica gel column using ether/petroleum ether (1:1) as eluant to give the sub-title compound, 4.0 g as a viscous oil.

Analysis:

| | |
|---|---|
| Found: | C, 63.68%; H, 7.35%; N, 4.63%; S, 9.7%; |
| $C_{17}H_{23}NO_3S$ Requires: | C, 63.6%; H, 7.17%; N, 4.86%; S, 9.97%; |

(c) S-(4-Acetyl-6-formyl-3-hydroxy-2-propylphenyl)-N,N-dimethylthiocarbamate

The product from step (b) (10 g) was dissolved in dry ethanol (500 ml) and cooled to −60°. Ozonised oxygen gas was passed through the solution at a rate of 25.5/hours from an ozonator whilst allowing the temperature to rise to −30 to −40°. The reaction mixture was flushed with nitrogen at −40° and then dimethyl sulphide (3.1 ml) was added. The reaction mixture was stirred at −10° for 1 hour, 0°C for 1 hour and finally room temperature for 1 hour. The solvent was evaporated to dryness to give a gum which was triturated with 30—40°C petroleum ether and cooled to 0°C to give 7.5 g of solid. A further 1.44 g was obtained by evaporating the petroleum ether washings and eluting the residue down a silica gel column using petroleum ether/ethyl acetate (7:3) as eluant, mp 70—72°C.

Analysis:

| | |
|---|---|
| Found: | C, 58.37%; H, 6.46%; N, 4.40%; S, 10.4%; |
| $C_{15}H_{19}NO_4S$ Requires: | C, 58.3%; H, 6.15%; N, 4.53%; S, 10.4% |

(d) Ethyl 5-acetyl-6-hydroxy-7-propylbenzo[b]thiophene-2-carboxylate

The product from step (c) (2 g) and sodium hydroxide (1.3 g) in water (50 ml) were heated at reflux under nitrogen for 2 hours. The reaction mixture was cooled, poured into water, acidified and extracted with ethyl acetate, which was washed well with water and dried. The solvent was evaporated to leave 5-acetyl-4-hydroxy-2-mercapto-3-propyl benzaldehyde (structure confirmed by nmr and ms investigation).

The thiol was dissolved in dry ethyl methyl ketone (30 ml) and potassium carbonate (1.20 g) and diethyl bromomalonate (1.23 ml) added. The reaction mixture was heated under reflux for 0.5 hours under nitrogen. The reaction mixture was then poured into water and extracted with ethyl acetate which was washed with brine twice and dried. The solvent was evaporated to give diethyl 5-acetyl-2,3-dihydro-3,6-dihydroxy-7-propylbenzo[b]thiophene-2,2-dicarboxylate which was confirmed by nmr and ms examination.

The dihydrobenzothiophene was heated under reflux with potassium hydroxide (1.29 g) in ethanol (45 ml) for 15 min. The reaction mixture was poured onto ice, acidified and extracted with ethyl acetate which was washed with water twice, saturated sodium bicarbonate solution twice, then brine and dried. The solvent was evaporated to leave 1.5 g of residue which was eluted down a silica gel column using ether/petroleum ether (3:2) as eluant to give 1.1 g of the sub-title product, mp 88—91°.

Analysis:

| | |
|---|---|
| Found: | C, 62.77%; H, 5.98%; S, 10.1%; |
| $C_{16}H_{18}O_4S$ Requires: | C, 62.7%; H, 5.88%; S, 10.5%; |

(e) Diethyl 5-oxo-9-propyl-5H-thieno[3,2-g][1]benzopyran-2,7-dicarboxylate

The product from step (d) (0.5 g) and diethyl oxalate (0.55 ml) dissolved in dry ethanol (10 ml) were added dropwise to sodium (0.15 g) in dry ethanol (20 ml) with stirring. The reaction mixture was heated under reflux and stirred for 1.5 hours, cooled, poured into a mixture of dilute hydrochloric acid and chloroform and the organic layer separated, washed with water and dried. The solvent was evaporated and the residue treated with hydrogen chloride gas in ethanol (30 ml) and heated under reflux for 15 min. The reaction mixture was cooled, poured into water and extracted with ethyl acetate which was washed with water and dried. The solvent was evaporated and the residue eluted through a silica gel pad using ethyl acetate as solvent to give 0.53 g of product which was recrystallised from ethanol to give the sub-title compound (0.34 g) as golden needles, mp 148—149°.

Analysis:

| | |
|---|---|
| Found: | C, 61.85%; H, 5.22%; |
| $C_{20}H_{20}O_6S$ Requires: | C, 61.9%; H, 5.15%; |

f) 5-Oxo-9-propyl-5H-thieno[3,2-g]benzopyran-2,7-dicarboxylic acid

The product from step e) (1.9 g, 4.92 mmol) was dissolved in glacial acetic acid (70 ml), treated with concentrated hydrochloric acid (13 ml), water (13 ml) and heated under reflux for 8 hours. The reaction mixture was allowed to cool to room temperature overnight, concentrated hydrochloric acid (10 ml) was added and refluxing continued for 2 hours. The reaction was cooled, poured into water (200 ml) and the product collected by filtration, washed well with water and dried. This crude diacid was treated with sodium bicarbonate (0.9 g) in water (200 ml) and the solution was treated with charcoal, filtered and the filtrate acidified. The gelatinous product was collected by filtration, washed well with water and dried to give 1.45 g (89%) of the title diacid, mp 310° dec.

g) Disodium 5-Oxo-9-propyl-5H-thieno[3,2-g]benzopyran-2,7-dicarboxylate

The product of step f) (1.0588 g, 3.16 mmol) was added to a solution of sodium bicarbonate (0.531 g, 6.32 mol) in water (100 ml) and stirred until dissolved. The solution was filtered and the filtrate freeze-dried to give 1.126 g of disodium salt after drying in vacuum at 80° for 3 hours.

Analysis:

| | | | |
|---|---|---|---|
| calcd. for $C_{16}H_{10}Na_2O_6$.21.8%$H_2O$: | C, 40.0; | H, 4.5; | S, 6.68%; |
| Found: | C, 40.5; | H, 4.29; | S, 6.59%; |

## Example 25

## 4-Oxo-9-propyl-4H,6H-pyrano[2,3-f]indole-2,7-dicarboxylic acid

a) Ethyl 5-hydroxy-4-propyl-1H-indole-2-carboxylate

Ethyl 5-hydroxy-4-(2-propenyl)-1H-indole-2-carboxylate (2.16 g) was dissolved in dry ethanol (100 ml) and hydrogenated at atmospheric pressure in the presence of 5% palladium on charcoal (0.2 g) until hydrogen uptake had ceased. The catalyst was filtered off, washed with hot ethanol, the filtrate evaporated and the residue recrystallised from aqueous ethanol to give 1.616 g of the desired product mp 185—186°:

Elemental analysis:

| | | | |
|---|---|---|---|
| Found: | C, 68.07; | H, 6.96; | N, 5.48%; |
| $C_{14}H_{17}NO_3$ Required: | C, 68.0; | H, 6.88; | N, 5.67%; |

b) 2-(2-Carboxy-4-propyl-1H-indole-5-yloxy)but-2-ene-1,4-dioic acid

The product of step a) (5.0 g) and dimethyl acetylenedicarboxylate (2.72 ml) were heated under reflux in ethanol (56 ml) with benzyl trimethylammonium hydroxide (2 drops) for 8 hours and left to stand at room temperature overnight. The reaction mixture was poured into water (100 ml), extracted with ethyl acetate (2 × 100 ml), washed with saturated brine solution (2 × 100 ml), and dried. The solvent was evaporated, and the residue was triturated with petroleum ether which was decanted off. The petroleum ether insoluble material was dissolved in ethanol (55 ml) and treated with sodium hydroxide (4.83 g) in water (55 ml). The resulting solution was heated under reflux for 2 hours, cooled, poured into water (100 ml), acidified, extracted with ethyl acetate (2 × 100 ml), washed with saturated brine (2 × 100 ml) and dried. The solvent was evaporated and the residue triturated with petroleum ether to give 6.3 g of the desired product as a mixture of E and Z acids.

c) Diethyl 4-oxo-9-propyl-4H,6H-pyrano[2,3-f]indole-2,7-dicarboxylate

The mixture of E and Z acids from step b) (5.3 g) was treated with polyphosphoric acid (130 ml) and heated on the steam bath with stirring for 1 hour. The reaction mixture was cooled, poured onto ice-water (400 ml), stirred for 3 hours and the product collected by filtration, washed with water and dried to give the crude ring-closed diacid. This was treated with a saturated solution of hydrogen chloride gas in ethanol (150 ml) and heated under reflux for 2 hours. The solution was poured into water (300 ml) and the product which precipitated was collected by filtration and dried. Trituration with ethyl acetate gave 1.35 g of the required diester as an insoluble yellow powder. The ethyl acetate was evaporated and the residue eluted down a silica gel column using ether/petroleum ether (7.3) as eluant to give a further 0.5 g of the desired product. A sample was recrystallised from ethanol to give material mp 227—228°.

| | | | |
|---|---|---|---|
| Found: | C, 64.82; | H, 5.64; | N, 3.66%; |
| $C_{20}H_{21}NO_6$ Required: | C, 64.7; | H, 5.66; | N, 3.77%; |

d) Disodium 4-oxo-9-propyl-4H,6H-pyrano[2,3-f]indole-2,7-dicarboxylate

A solution of the diester from step c) (1.3 g) in glacial acetic acid (40 ml) and concentrated hydrochloric acid (13 ml) was heated under reflux for 16 hours. More concentrated hydrochloric acid (3 ml) was added and the solution heated under reflux for a further 8 hours. The reaction mixture was allowed to cool overnight and the insoluble product collected by filtration, washed with a little glacial acetic acid, then petroleum ether and dried at 100° *in vacuo* for 4 hours to give 1.008 g of the diacid, which contained a trace of monoester as shown by NMR and MS. The crude diacid was treated with sodium bicarbonate (0.537 g) in water (30 ml), the resulting solution filtered, and the filtrate treated with acetone until there was complete precipitation of the disodium salt. The salt was collected by filtration and dried to give 0.825 g of the desired

product. The disodium salt (0.52 g) was dissolved in water (30 ml) and freeze-dried to give 0.497 g of the disodium salt of the title compound.

| | | | | |
|---|---|---|---|---|
| Found: | C, 44.0; | H, 3.0; | N, 3.1; | Na, 11.2; |
| $C_{16}H_{11}Na_2NO_6$17%$H_2O$ Required: | C, 44.4; | | N, 3.66; | Na, 10.0; |

Thermogravimetric analysis showed 16.3% weight loss at 290°.

Example 26

8-Oxo-4-propyl-8H-furo[2,3-g][1]benzopyran-2,6-dicarboxylic acid

a) Ethyl 5-hydroxybenzofuran-2-carboxylate

5-Hydroxybenzofuran-2-carboxylic acid (1.1 g) was heated under reflux for 1 hour in ethanol (50 ml) saturated with hydrogen chloride gas. The reaction mixture was cooled, poured into water and extracted with ethyl acetate, which was washed with water and dried over magnesium sulphate. The solvent was evaporated and the residue triturated with petroleum ether to give a brown powder, which was treated with ether, filtered from a black tar and the filtrate eluted down a silica gel column using ether as eluant to give 0.78 g of the sub-title ester as a colourless solid mp 110—112°.

Elemental analysis:

| | | |
|---|---|---|
| Found: | C, 64.0; | H, 5.0%; |
| $C_{11}H_{10}O_4$ Required: | C, 64.1; | H, 4.85%; |

b) Ethyl 5-(2-propenyloxy)benzofuran-2-carboxylate

The ester from step a) (9.2 g), anhydrous potassium carbonate (7.43 g) and allyl bromide (6.13 g) were heated under reflux in dry acetone (200 ml) with stirring for 18 hours. The reaction mixture was cooled, poured into water, and extracted with ethyl acetate which was washed with water and dried over magnesium sulphate. The solvent was evaporated to leave a dark oil which was triturated with boiling 40—60 petroleum ether, decanted from a black tar and cooled to less than 0° to give a sticky solid, which was dissolved in ether, washed with 10% sodium hydroxide solution and dried over magnesium sulphate. The solvent was evaporated to give 8.3 g of the sub-title compound as a yellow solid mp 34—36°.

Elemental analysis:

| | | |
|---|---|---|
| Found: | C, 68.38; | H, 5.63%; |
| $C_{14}H_{14}O_4$ Required: | C, 68.29; | H, 5.69%; |

c) Ethyl 5-hydroxy-4-(2-propenyl)benzofuran-2-carboxylate

The allyl ether from step b) (1.0 g) was heated at 190—200°C for 2 hours under nitrogen. The flask was cooled and the product was powdered in a mortar under petroleum ether (40—60°) and collection by filtration to give 0.9 g of the sub-title compound, mp 94—95°.

Elemental analysis:

| | | |
|---|---|---|
| Found: | C, 68.45; | H, 5.6%; |
| $C_{14}H_{14}O_4$ Required: | C, 68.3; | H, 5.69%; |

d) Ethyl 5-hydroxy-4-propylbenzofuran-2-carboxylate

The propenyl compound from step c) (6.8 g) was dissolved in dry ethanol (150 ml) and hydrogenated at 45 psi in the presence of 5% palladium on charcoal (0.6 g) until hydrogen uptake had ceased. The catalyst was filtered off, the filtrate evaporated and the residue triturated with petroleum ether to give 5.0 g of the required product mp 99—101°.

Elemental analysis:

| | | |
|---|---|---|
| Found: | C, 67.51; | H, 6.34%; |
| $C_{14}H_{16}O_4$ Required: | C, 67.7; | H, 6.45%; |

e) E/Z-2-(2-carboxy-4-propylbenzofuran-5-yloxy)but-2-ene-1,4-dioic acid

The product of step d) (5.66 g) and dimethyl acetylenedicarboxylate (3.08 ml) were heated under reflux in ethanol (50 ml) with benzyltrimethylammonium hydroxide (a few drops) for 1 hour. The reaction mixture was cooled, sodium hydroxide (5.64 g) in water (50 ml) was added and refluxing continued for 1 hour. The reaction was cooled, diluted with water, acidified and extracted with ethyl acetate which was washed with water and dried over magnesium sulphate. The solvent was evaporated and the solid residue powdered under petroleum ether (bp 40—60°), collected by filtration and dried to give 7.3 g of the title compound as a pale yellow powder.

f) Diethyl 8-oxo-4-propyl-8H-furo[2,3-g][1]benzopyran-2,6-dicarboxylate

The product of step e) (1.3 g) was heated on the steam bath with stirring in polyphosphoric acid (40 ml)

for 1.5 hours. The reaction mixture was poured onto ice and extracted with ethyl acetate, which was washed with water and dried over magnesium sulphate. The solvent was evaporated and the residue treated with ethanol (100 ml) saturated with hydrogen chloride gas and heated under reflux for 2 hours. The reaction mixture was cooled, diluted with water and extracted with ethyl acetate which was washed with water and dried over magnesium sulphate. The solvent was evaporated to leave a sticky solid which was triturated and finally powdered under petroleum ether (bp 40—60°) to give 0.6 g of the sub-title compound as a pale yellow solid mp 145—147°

Elemental analysis:

| | | |
|---|---|---|
| Found: | C, 64.33; | H, 5.23% |
| $C_{20}H_{20}O_7$ Required: | C, 64.6; | H, 5.38%; |

g) 8-Oxo-4-propyl-8H-furo[2,3-g][1]benzopyran-2,6-dicarboxylic acid

The diester from step f) (3.0 g) was heated under reflux in a glacial acetic acid (100 ml) and concentrated hydrochloric acid (50 ml) for 16 hours. The reaction mixture was cooled, the insoluble product collected by filtration, washed with ether and dried to give 1.92 g of the title product mp 309—310° dec

Elemental analysis:

| | | |
|---|---|---|
| Found: | C, 60.43; | H, 3.9%; |
| $C_{16}H_{12}O_7$ Required: | C, 60.8; | H, 3.8%; |

h) Disodium 8-oxo-4-propyl-8H-furo[2,3-g][1]benzopyran-2,6-dicarboxylate

The diacid from step g) (1.84 g) was treated with sodium bicarbonate (0.978 g) in water (80 ml), filtered and the filtrate treated with acetone. A milky precipitate was formed which coagulated on scratching and standing. The di-sodium salt was collected by filtration, redissolved in water (80 ml) and freeze-dried to give 1.89 g of the sub-title compound as a cream coloured powder.

Elemental analysis:

| | | |
|---|---|---|
| Found: | C, 45.8; | H, 2.39—3.94%; |
| $C_{16}H_{10}Na_2O_7$.14.07% $H_2O$ Required: | C, 45.87; | H, 3.06%; |

Thermogravimetric analysis at 200° showed 14.07% water.

Example 27

6-Ethyl-4-oxo-9-propyl-4H-pyrano[2,3-f]indole-2,7-dicarboxylic acid

a) 6-Ethyl-4-oxo-9-propyl-4H-pyrano[2,3-f]indole-2,7-dicarboxylic acid

Diethyl 4-oxo-9-propyl-4H-pyrano[2,3-f]indole-2,7-dicarboxylate (0.954 g) was added to ether washed 50% sodium hydride (0.136 g) suspended in dry dimethylformamide (60 ml) with stirring under nitrogen. The reaction mixture was stirred for 0.5 hours, ethyl bromide (0.21 ml) added and stirring continued for 24 hours. The reaction was poured into water and the product which had precipitated collected by filtration, washed with water and dried to give 0.45 g of diethyl 6-ethyl-4-oxo-9-propyl-4H-pyrano[2,3-f]indole-2,7-dicarboxylate as a pale yellow solid. The structure was confirmed by nmr and ms.

The aqueous filtrate was acidified and extracted with ethyl acetate which was washed with water and dried over magnesium sulphate. The solvent was evaporated to leave 0.3 g of 7-ethyl-6-ethyl-4-oxo-9-propyl-4H-pyrano [2,3-f]indole-2,7-dicarboxylic acid. The structure was confirmed by nmr and ms.

The diethyl ester (0.57 g) and mono ester (0.34 g) were combined, dissolved in glacial acetic acid (30 ml) treated with concentrated hydrochloric acid (10 ml) and heated under reflux for 8 hours. More concentrated hydrochloric acid (8 ml) was added and refluxing continued overnight. The reaction mixture was allowed to cool to room temperature and the product which had precipitated collected by filtration, washed with a little glacial acetic acid, then petroleum ether and dried at 100° *in vacuo* to give 0.748 g of the sub-title compound as yellow needles.

b) Disodium 6-ethyl-4-oxo-9-propyl-4H-pyrano[2,3-f]indole-2,7-dicarboxylate

The diacid product of step a) (0.738 g) was treated with sodium bicarbonate (0.362 g) in water (50 ml) and stirred until complete solution was obtained. The solution was filtered and the filtrate treated with acetone until complete precipitation of the disodium salt was achieved. This was collected by filtration, dissolved in water (50 ml) and freeze-dried to give 0.648 g of the sub-title product as a yellow powder.

Elemental analysis:

| | | |
|---|---|---|
| Found: | C, 46.6; | N, 2.94 |
| $C_{18}H_{15}Na_2NO_6$ 15.8%$H_2O$ Required: | C, 46.98; | N, 3.05% |

Gravimetric analysis at 200° showed 15.8% weight loss.

Example 28

4-Oxo-9-propyl-4H,8H-pyrano[3,2-f]indole-2,7-dicarboxylic acid

a) Ethyl 6-(2-propenyloxy)-1H-indole-2-carboxylate

Ethyl 6-hydroxy-1H-indole-2-carboxylate (4.6 g, 0.02M) was heated under reflux with allylbromide (3 g, 2.2 mls, 0.025M), $K_2CO_3$(4.14 g 0.03M) and acetone (150mls) for 20 hours. On cooling the mixture was poured into brine and extracted into ethyl acetate, washed with brine, dried over magnesium sulphate and evaporated to dryness. The resulting crude brown solid was purified by extraction into hot 60°—80° petroleum ether to yield a yellow solid which was dried *in vacuo,* 4 g, 72%, mp 89—91°.

b) Ethyl 6-hydroxy-7-(2-propenyl)-1H-indole-2-carboxylate

The product of step a), (2.55 g, 10 mmol) was heated at 205°, under $N_2$, with stirring for 0.75 hour. The product obtained on cooling was powdered under petroleum ether (60—80°) to give the sub-title compound as a pale brown solid which was recrystallised from aqueous ethanol, 2.14 g (84%), mp 146—147°.

c) Ethyl 6-hydroxy-7-propyl-1H-indole-2-carboxylate

The product of step b) was dissolved in dry ethanol (350 ml), treated with 10% Pd/C (0.3 g) and hydrogenated at 35 psi at room temperature until $H_2$ uptake ceased (1.5 hours). The catalyst was filtered off and the filtrate evaporated to dryness.

The light brown product was purified by extraction into hot 60—80° petroleum ether to give the sub-title compound as a pale yellow powder 2.94 g, (84%) mp 125—127°C.

d) Dimethyl 2-(2-ethoxycarbonyl-7-propyl-1H-indol-5-yloxy-but-2-ene-1,4-dioate

The product of step c) (0.6 g, 0.0024M), dimethyl acetylene dicarboxylate (0.33mls, 0.0027M) and "Triton B" (2 drops) were heated under reflux for 6 hours in dry ethanol (10 ml). The reaction mixture was cooled, NaOH (0.6 g, 14.6 mmol) in water (6 ml) was added and refluxing continued for 1 hour. The whole was cooled, poured into dil. HCl, extracted into ethyl acetate and washed with brine before drying over $MgSO_4$ and evaporating to dryness to yield the sub-title compound as a pale yellow solid, 0.7 g, (86%).

e) Diethyl 4-oxo-9-propyl-4H,8H-pyrano[3,2-f]indole-2,7-dicarboxylate

The product of step d) (1.7 g, 5mmol) was stirred in polyphosphoric acid (15 g) on a steam bath for 1 hour. The reaction mixture was then poured onto ice and extracted with ethyl acetate, washed with brine, dried over $MgSO_4$ and evaporated to dryness. The brown solid obtained (the free di-acid corresponding to the title compound) was treated with ethanolic HCl (30 mls) and heated under reflux conditions for 1.5 hours. A pale yellow precipitate formed on cooling and was filtered off (90 mgs). The filtrate was poured into water, extracted into ethyl acetate, washed, dried and evaporated to dryness. The resulting crude brown solid was eluted down a silica gel column using ether as elutant to give a yellow solid 130 mgs. Total yield of sub-title compound 220 mg 12%.
mp 229—231°.

f) Disodium 4-oxo-9-propyl-4H,8H-pyrano[3,2-f]indole-2,7-dicarboxylate

The product of step e) (0.435 g; 12 mmol) was heated under reflux in methanol (20 ml). N/10 NaOH (23.5 ml 2.4 mmol) was added dropwise to the solution over a 35 minute period and the reaction mixture was heated for a further 25 minutes. After cooling to room temperature, the methanol was removed by evaporation and the aqueous solution was poured into acetone. The bright yellow precipitate was filtered off and dried *in vacuo.* 0.39 g (95%). mp>300°C

Example 29

5-Oxo-9-propyl-5H-thieno[3,2-g][1]benzopyran-3,7-dicarboxylic acid

a) 1-(2-Hydroxy-4-mercapto-3-propylphenyl)ethanone

i) A mixture of 2,4-dihydroxy-3-propylacetophenone (69 g, 355 mmol), anhydrous potassium carbonate (56 g, 400 mmol) and dimethylthiocarbamoyl chloride (50 g, 400 mmol) in acetone (600 ml) was heated and stirred at reflux for 3 hours. The mixture was cooled and filtered, and the filtrate was evaporated to dryness. The solid obtained was crystallised from ethanol to give the thiocarbamate as colourless prisms, 74.3 g (74%) mp 113—4°.

ii) The thiocarbamate (60 g, 213 mmol) form (i) was heated at reflux in diphenyl ether (200 ml) for 5 minutes. The diphenyl ether was removed by distillation and the residue was triturated with ethanol to give the rearranged ester 42.1 g (86%) mp 73.5°.

iii) To the product of part (ii) (28 mg, 100 mmol) in methanol (200 ml) was added a solution of sodium hydroxide (20 g, 500 mmol) in water (100 ml) and the mixture was heated at reflux under nitrogen for 2.5 hours. The reaction mixture was then acidified, and extracted with dichlormethane. The extracts were washed, dried, filtered, and the filtrate was evaporated to dryness leaving an oil which solidified on cooling, to give the sub-title compound, 21 g (100%).

b) 5-Acetyl-6-hydroxy-7-propylbenzo[b]thiophene-3-carboxylate

i) A solution of the benzenethiol product of step a) (21 g 100 mmol) in pyridine (100 ml) was stirred and

cooled at 0° under nitrogen while ethyl bromopyruvate (19.5 g, 0.10 M) was added dropwise. After the addition the mixture was stirred for 30 minutes and was then poured into a large excess of dil. HCl. The mixture was extracted with chloroform and the extracts were washed well with dil. HCl and water, and were dried ($MgSO_4$), filtered and the filtrate was evaporated to dryness leaving a red oil, 28 g, (87%).

ii) The oil from part (i) was heated in polyphosphoric acid (300 ml) at 100° overnight. The reaction was quenched with water, extracted with chloroform and the extracts were washed with aqueous sodium bicarbonate and water, dried ($MgSO_4$), filtered and the filtrate was evaporated to dryness leaving a brown oil 17.5 g (57% overall). The oil was purified by chromatography on silica eluting with toluene/ethyl acetate 3:1. The oil obtained by evaporation of the eluent solidified on cooling 12 g (39%).

c) Diethyl 5-oxo-9-propyl-5H-thieno[3,2-g]benzopyran-3,7-dicarboxylate

The ester product of step b) (6.5 g, 21 mmol) was added to a solution of freshly prepared sodium ethoxide (2.4 g sodium in ethanol 100 ml) and the mixture was stirred at room temperature for 15 minutes. Diethyl oxalate (7.75 g, 55 mmol) was added and the mixture was heated at reflux for 2.5 hours. HCl gas was bubbled through the cooled, stirred mixture after which the mixture was heated at reflux for 1 hour. The resulting mixture was poured into water and extracted with chloroform. The extracts were washed with water, dried ($MgSO_4$), filtered and the filtrate was evaporated to dryness leaving an oil. This oil was chromatographed on silica eluting with toluene/ethyl acetate (3:1). The solid obtained upon evaporation of the eluant was crystallised from ethanol to give the required diester 1.0 g (12%). mp 142—3°.

Analysis:

| | |
|---|---|
| Found: | C, 61.4; H, 5.25% |
| $C_{20}H_{20}O_6$ Requires: | C, 61.85; H, 5.15% |

d) 5-Oxo-9-propyl-5H-thieno[3,2-g]benzopyran-3,7-dicarboxylic acid

The product of step c) (1.0 g, 2.57 mmol) was heated in methanol (20 ml), while N/10 aqueous sodium hydroxide solution (51.55 ml) was added. The mixture was heated at reflux for 2.5 hours. Methanol was removed *in vacuo* and the aqueous solution was acidified. The solid produced was filtered off, washed well with water and was dried to give the required diacid 0.85 g (100%) mp>250°.

e) Disodium 5-Oxo-9-propyl-5H-thieno[3,2-g]benzopyran-3,7-dicarboxylate

The product of step d) (0.825 g, 2.485 mmol) was added to a solution of sodium hydrogen carbonate (0.4175 g, 4.97 mmol) in water (25 ml). The resulting solution was filtered through a glass filter and freeze-dried. The solid obtained was dried *in vacuo* to give the disodium salt of the title compound, 0.85 g (93%).

Analysis:

| | |
|---|---|
| Found: | C, 43.79; H, 2.3%; S, 7.79% |
| $C_{16}H_{10}N_aO_6S$ Requires: | with 3.5 moles $H_2O$ |
| | C, 43.73; H, 2.8%; S, 7.3% |

**Claims for the Contracting States: BE CH DE FR GB IT LI LU NL SE**

1. A benzopyran of formula I,

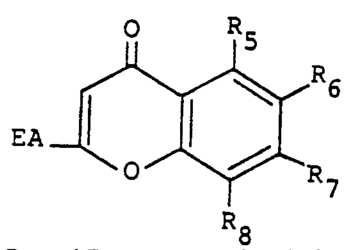

I

wherein an adjacent pair of $R_5$, $R_6$, $R_7$ and $R_8$ represent the chain $—X—CR_{12}R_{13}—CR_{14}R_{15}—Y—$ in which the chain is substituted by $—A_1E_1$,

X represents $CR_{10}R_{11}$ or a single bond,

Y represents O,S, $NR_{20}$ or $CH_2$, and (a) $R_{15}$ and $R_{20}$ together form a single bond and optionally $R_{10}$ and $R_{12}$ together form a single bond, or (b) $R_{12}$ and $R_{14}$ together form a single bond, or (c) $R_{10}$, $R_{12}$ and $R_{14}$ each represent hydrogen; and

the remainder of $R_{10}$, $R_{11}$, $R_{12}$, $R_{13}$, $R_{14}$ and $R_{15}$, which may be the same or different, independently represent hydrogen, alkyl Cl to 6, $NR_{21}COR_{22}$, CN or $C_nH_xF_{(2n+1-x)}$; in addition, $R_{10}$ and $R_{11}$, together with the carbon atom to which they are attached, may represent C=O or $C=N—OR_{23}$; also $R_{13}$ and $R_{15}$ may form the chain —CH=CH—CH=CH— which is substituted by $A_1E_1$,

A and $A_1$, which may be the same or different, represent a single bond, $(CH_2)_m$ or phenylene,

n represents an integer from 1 to 4 inclusive,

x represents 0 or an integer from 1 to 2n inclusive,

m represents an integer from 1 to 4 inclusive,

when $R_{20}$ does not form a single bond with $R_{14}$, $R_{20}$ may represent hydrogen, alkyl C1 to 6 or alkyl C1 to 6 substituted by phenyl,

$R_{21}$, $R_{22}$, and $R_{23}$, which may be the same or different, independently represent hydrogen or alkyl C1 to 6;

the remainder of $R_5$, $R_6$, $R_7$ and $R_8$, which may be the same or different, each independently represent hydrogen, hydroxy or alkyl C1 to 6,

E and $E_1$, which may be the same or different, independently represent —COOH, or 5H-tetrazolyl, provided that when the chain $—X—CR_{12}R_{13}=CR_{14}R_{15}—Y$

i) represents a chain $—CO—CR_{13}=CR_{15}—Y—$ in which Y is O or S and A and $A_1$ each represent a single bond, then Y is bonded to $R_7$, $R_5$ and $R_{13}$ each represent hydrogen, $R_8$ represents propyl and $R_{15}$ represents $E_1$, in which $E_1$, instead of being as defined above, represents 5H-tetrazolyl or $CONR_{24}R_{25}$, wherein $R_{24}$ and $R_{25}$, which may be the same or different, represent hydrogen or alkyl C1 to 6;

ii) represents a chain $—CO—CR_{13}=CR_{15}—NR_{20}—$, in which one of $R_{13}$ and $R_{15}$ represents $E_1$, then $E_1$, instead of being as defined above, represents hydrogen, and

iii) represents a chain $—CR_{11}=CR_{13}—CR_{15}=N—$, then at least one of $R_{11}$, $R_{13}$ or $R_{15}$, which may be the same or different, represents $NR_{21}COR_{22}$, CN or $C_nH_xF_{(2n+1-x)}$, and pharmaceutically acceptable salts thereof and when any one of E or $E_1$ represents a carboxylic acid group, pharmaceutically acceptable esters or amides of the carboxylic acid groups.

2. A benzopyran according to Claim 1, wherein the chain $—X—CR_{12}R_{13}—CR_{14}R_{15}—Y—$ is formed between $R_6$ and $R_7$.

3. A benzopyran according to Claim 1 or Claim 2, wherein Y represents —O—.

4. A benzopyran according to any one of the preceding claims, wherein

an adjacent pair of $R_5$, $R_6$, $R_7$ and $R_8$ represents the chain $—CO—CR_{12}R_{13}CR_{14}R_{15}—O—$,

$R_{12}$ and $R_{14}$ together form a single bond,

one of $R_{13}$ and $R_{15}$ represents $A_1E_1$ and the other represents hydrogen, or $R_{13}$ and $R_{15}$ together form a chain —CH=CH—CH=CH—, the chain being substituted by $A_1E_1$,

A represents a single bond,

$A_1$ represents a single bond, phenylene or $(CH_2)_m$ and E, $E_1$, the remainder of $R_5$, $R_6$, $R_7$, $R_8$, m and the provisos are as defined in Claim 1.

5. A benzopyran according to Claim 1 or Claim 2, wherein

an adjacent pair of $R_5$, $R_6$, $R_7$ and $R_8$ represents a chain $—CR_{10}R_{11}—CR_{12}R_{13}—CR_{14}R_{15}—NR_{20}—$,

in which the chain is substituted by $—A_1E_1$, wherein either (a) $R_{10}$ and $R_{12}$ together form a single bond and $R_{14}$ and $R_{20}$ together form a single bond, or (b) $R_{12}$ and $R_{14}$ together form a single bond,

the remainder of $R_{10}$, $R_{11}$, $R_{12}$, $R_{13}$, $R_{14}$ and $R_{15}$, which may be the same or different, independently represent hydrogen, alkyl C1 to 6, $NHCOR_{22}$, CN or $C_nH_xF_{(2n+1-x)}$; in addition $R_{10}$ and $R_{11}$, together with the carbon atom to which they are attached, may represent C=O or $C=NOR_{23}$,

A and $A_1$ each represent a single bond,

when $R_{20}$ does not form a single bond with $R_{14}$, $R_{20}$ may represent hydrogen, alkyl C1 to 6 or alkyl C1 to 6 substituted by phenyl,

n, x, $R_{21}$, $R_{22}$ are as defined in Claim 1, the remainder of $R_5$, $R_6$, $R_7$ and $R_8$, which may be the same or different, each independently represent hydrogen or hydroxy,

E and $E_1$ represent —COOH, save that when the chain represents $—COCR_{13}=CR_{15}—NR_{20}—$, in which one of $R_{13}$ and $R_{15}$ represents $E_1$ and the other represents hydrogen or alkyl C1 to 6, then $E_1$ represents hydrogen, and

provided that when the chain represents $—CR_{11}=CR_{13}—CR_{15}=N—$, then at least one of $R_{11}$, $R_{13}$ or $R_{15}$, which may be the same or different represents $NHCOR_{22}$, CN or $C_nH_xF_{(2n+1-x)}$.

6. A benzopyran according to Claim 1, which is 10-Propyl-2,8-bis(1H-tetrazol-5-yl)-4H,6H-benzo[1,2-b:5,4-b']dipyran-4,6-dione or a pharmaceutically acceptable salt thereof.

7. A benzopyran of formula I, as defined in Claim 1, which is

4-Oxo-10-propyl-6-(trifluoromethyl)-4H-pyrano[3,2-g]quinoline-2,8-dicarboxylic acid, or

8-(4-Carboxyphenyl)-4,6-dioxo-10-propyl-4H,6H-benzo[1,2-b:5,4-b']dipyran-2-carboxylic acid

or a pharmaceutically acceptable salt, ester or amide thereof.

8. A benzopyran of Formula I, as defined in claim 1, which is

4,6-Dioxo-10-propyl-6-(1H-tetrazol-5-yl)-4H,6H-benzo[1,2-b:5,4-b']dipyran-2-carboxylate

8-Aminocarbonyl-4,6-dioxo-10-propyl-4H,6H-benzo[1,2-b:5,4-b']dipyran-8-carboxylic acid

8-(N,N-Dimethylcarbonylamino)-4,6-dioxo-10-propyl-4H,6H-benzo[1,2-b:5,4-b']dipyran-2-carboxylic acid

3-(8-Carboxy-4,6-dioxo-10-propyl-4H,6H-benzo[1,2-b:5,4-b')dipyran-2)propanoic acid

4,6-Dioxo-12-propyl-4H,6H-[1]-benzopyrano[3,2-g][1]benzopyran-2,8-dicarboxylic acid

6,7,8,9-Tetrahydro-4-oxo-10-propyl-4H-naphtho[2,3-b]pyran-2,7-dicarboxylic acid

6,7,8,9-Tetrahydro-5-hydroxy-4-oxo-10-propyl-4H-naphtho[2,3-b]pyran-2,7-dicarboxylic acid

6,7,8,9-Tetrahydro-5-hydroxy-4-oxo-10-propyl-4H-naphtho-[2,3-b]pyran-2,8-dicarboxylic acid

7,8,9,10-Tetrahydro-1-oxo-5-propyl-1H-naphtho [2,1-b]pyran-3,8-dicarboxylic acid

6-(Heptafluoropropyl)-4-oxo-10-propyl-4H-pyrano[3,2-g]quinoline-2,8-dicarboxylic acid

4-Oxo-6-(pentafluoroethyl)-10-propyl-4H-pyrano[3,2-g]quinoline-2,8-dicarboxylic acid

6-(Difluoromethyl)-4-oxo-10-propyl-4H-pyrano[3,2-g]quinoline-2,8-dicarboxylic acid

6,9-Dihydro-4,6-dioxo-10-propyl-4H,6H-pyrano[3,2-g]quinoline-2-carboxylic acid

6,9-Dihydro-4,6-dioxo-9-ethyl-10-propyl-4H,6H-pyrano[3,2-g]-quinoline-2-carboxylic acid
6,9-dihydro-4,6-dioxo-9-phenylmethyl-10-propyl-4H-pyrano[3,2-g]quinoline-2-carboxylic acid
9-Ethyl-7,9-dihydro-6-methoxyimino-4-oxo-10-propyl-4H,6H-pyrano[3,2-g]quinoline-2,8-dicarboxylic acid
6-Methoxyimino-4-oxo-10-propyl-4H,6H-benzo[1,2-b:5,4-b']dipyran-2,8-dicarboxylic acid
6-(N-Methyl-N-acetylamino)-4-oxo-10-propyl-4H-pyrano[3,2-g]quinoline-2,8-dicarboxylic acid
6-Cyano-4-oxo-10-propyl-4H-pyrano[3,2-g]quinoline-2,8-dicarboxylic acid
5-Oxo-9-propyl-5H-furo[3,2-g][1]-benzopyran-2,7-dicarboxylic acid
5-Oxo-9-propyl-5H-thieno[3,2-g][1]-benzopyran-2,7-dicarboxylic acid
4-Oxo-9-propyl-4H,6H-pyrano[2,3-f]indole-2,7-dicarboxylic acid
8-Oxo-4-propyl-8H-furo[2,3-g][1]benzopyran-2,6-dicarboxylic acid
6-Ethyl-4-oxo-9-propyl-4H-pyrano[2,3-f]indole-2,7-dicarboxylic acid
4-Oxo-9-propyl-4H,8H-pyrano[3,2-f]indole-2,7-dicarboxylic acid
5-Oxo-9-propyl-5H-thieno[3,2-g][1]benzopyran-3,7-dicarboxylic acid
or a pharmaceutically acceptable salt, ester or amide of any one thereof.

9. A pharmaceutical composition which comprises a compound according to any one of the preceding claims in admixture with a pharmaceutically acceptable adjuvant, diluent or carrier.

10. A process for the preparation of a benzopyran according to any one of Claims 1 to 8, which comprises

(a) cyclising a compound of formula II, or a suitable derivative thereof,

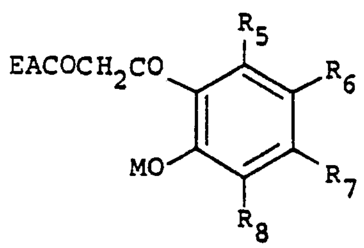

II

in which M represents a hydrogen atom or an alkali metal,

and E, A, $R_4$, $R_6$, $R_7$ and $R_8$ are as defined in Claim 1,

(b) producing a compound of formula I, in which at least one of E and $E_1$ represents —COOH, by hydrolysing a compound of formula I in which the corresponding E, or $E_1$ represents a group hydrolysable to a —COOH group,

(c) producing a compound of formula I, in which at least one of E and $E_1$ represents 5-tetrazolyl, by reacting a compound of formula I, in which the corresponding E or $E_1$ represents —CN, with an azide in a solvent which is inert under the reaction conditions,

(d) producing a compound of formula I, in which $E_1$ represents —$CONR_{24}R_{25}$, by reacting a compound of formula I in which $E_1$ represents —COL, wherein L is a good leaving group, with a compound of formula III,

$$R_{24}R_{25}NH \qquad \text{III}$$

in which $R_{24}$ and $R_{25}$ are as defined in Claim 1, or (e) producing a compound of formula I, in which at least one of $R_{10}$, $R_{11}$, $R_{12}$, $R_{13}$, $R_{14}$ and $R_{15}$ represents $C_nH_xF_{(2n+1-x)}$,

by reacting a compound of formula I, or a derivative thereof, in which the corresponding $R_{10}$, $R_{11}$, $R_{12}$, $R_{13}$, $R_{15}$ or $R_{15}$ represents $L_1$,

wherein $L_1$ is a halogen atom,

with a compound $C_nH_xF_{(2n+1-x)}L_2$, in which n and x are as defined in Claim 1 and $L_2$ represents chlorine, bromine or iodine,

and if necessary or desired converting the compound of formula I to a pharmaceutically acceptable salt thereof and when any one of E or $E_1$ represents a carboxylic acid group, pharmaceutically acceptable esters or amides of the carboxylic acid groups or vice versa.

11. The use of a compound according to any one of Claims 1 to 8 to make a pharmaceutical formulation for the treatment of asthma.

**Claims for the Contracting State: AT**

1. A process for the preparation of a benzopyran of formula I

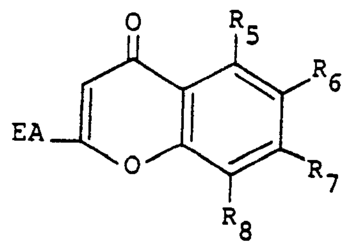

I

wherein an adjacent pair of $R_5$, $R_6$, $R_7$ and $R_8$ represent the chain $-X-CR_{12}R_{13}-CR_{14}R_{15}-Y-$ in which the chain is substituted by $-A_1E_1$,

X represents $CR_{10}R_{11}$ or a single bond,

Y represents O,S, $NR_{20}$ or $CH_2$, and (a) $R_{15}$ and $R_{20}$ together form a single bond and optionally $R_{10}$ and $R_{12}$ together form a single bond, or (b) $R_{12}$ and $R_{14}$ together form a single bond, or (c) $R_{10}$, $R_{12}$ and $R_{14}$ each represent hydrogen; and

the remainder of $R_{10}$, $R_{11}$, $R_{12}$, $R_{13}$, $R_{14}$ and $R_{15}$, which may be the same or different, independently represent hydrogen, alkyl C1 to 6, $NR_{21}COR_{22}$, CN or $C_nH_xF_{(2n+1-x)}$; in addition, $R_{10}$ and $R_{11}$, together with the carbon atom to which they are attached, may represent C=O or $C=N-OR_{23}$; also $R_{13}$ and $R_{15}$ may form the chain $-CH=CH-CH=CH-$ which is substituted by $A_1E_1$,

A and $A_1$, which may be the same or different, represent a single bond, $(CH_2)_m$ or phenylene,

n represents an integer from 1 to 4 inclusive,

x represents 0 or an integer from 1 to 2 inclusive,

m represents an integer from 1 to 4 inclusive,

when $R_{20}$ does not form a single bond with $R_{14}$, $R_{20}$ may represent hydrogen, alkyl C1 to 6 or alkyl C1 to 6 substituted by phenyl,

$R_{21}$, $R_{22}$, and $R_{23}$, which may be the same or different, independently represent hydrogen or alkyl C1 to 6;

the remainder of $R_5$, $R_6$, $R_7$ and $R_8$, which may be the same or different, each independently represent hydrogen, hydroxy or alkyl C1 to 6,

E and $E_1$, which may be the same or different, independently represent $-COOH$, or 5H-tetrazolyl, provided that when the chain $-X-CR_{12}R_{13}=CR_{14}R_{15}-Y$

i) represents a chain $-CO-CR_{13}=CR_{15}-Y-$ in which Y is O or S and A and $A_1$ each represent a single bond, then Y is bonded to $R_7$, $R_5$ and $R_{13}$ each represent hydrogen, $R_8$ represents propyl and $R_{15}$ represents $E_1$, in which $E_1$, instead of being as defined above, represents 5H-tetrazolyl or $CONR_{24}R_{25}$, wherein $R_{24}$ and $R_{25}$, which may be the same or different, represent hydrogen or alkyl C1 to 6;

ii) represents a chain $-CO-CR_{13}=CR_{15}-NR_{20}-$, in which one of $R_{13}$ and $R_{15}$ represents $E_1$, then $E_1$, instead of being as defined above, represents hydrogen, and

iii) represents a chain $-CR_{11}=CR_{13}-CR_{15}=N-$, then at least one of $R_{11}$, $R_{13}$ or $R_{15}$, which may be the same or different, represents $NR_{21}COR_{22}$, CN or $C_nH_xF_{(2n+1-x)}$, and pharmaceutically acceptable salts thereof and when any one of E or $E_1$ represents a carboxylic acid group, pharmaceutically acceptable esters or amides of the carboxylic acid groups, which comprises

(a) cyclising a compound of formula II, or a suitable derivative thereof,

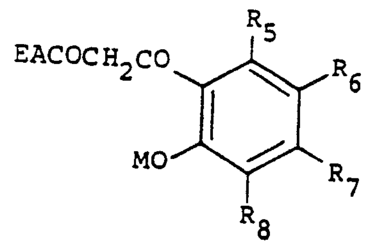

II

in which M represents a hydrogen atom or an alkali metal,

and E, A, $R_5$, $R_6$, $R_7$ and $R_8$ are as defined above,

(b) producing a compound of formula I, in which at least one of E and $E_1$ represents $-COOH$, by hydrolysing a compound of formula I in which the corresponding E, or $E_1$ represents a group hydrolysable to a $-COOH$ group,

(c) producing a compound of formula I, in which at least one of E and $E_1$ represents 5-tetrazolyl, by reacting a compound of formula I, in which the corresponding E or $E_1$ represents $-CN$, with an azide in a solvent which is inert under the reaction conditions,

(d) producing a compound of formula I, in which $E_1$ represents $—CONR_{24}R_{25}$, by reacting a compound of formula I in which $E_1$ represents —COL, wherein L is a good leaving group,

with a compound of formula III,

$$R_{24}R_{25}NH \qquad III$$

in which $R_{24}$ and $R_{25}$ are as defined above, or

(e) producing a compound of formula I, in which at least one of $R_{10}$, $R_{11}$, $R_{12}$, $R_{13}$, $R_{14}$ and $R_{15}$ represents $C_nH_xF_{(2n+1-x)}$,

by reacting a compound of formula I, or a derivative thereof, in which the corresponding $R_{10}$, $R_{11}$, $R_{12}$, $R_{13}$, $R_{14}$ or $R_{15}$ represents $L_1$,

wherein $L_1$ is a halogen atom,

with a compound $C_nH_xF_{(2n+1-x)}L_2$, in which n and x are as defined above and $L_2$ represents chlorine, bromine or iodine,

and if necessary or desired converting the compound of formula I to a pharmaceutically acceptable salts thereof and when any one of E or $E_1$ represents a carboxylic acid group, pharmaceutically acceptable esters or amides of the carboxylic acid groups or vice versa.

2. A process for the preparation of a benzopyran according to Claim 1, wherein the chain $—X—CR_{12}R_{13}—CR_{14}R_{15}—Y—$ is formed between $R_6$ and $R_7$.

3. A process for the preparation of a benzopyran according to Claim 1 or Claim 2, wherein Y represents —O—.

4. A process for the preparation of a benzopyran according to any one of the preceding claims, wherein an adjacent pair of $R_5$, $R_6$, $R_7$ and $R_8$ represents the chain $—CO—CR_{12}R_{13}CR_{14}R_{15}—O—$,

$R_{12}$ and $R_{14}$ together form a single bond,

one of $R_{13}$ and $R_{15}$ represents $A_1E_1$ and the other represents hydrogen, or $R_{13}$ and $R_{15}$ together form a chain —CH=CH—CH=CH—, the chain being substituted by $A_1E_1$,

A represents a single bond,

$A_1$ represents a single bond, phenylene or $(CH_2)_m$ and E, $E_1$, the remainder of $R_5$, $R_6$, $R_7$, $R_8$, m and the provisos are as defined in Claim 1.

5. A process for the preparation of a benzopyran according to Claim 1 or Claim 2, wherein

an adjacent pair of $R_5$, $R_6$, $R_7$ and $R_8$ represents a chain $—CR_{10}R_{11}—CR_{12}R_{13}—CR_{14}R_{15}—NR_{20}—$,

in which the chain is substituted by $—A_1E_1$, wherein either (a) $R_{10}$ and $R_{12}$ together form a single bond and $R_{14}$ and $R_{20}$ together form a single bond, or (b) $R_{12}$ and $R_{14}$ together form a single bond,

the remainder of $R_{10}$, $R_{11}$, $R_{12}$, $R_{13}$, $R_{14}$ and $R_{15}$, which may be the same or different, independently represent hydrogen, alkyl C1 to 6, $NHCOR_{22}$, CN or $C_nH_xF_{(2n+1-x)}$; in addition $R_{10}$ and $R_{11}$, together with the carbon atom to which they are attached, may represent C=O or $C=NOR_{23}$,

A and $A_1$ each represent a single bond,

when $R_{20}$ does not form a single bond with $R_{14}$, $R_{20}$ may represent hydrogen, alkyl C1 to 6 or alkyl Cl to 6 substituted by phenyl,

n, x, $R_{21}$, $R_{22}$ are as defined in Claim 1, the remainder of $R_5$, $R_6$, $R_7$ and $R_8$, which may be the same or different, each independently represent hydrogen or hydroxy,

E and $E_1$ represent —COOH, save that when the chain represents $—COCR_{13}=CR_{15}—NR_{20}—$, in which one of $R_{13}$ and $R_{15}$ represents $E_1$ and the other represents hydrogen or alkyl C1 to 6, then $E_1$ represents hydrogen, and

provided that when the chain represents $—CR_{11}=CR_{13}—CR_{15}=N—$, then at least one of $R_{11}$, $R_{13}$ or $R_{15}$, which may be the same or different represents $NHCOR_{22}$, CN or $C_nH_xF_{(2n+1-x)}$.

6. A process for the preparation of a benzopyran according to Claim 1, wherein the compound of formula I is

10-Propyl-2,8-bis(1H-tetrazol-5-yl)-4H,6H-benzo[1,2-b:5,4-b']dipyran-4,6-dione

or a pharmaceutically acceptable salt thereof.

7. A process for the preparation of a benzopyran according to Claim 1, in which the compound of formula I is

4-Oxo-10-propyl-6-(trifluoromethyl)-4H-pyrano[3,2-g]quinoline-2,8-dicarboxylic acid, or

8-(4-Carboxyphenyl)-4,6-dioxo-10-propyl-4H,6H-benzo[1,2-b:5,4-b']dipyran-2-carboxylic acid

or a pharmaceutically acceptable salt, ester or amide thereof.

8. A process for the preparation of a benzopyran according to Claim 1, in which the compound of formula I is

4,6-Dioxo-10-propyl-6-(1H-tetrazol-5-yl)-4H,6H-benzo[1,2-b:5,4-b']dipyran-2-carboxylate

8-Aminocarbonyl-4,6-dioxo-10-propyl-4H,6H-benzo[1,2-b:5,4-b']dipyran-8-carboxylic acid

8-(N,N-Dimethylcarbonylamino)-4,6-dioxo-10-propyl-4H,6H-benzo[1,2-b:5,4-b']dipyran-2-carboxylic acid

3-(8-Carboxy-4,6-dioxo-10-propyl-4H,6H-benzo[1,2-b:5,4-b')dipyran-2)propanoic acid

4,6-Dioxo-12-propyl-4H,6H-[1]-benzopyrano[3,2-g][1]benzopyran-2,8-dicarboxylic acid

6,7,8,9-Tetrahydro-4-oxo-10-propyl-4H-naphtho[2,3-b]pyran-2,7-dicarboxylic acid

6,7,8,9-Tetrahydro-5-hydroxy-4-oxo-10-propyl-4H-naphtho[2,3-b]pyran-2,7-dicarboxylic acid

6,7,8,9-Tetrahydro-5-hydroxy-4-oxo-10-propyl-4H-naphtho-[2,3-b]pyran-2,8-dicarboxylic acid
7,8,9,10-Tetrahydro-1-oxo-5-propyl-1H-naphtho [2,1-b]pyran-3,8-dicarboxylic acid
6-(Heptafluoropropyl)-4-oxo-10-propyl-4H-pyrano[3,2-g]quinoline-2,8-dicarboxylic acid
4-Oxo-6-(pentafluoroethyl)-10-propyl-4H-pyrano[3,2-g]quinoline-2,8-dicarboxylic acid
6-(Difluoromethyl)-4-oxo-10-propyl-4H-pyrano[3,2-g]quinoline-2,8-dicarboxylic acid
6,9-Dihydro-4,6-dioxo-10-propyl-4H,6H-pyrano[3,2-g]quinoline-2-carboxylic acid
6,9-Dihydro-4,6-dioxo-9-ethyl-10-propyl-4H,6H-pyrano[3,2-g]-quinoline-2-carboxylic acid
6,9-dihydro-4,6-dioxo-9-phenylmethyl-10-propyl-4H-pyrano[3,2-g]quinoline-2-carboxylic acid
9-Ethyl-7,9-dihydro-6-methoxyimino-4-oxo-10-propyl-4H,6H-pyrano[3,2-g]quinoline-2,8-dicarboxylic acid
6-Methoxyimino-4-oxo-10-propyl-4H,6H-benzo[1,2-b:5,4-b']dipyran-2,8-dicarboxylic acid
6-(N-Methyl-N-acetylamino)-4-oxo-10-propyl-4H-pyrano[3,2-g]quinoline-2,8-dicarboxylic acid
6-Cyano-4-oxo-10-propyl-4H-pyrano[3,2-g]quinoline-2,8-dicarboxylic acid
5-Oxo-9-propyl-5H-furo[3,2-g][1]-benzopyran-2,7-dicarboxylic acid
5-Oxo-9-propyl-5H-thieno[3,2-g][1]-benzopyran-2,7-dicarboxylic acid
4-Oxo-9-propyl-4H,6H-pyrano[2,3-f]indole-2,7-dicarboxylic acid
8-Oxo-4-propyl-8H-furo[2,3-g][1]benzopyran-2,6-dicarboxylic acid
6-Ethyl-4-oxo-9-propyl-4H-pyrano[2,3-f]indole-2,7-dicarboxylic acid
4-Oxo-9-propyl-4H,8H-pyrano[3,2-f]indole-2,7-dicarboxylic acid
5-Oxo-9-propyl-5H-thieno[3,2-g][1]benzopyran-3,7-dicarboxylic acid
or a pharmaceutically acceptable salt, ester or amide of any one thereof.

**Patentansprüche für die Vertragsstaaten: BE CH DE FR GB IT LI LU NL SE**

1. Benzopyran der Formel (I)

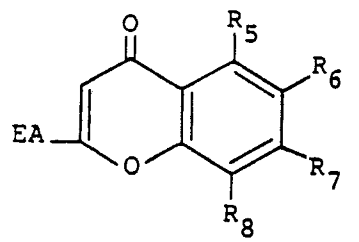

I

worin ein benachbartes Paar von $R_5$, $R_6$, $R_7$ und $R_8$ die Kette $—X—CR_{12}R_{13}—CR_{14}CR_{15}—Y—$ darstellt, wobei die Kette durch $—A_1E_1$ substituiert ist,

X $CR_{10}R_{11}$ oder eine Einfachbindung bedeutet,

Y O, S, $NR_{20}$ oder $CH_2$ ist und

(a) $R_{14}$ und $R_{20}$ miteinander eine Einfachbindung bilden und gegebenenfalls $R_{10}$ und $R_{12}$ miteinander eine Einfachbindung bilden oder (b) $R_{12}$ und $R_{14}$ miteinander eine Einfachbindung bilden oder (c) $R_{10}$, $R_{12}$ und $R_{14}$ jeweils Wasserstoff darstellen; und

der Rest von $R_{10}$, $R_{11}$, $R_{12}$, $R_{13}$, $R_{14}$ und $R_{15}$, die gleich oder verschieden sein können, unabhängig Wasserstoff, $C_{1-6}$-Alkyl, $NR_{21}COR_{22}$, CN oder $C_nH_xF_{(2n+1-x)}$ ist; außerdem $R_{10}$ und $R_{11}$ zusammen mit dem Kohlenstoffatom, an das sie gebunden sind, C=O oder C=N—$OR_{23}$ bilden können; $R_{13}$ und $R_{15}$ auch die Kette —CH=CH—CH=CH— bilden können, die durch $A_1E_1$ substituiert ist,

A und $A_1$, die gleich oder verschieden sein können, eine Einfachbindung, $(CH_2)_m$ oder Phenylen darstellen,

n eine ganze Zahl von 1 bis 4 inklusive ist,

x Null oder eine ganze Zahl von 1 bis 2n inklusive ist,

m eine ganze Zahl von 1 bis 4 inklusive ist,

wenn $R_{20}$ keine Einfachbindung mit $R_{14}$ bildet, $R_{20}$ Wasserstoff, $C_{1-6}$-Alkyl oder phenylsubstituiertes $C_{1-6}$-Alkyl bedeuten kann,

$R_{21}$, $R_{22}$ und $R_{23}$, die gleich oder verschieden sein können, unabhängig Wasserstoff oder $C_{1-6}$-Alkyl darstellen,

der Rest von $R_5$, $R_6$, $R_7$ und $R_8$, die gleich oder verschieden sein können, jeweils Wasserstoff, Hydroxy oder $C_{16}$-Alkyl ist,

E und $E_1$, die gleich oder verschieden sein können, unabhängig die Bedeutung —COOH oder 5H-Tetrazolyl haben,

mit der Maßgabe daß, wenn die Kette $—X—CR_{12}R_{13}=CR_{14}R_{15}—Y$

i) eine Kette $—CO—CR_{13}=CR_{15}—Y—$ darstellt, worin Y O oder S ist, und A und $A_1$ jeweils eine Einfachbindung bedeuten, dann Y an $R_7$ gebunden ist, $R_5$ und $R_{13}$ jeweils Wasserstoff bedeuten, $R_8$ Propyl ist und $R_{15}$ $E_1$ darstellt, wobei $E_1$, anstatt die oben angegebene Bedeutung zu haben, 5H-Tetrazolyl oder $CONR_{24}R_{25}$ bedeutet, worin $R_{24}$ und $R_{25}$, die gleich oder verschieden sein können, Wasserstoff oder $C_{1-6}$-Alkyl sind;

ii) eine Kette —CO—$CR_{13}$=$CR_{15}$—$NR_{20}$— darstellt, worin eines von $R_{13}$ und $R_{15}$ $E_1$ ist, dann $E_1$, anstatt die oben angegebene Bedeutung zu haben, Wasserstoff ist, und

iii) eine Kette —$CR_{11}$=$CR_{13}$—$CR_{15}$=N— darstellt, dann mindestens eines von $R_{11}$, $R_{13}$ oder $R_{15}$, die gleich oder verschieden sein können, die Bedeutung $NR_{21}COR_{22}$, CN oder $C_nH_xF_{(2n+1-x)}$ hat,

und pharmazeutisch annehmbare Salze hievon und, wenn eines von E oder $E_1$ eine Carbonsäuregruppe darstellt, pharmazeutisch annehmbare Ester oder Amide der Carbonsäuregruppen.

2. Benzopyran nach Anspruch 1, worin die kette —X—$CR_{12}R_{13}$—$CR_{14}R_{15}$—Y— zwischen $R_6$ und $R_7$ gebildet ist.

3. Benzopyran nach Anspruch 1 oder 2, worin Y die Bedeutung —O— hat.

4. Benzopyran nach einem der vorhergehenden Ansprüche, worin

ein benachbartes Paar von $R_5$, $R_6$, $R_7$ und $R_8$ die Kette —CO—$CR_{12}R_{13}CR_{14}R_{15}$—O— darstellt,

$R_{12}$ und $R_{14}$ miteinander eine Einfachbindung bilden,

eines von $R_{13}$ und $R_{15}$ die Bedeutung $A_1E_5$ hat und das andere Wasserstoff ist oder $R_{13}$ und $R_{15}$ miteinander eine Kette —CH=CH—CH=CH— bilden, wobei die Kette durch $A_1E_1$ substituiert ist,

A eine Einfachbindung darstellt,

$A_1$ eine Einfachbindung, Phenylen oder $(CH_2)_m$ bedeutet und

E, $E_1$, der Rest von $R_5$, $R_6$, $R_7$, $R_8$, m und die Maßgaben wie in Anspruch 1 definiert sind.

5. Benzopyran nach Anspruch 1 oder 2, worin ein benachbartes Paar von $R_5$, $R_6$, $R_7$ und $R_8$ eine Kette —$CR_{10}R_{11}$—$CR_{12}R_{13}$—$CR_{14}R_{15}$—$NR_{20}$ ist, wobei die Kette durch —$A_{11}E_1$ substituiert ist, wobei entweder

(a) $R_{10}$ und $R_{12}$ miteinander eine Einfachbindung bilden und $R_{14}$ und $R_{20}$ miteinander eine Einfachbindung bilden oder

(b) $R_{12}$ und $R_{14}$ miteinander eine Einfachbindung bilden,

der Rest von $R_{10}$, $R_{11}$, $R_{12}$, $R_{13}$, $R_{14}$ und $R_{15}$, die gleich oder verschieden sein können, unabhängig Wasserstoff, $C_{1-6}$-Alkyl, $NHCOR_{22}$, CN oder $C_nH_xF_{(2n+1-x)}$ ist; außerdem $R_{10}$ und $R_{11}$ zusammen mit dem Kohlenstoffatom, an das sie gebunden sind, C=O oder C=$NOR_{23}$ bilden können,

A und $A_1$ jeweils ein Einfachbindung darstellen,

wenn $R_{20}$ keine Einfachbindung mit $R_{14}$ bildet, $R_{20}$ Wasserstoff, $C_{1-6}$-Alkyl oder phenylsubstituiertes $C_{1-6}$-Alkyl bedeuten kann,

n, x, $R_{21}$ und $R_{22}$ wie in Anspruch 1 definiert sind,

der Rest von $R_5$, $R_6$, $R_7$ und $R_8$, die gleich oder verschieden sein können, jeweils Wasserstoff oder Hydroxy ist,

E und $E_1$ die Bedeutung —COOH haben, ausgenommen daß, wenn die Kette —$COCR_{13}$=$CR_{15}$—$NR_{20}$— ist, in der eines von $R_{13}$ und $R_{15}$ die Bedeutung $E_1$ hat und das andere Wasserstoff oder $C_{1-6}$-Alkyl ist, dann $E_1$ Wasserstoff darstellt, und

mit der Maßgabe daß, wenn die Kette —$CR_{11}$=$CR_{13}$—$CR_{15}$=N— ist, dann mindestens eines von $R_{11}$, $R_{13}$ oder $R_{15}$, die gleich oder verschieden sein können, $NHCOR_{22}$, CN oder $C_nH_xF_{(2n+1-x)}$ bedeutet.

6. Benzopyran nach Anspruch 1, das 10-Propyl-2,8-bis-(1H-tetrazol-5-yl)-4H,6H-benzo[1,2-b:5,4-b')]dipyran-4,6-dion oder ein pharmazeutisch annehmbares Salz hievon ist.

7. Benzopyran der Formel (I), wie in Anspruch 1 definiert, das

4-Oxo-10-propyl-6-(trifluormethyl)-4H-pyrano[3,2-g]chinolin-2,8-dicarbonsäure oder

8-(4-Carboxyphenyl)-4,6-dioxo-10-propyl-4H,6H-benzo[1,2-b:5,4-b']dipyran-2-carbonsäure

oder ein pharmazeutisch annehmbares Salz, Ester oder Amid hievon ist.

8. Benzopyran der Formel (I), wie in Anspruch 1 definiert, das

4,6-Dioxo-10-propyl-8-(1H-tetrazol-5-yl)-4H,6H-benzo[1,2-b:5,4-b']dipyran-2-carboxylat,

8-Aminocarbonyl-4,6-dioxo-10-propyl-4H,6H-benzo[1,2-b:5,4-b']dipyran-8-carbonsäure,

8-(N,N-Dimethylcarbonylamino)-4,6-dioxo-10-propyl-4H,6H-benzo[1,2-bis:5,4-b']dipyran-2-carbonsäure,

3-(8-Carboxy-4,6-dioxo-10-propyl-4H,6H-benzo[1,2-b:5,4-b']dipyran-2)-propansäure,

4,6-Dioxo-12-propyl-4H,6H[1]benzopyrano[3,2-g][1]benzopyran-2,8-dicarbonsäure,

6,7,8,9-Tetrahydro-4-oxo-10-propyl-4H-naphtho[2,3-b]pyran-2,7-dicarbonsäure,

6,7,8,9-Tetrahydro-5-hydroxy-4-oxo-10-propyl-4H-naphtho[2,3-b]pyran-2,7-dicarbonsäure,

6,7,8,9-Tetrahydro-5-hydroxy-4-oxo-10-propyl-4H-naphtho[2,3-b]pyran-2,8-dicarbonsäure,

7,8,9,10-Tetrahydro-1-oxo-5-propyl-1H-naphtho[2,3-b]pyran-3,8-dicarbonsäure,

6-(Heptafluorpropyl)-4-oxo-10-propyl-4H-pyrano[3,2-g]chinolin-2,8-dicarbonsäure,

4-Oxo-6-(pentafluoräthyl)-10-propyl-4H-pyrano[3,2-g]chinolin-2,8-dicarbonsäure,

6-(Difluormethyl)-4-oxo-10-propyl-4H-pyrano[3,2-g]chinolin-2,8-dicarbonsäure,

6,9-Dihydro-4,6-dioxo-10-propyl-4H,6H-pyrano[3,2-g]chinolin-2-carbonsäure,

6,9-Dihydro-4,6-dioxo-9-äthyl-10-propyl-4H,6H-pyrano[3,2-g]chinolin-2-carbonsäure,

6,9-Dihydro-4,6-dioxo-9-phenylmethyl-10-propyl-4H-pyrano[3,2-g]chinolin-2-carbonsäure,

9-Äthyl-7,9-dihydro-6-methoxyimino-4-oxo-10-propyl-4H,6H-pyrano[3,2-g]chinolin-2,8-dicarbonsäure,

6-Methoxyimino-4-oxo-10-propyl-4H,6H-benzo[1,2-b:5,4-b']dipyran-2,8-dicarbonsäure,

6-(N-Methyl-N-acetylamino)-4-oxo-10-propyl-4H-pyrano[3,2-g]chinolin-2,8-dicarbonsäure,

6-Cyano-4-oxo-10-propyl-4H-pyrano[3,2-g]chinolin-2,8-dicarbonsäure,

5-Oxo-9-propyl-5H-furo[3,2-g][1]benzopyran-2,7-dicarbonsäure,

5-Oxo-9-propyl-5H-thieno[3,2-g[1]benzopyran-2,7-dicarbonsäure,

4-Oxo-9-propyl-4H,6H-pyrano[2,3-f]indol-2,7-dicarbonsäure,
8-Oxo-4-propyl-8H-furo[2,3-g][1]benzopyran-2,6-dicarbonsäure,
6-Äthyl-4-oxo-9-propyl-4H-pyrano[2,3-f]indol-2,7-dicarbonsäure,
4-Oxo-9-propyl-4H,8H-pyran[3,2-f]indol-2,7-dicarbonsäure,
5-Oxo-9-propyl-5H-thieno[3,2-g][1]benzopyran-3,7-dicarbonsäure
oder ein pharmazeutisch annehmbares Salz, Ester oder Amid eines derselben ist.

9. Pharmazeutische Zusammensetzung, die eine Verbindung nach einem der vorhergehenden Ansprüche in Mischung mit einem pharmazeutisch annehmbaren Adjuvans, Verdünnungsmittel oder Träger umfaßt.

10. Verfahren zur Herstellung eines Benzopyrans nach einem der Ansprüche 1 bis 8, welches umfaßt:
(a) das Cyclisieren einer Verbindung der Formel (II) oder eines geeigneten Derivates hievon

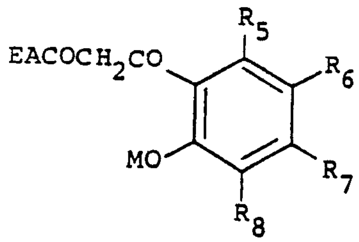

II

worin M ein Wasserstoffatom oder ein Alkalimetall bedeutet und E, A, $R_5$, $R_6$, $R_7$ und $R_8$ wie in Anspruch 1 definiert sind, (b) das Herstellen einer Verbindung der Formel (I), worin mindestens eines von E und $E_1$ —COOH darstellt, durch Hydrolysieren einer Verbindung der Formel (I), worin das entsprechende E oder $E_1$ eine zu einer Gruppe —COOH hydrolysierbare Gruppe bedeutet, (c) das Herstellen einer Verbindung der Formel (I), worin mindestens eines von E und $E_1$ 5-Tetrazolyl bedeutet, durch Umsetzen einer Verbindung der Formel (I), worin das entsprechende E oder $E_1$ die Bedeutung —CN hat, mit einem Azid in einem Lösungsmitteln, das unter den Reaktionsbedingungen inert ist, (d) das Herstellen einer Verbindung der Formel (I), worin $E_1$ —$CONR_{24}R_{25}$ bedeutet, durch Umsetzen einer Verbindung der Formel (I), worin $E_1$ die Bedeutung —COL hat, wobei L eine gut abspaltbare Gruppe ist, mit einer Verbindung der Formel (III)

$$R_{24}R_{25}NH \qquad (III),$$

worin $R_{24}$ und $R_{25}$ wie in Anspruch 1 definiert sind, oder
(e) das Herstellen einer Verbindung der Formel (I)), worin mindestens eines von $R_{10}$, $R_{11}$, $R_{12}$, $R_{13}$, $R_{14}$ und $R_{15}$ die Bedeutung $C_nH_xF_{(2n+1-x)}$ hat, durch Umsetzen einer Verbindung der Formel (I) oder eines Derivates hievon, worin das entsprechende $R_{10}$, $R_{11}$, $R_{12}$, $R_{13}$, $R_{14}$ oder $R_{15}$ die Bedeutung $L_1$ hat, wobei $L_1$ ein Halogenatom bedeutet, mit einer Verbindung $C_nH_xF_{(2n+1-x)}L_2$, worin n und x wie in Anspruch 1 definiert sind und $L_2$ Chlor, Brom oder Jod darstellt, und
wenn notwendig oder gewünscht, das überführen der Verbindung der Formel (I) in ein pharmazeutisch annehmbares Salz hievon und, wenn eines von E oder $E_1$ eine Carbonsäuregruppe bedeutet, in pharmazeutisch annehmbare Ester oder Amide der Carbonsäuregruppe.

11. Verwendung einer Verbindung nach einem der Ansprüche 1 bis 8 zum Herstellen einer pharmazeutischen Formulierung zur Behandlung von Asthma.

**Patentansprüche für den Vertragsstaat: AT**

1. Verfahren zur Herstellung eines Benzopyrans der Formel (L)

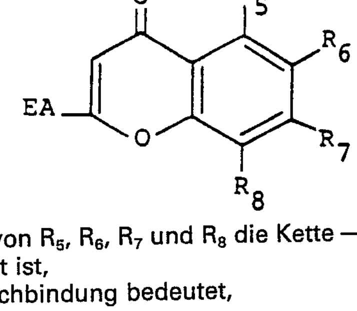

I

worin ein benachbartes Paar von $R_5$, $R_6$, $R_7$ und $R_8$ die Kette —X—$CR_{12}R_{13}$—$CR_{14}$—Y— darstellt, wobei die Kette durch —$A_1E_1$ substituiert ist,
X $CR_{10}R_{11}$ oder eine Einfachbindung bedeutet,
Y O, S, $NR_{20}$ oder $CH_2$ ist und
(a) $R_{14}$ und $R_{20}$ miteinander eine Einfachbindung bilden und gegebenenfalls $R_{10}$ und $R_{12}$ miteinander eine Einfachbindung bilden oder (b) $R_{12}$ und $R_{14}$ miteinander eine Einfachbindung bilden oder (c) $R_{10}$, $R_{16}$ und $R_{14}$ jeweils Wasserstoff darstellen; und
der Rest von $R_{10}$, $R_{11}$, $R_{12}$, $R_{13}$, $R_{14}$ und $R_{15}$, die gleich oder verschieden sein können, unabhängig

Wasserstoff, $C_{1-6}$-Alkyl, $NR_{21}COR_{22}$, CN oder $C_nH_xF_{(2n+1-x)}$ ist; außerdem $R_{10}$ und $R_{11}$ zusammen mit dem Kohlenstoffatom, an das sie gebunden sind, C=O oder C=N—$OR_{23}$ bilden können; $R_{13}$ und $R_{15}$ auch die Kette —CH=CH—CH=CH— bilden können, die durch $A_1E_1$ substituiert ist,

A und $A_1$, die gleich oder verschieden sein können, eine Einfachbindung, $(CH_2)_m$ oder Phenylen darstellen,

n eine ganze Zahl von 1 bis 4 inklusive ist,

x Null oder eine ganze Zahl von 1 bis 2n inklusive ist,

m eine ganze Zahl von 1 bis 4 inklusive ist,

wenn $R_{20}$ keine Einfachbindung mit $R_{14}$ bildet, $R_{20}$ Wasserstoff, $C_{1-6}$-Alkyl oder phenylsubstituiertes $C_{1-6}$-Alkyl bedeuten kann,

$R_{21}$, $R_{22}$ und $R_{23}$, die gleich oder verschieden sein können, unabhängig Wasserstoff oder $C_{1-6}$-Alkyl darstellen,

der Rest von $R_5$, $R_6$, $R_7$ und $R_8$, die gleich oder verschieden sein können, jeweils Wasserstoff, Hydroxy oder $C_{1-6}$-Alkyl ist,

E und $E_1$, die gleich oder verschieden sein können, unabhängig die Bedeutung —COOH oder 5H-Tetrazolyl haben,

mit der Maßgabe daß, wenn die Kette —X—$CR_{12}R_{13}$=$CR_{18}R_{15}$-Y

i) eine Kette —CO—$CR_{13}$=$CR_{15}$—Y— darstellt, worin Y O oder S ist, und A und $A_1$ jeweils eine Einfachbindung bedeuten, dann Y an $R_7$ gebunden ist, $R_5$ und $R_{13}$ jeweils Wasserstoff bedeuten, $R_8$ Propyl ist und $R_{15}$ $E_1$ darstellt, wobei $E_1$, anstatt die oben angegebene Bedeutung zu haben, 5H-Tetrazolyl oder $CONR_{24}R_{25}$ bedeutet, worin $R_{24}$ und $R_{25}$, die bleich oder verschieden sein können, Wasserstoff oder $C_{1-6}$-Alkyl sind;

ii) eine Kette —CO—$CR_{13}$=$CR_{15}$—$NR_{20}$— darstellt, worin eines von $R_{13}$ und $R_{15}$ $E_1$ ist, dann $E_1$, anstatt die oben angegebene Bedeutung zu haben, Wasserstoff ist, und

iii) eine Kette —$CR_{11}$=$CR_{13}$—$CR_{15}$=N— darstellt, dann mindestens eines von $R_{11}$, $R_{13}$ oder $R_{15}$, die gleich oder verschieden sein können, die Bedeutung $NR_{21}COR_{22}$, CN oder $C_nH_xF_{(2n+1-x)}$ hat,

und pharmazeutisch annehmbaren Salzen hievon und, wenn eines von E oder $E_1$ eine Carbonsäuregruppe darstellt, pharmazeutisch annehmbaren Estern oder Amiden der Carbonsäuregruppen, welches umfaßt:

(a) das Cyclisieren einer Verbindung der Formel (II) oder eines geeigneten Derivates hievon

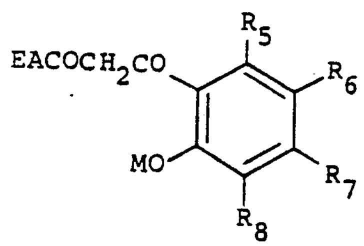

II

worin M ein Wasserstoffatom oder ein Alkalimetall bedeutet und E, A, $R_5$, $R_6$, $R_7$ und $R_8$ wie oben definiert sind, (b) das Herstellen einer Verbindung der Formel (I), worin mindestens eines von E und $E_1$ —COOH darstellt, durch Hydrolysieren einer Verbindung der Formel (I), worin das entsprechende E oder $E_1$ eine zu einer Gruppe —COOH hydrolysierbare Gruppe bedeutet, (c) das Herstellen einer Verbindung der Formel (I), worin mindestens eines von E und $E_1$ 5-Tetrazolyl bedeutet, durch Umsetzen einer Verbindung der Formel (I), worin das entsprechende E oder $E_1$ die Bedeutung —CN hat, mit einem Azid in einem Lösungsmitteln, das unter den Reaktionsbedingungen inert ist, (d) das Herstellen einer Verbindung der Formel (I), worin $E_1$ —$CONR_{24}R_{25}$ bedeutet, durch Umsetzen einer Verbindung der Formel (I), worin $E_1$ die Bedeutung —COL hat, wobei L eine gut abspaltbare Gruppe ist, mit einer Verbindung der Formel (III)

$$R_{24}R_{25}NH \qquad (III),$$

worin $R_{24}$ und $R_{25}$ wie oben 1 definiert sind, oder

(e) das Herstellen einer Verbindung der Formel (I)), worin mindestens eines von $R_{10}$, $R_{11}$, $R_{12}$, $R_{13}$, $R_{14}$ und $R_{15}$ die Bedeutung $C_nH_xF_{(2n+1-x)}$ hat, durch Umsetzen einer Verbindung der Formel (I) oder eines Derivatves hievon, worin das entsprechende $R_{10}$, $R_{11}$, $R_{12}$, $R_{13}$, $R_{14}$ oder $R_{15}$ die Bedeutung $L_1$ hat, wobei $L_1$ ein Halogenatom bedeutet, mit einer Verbindung $C_nH_xF_{(2n+1-x)}L_2$, worin n und x wie oben definiert sind und $L_2$ Chlor, Brom oder Jod darstellt, und

wenn notwendig oder gewünscht, das überführen der Verbindung der Formel (I) in ein pharmazeutisch annehmbares Salz hievon und, wenn eines von E oder $E_1$ eine Carbonsäuregruppe bedeutet, in pharmazeutisch annehmbare Ester oder Amide der Carbonsäuregruppe.

2. Verfahren zur Herstellung eines Benzopyrans nach Anspruch 1, worin die kette —X—$CR_{12}R_{13}$—$CR_{14}R_{15}$—Y— zwischen $R_6$ und $R_7$ gebildet ist.

3. Verfahren zur Herstellung eines Benzopyrans nach Anspruch 1 oder 2, worin Y die Bedeutung —O— hat.

4. Verfahren zur Herstellung eines Benzopyrans nach einem der vorhergehenden Ansprüche, worin ein benachbartes Paar von $R_5$, $R_6$, $R_7$ und $R_8$ die Kette —CO—$CR_{12}R_{13}CR_{14}R_{15}$—O— darstellt,

$R_{12}$ und $R_{14}$ miteinander eine Einfachbindung bilden,

eines von $R_{13}$ und $R_{15}$ die Bedeutung $A_1E_5$ hat und das andere Wasserstoff ist oder $R_{13}$ und $R_{15}$ miteinander eine Kette —CH=CH—CH=CH— bilden, wobei die Kette durch $A_1E_1$ substituiert ist,

A eine Einfachbindung darstellt,

$A_1$ eine Einfachbindung, Phenylen oder $(CH_2)_m$ bedeutet und

E, $E_1$, der Rest von $R_5$, $R_6$, $R_7$, $R_8$, m und die Maßgaben wie in Anspruch 1 definiert sind.

5. Verfahren zur Herstellung eines Benzopyrans nach Anspruch 1 oder 2, worin ein benachbartes Paar von $R_5$, $R_6$, $R_7$ und $R_8$ eine Kette —$CR_{10}R_{11}$—$CR_{12}R_{13}$—$CR_{14}R_{15}$—$NR_{20}$ ist, wobei die Kette durch —$A_1E_1$ substituiert ist, wobei entweder

(a) $R_{10}$ und $R_{12}$ miteinander eine Einfachbindung bilden und $R_{14}$ und $R_{20}$ miteinander eine Einfachbindung bilden oder

(b) $R_{12}$ und $R_{14}$ miteinander eine Einfachbindung bilden,

der Rest von $R_{10}$, $R_{11}$, $R_{12}$, $R_{13}$, $R_{14}$ und $R_{15}$, die gleich oder verschieden sein können, unabhängig Wasserstoff, $C_{1-6}$-Alkyl, $NHCOR_{22}$, CN oder $C_nH_xF_{(2n+1-x)}$ ist; außerdem $R_{10}$ und $R_{11}$ zusammen mit dem Kohlenstoffatom, an das sie gebunden sind, C=O oder $C=NOR_{23}$ bilden können,

A und $A_1$ jeweils ein Einfachbindung darstellen,

wenn $R_{20}$ keine Einfachbindung mit $R_{14}$ bildet, $R_{20}$ Wasserstoff, $C_{1-6}$-Alkyl oder phenylsubstituiertes $C_{1-6}$-Alkyl bedeuten kann,

n, x, $R_{21}$ und $R_{22}$ wie in Anspruch 1 definiert sind,

der Rest von $R_5$, $R_6$, $R_7$ und $R_8$, die gleich oder verschieden sein können, jeweils Wasserstoff oder Hydroxy ist,

E und $E_1$ die Bedeutung —COOH haben, ausgenommen daß, wenn die Kette —$COCR_{13}$=$CR_{15}$—$NR_{20}$— ist, in der eines von $R_{13}$ und $R_{15}$ die Bedeutung $E_1$ hat und das andere Wasserstoff oder $C_{1-6}$-Alkyl ist, dann $E_1$ Wasserstoff darstellt, und

mit der Maßgabe daß, wenn die Kette —$CR_{11}$=$CR_{13}$—$CR_{15}$=N— ist, dann mindestens eines von $R_{11}$, $R_{13}$ oder $R_{15}$, die gleich oder verschieden sein können, $NHCOR_{22}$, CN oder $C_nH_xF_{(2n+1-x)}$ bedeutet.

6. Verfahren zur Herstellung eines Benzopyrans nach Anspruch 1, worin die Verbindung der Formel (I) 10-Propyl-2,8-bis-(1H-tetrazol-5-yl)-4H,6H-benzo[1,2-b:5,4-b')]dipyran-4,6-dion oder ein pharmazeutisch annehmbares Salz hievon ist.

7. Verfahren zur Herstellung eines Benzopyrans nach Anspruch 1, worin die Verbindung der Formel (I) 4-Oxo-10-propyl-6-(trifluormethyl)-4H-pyrano[3,2-g]chinolin-2,8-dicarbonsäure oder

8-(4-Carboxyphenyl)-4,6-dioxo-10-propyl-4H,6H-benzo[1,2-b:5,4-b']dipyran-2-carbonsäure

oder ein pharmazeutisch annehmbares Salz, Ester oder Amid hievon ist.

8. Verfahren zur Herstellung eines Benzopyrans nach Anspruch 1, worin die Verbindung der Formel (I)

4,6-Dioxo-10-propyl-8-(1H-tetrazol-5-yl)-4H,6H-benzo[1,2-b:5,4-b']dipyran-2-carboxylat,

8-Aminocarbonyl-4,6-dioxo-10-propyl-4H,6H-benzo[1,2-b:5,4-b']dipyran-8-carbonsäure,

8-(N,N-Dimethylcarbonylamino)-4,6-dioxo-10-propyl-4H,6H-benzo[1,2-bis:5,4-b']dipyran-2-carbonsäure,

3-(8-Carboxy-4,6-dioxo-10-propyl-4H,6H-benzo[1,2-b:5,4-b']dipyran-2)-propansäure,

4,6-Dioxo-12-propyl-4H,6H[1]benzopyrano[3,2-g][1]benzopyran-2,8-dicarbonsäure,

6,7,8,9-Tetrahydro-4-oxo-10-propyl-4H-naphtho[2,3-b]pyran-2,7-dicarbonsäure,

6,7,8,9-Tetrahydro-5-hydroxy-4-oxo-10-propyl-4H-naphtho[2,3-b]pyran-2,7-dicarbonsäure,

6,7,8,9-Tetrahydro-5-hydroxy-4-oxo-10-propyl-4H-naphtho[2,3-b]pyran-2,8-dicarbonsäure,

7,8,9,10-Tetrahydro-1-oxo-5-propyl-1H-naphtho[2,3-b]pyran-3,8-dicarbonsäure,

6-(Heptafluorpropyl)-4-oxo-10-propyl-4H-pyrano[3,2-g]chinolin-2,8-dicarbonsäure,

4-Oxo-6-(pentafluoräthyl)-10-propyl-4H-pyrano[3,2-g]chinolin-2,8-dicarbonsäure,

6-(Difluormethyl)-4-oxo-10-propyl-4H-pyrano[3,2-g]chinolin-2,8-dicarbonsäure,

6,9-Dihydro-4,6-dioxo-10-propyl-4H,6H-pyrano[3,2-g]chinolin-2-carbonsäure,

6,9-Dihydro-4,6-dioxo-9-äthyl-10-propyl-4H,6H-pyrano[3,2-g]chinolin-2-carbonsäure,

6,9-Dihydro-4,6-dioxo-9-phenylmethyl-10-propyl-4H-pyrano[3,2-g]chinolin-2-carbonsäure,

9-Äthyl-7,9-dihydro-6-methoxyimino-4-oxo-10-propyl-4H,6H-pyrano[3,2-g]chinolin-2,8-dicarbonsäure,

6-Methoxyimino-4-oxo-10-propyl-4H,6H-benzo[1,2-b:5,4-b']dipyran-2,8-dicarbonsäure,

6-(N-Methyl-N-acetylamino)-4-oxo-10-propyl-4H-pyrano[3,2-g]chinolin-2,8-dicarbonsäure,

6-Cyano-4-oxo-10-propyl-4H-pyrano[3,2-g]chinolin-2,8-dicarbonsäure,

5-Oxo-9-propyl-5H-furo[3,2-g][1]benzopyran-2,7-dicarbonsäure,

5-Oxo-9-propyl-5H-thieno[3,2-g[1]benzopyran-2,8-dicarbonsäure,

4-Oxo-9-propyl-4H,6H-pyrano[2,3-f]indol-2,7-dicarbonsäure,

8-Oxo-4-propyl-8H-furo[2,3-g][1]benzopyran-2,6-dicarbonsäure,

6-Äthyl-4-oxo-9-propyl-4H-pyrano[2,3-f]indol-2,7-dicarbonsäure,

4-Oxo-9-propyl-4H,8H-pyran[3,2-f]indol-2,7-dicarbonsäure,

5-Oxo-9-propyl-5H-thieno[3,2-g][1]benzopyran-3,7-dicarbonsäure

oder ein pharmazeutisch annehmbares Salz, Ester oder Amid eines derselben ist.

**Revendications pour les Etats contractants: BE CH DE FR GB IT LI LU NL SE**

1. Benzopyranne de formule I,

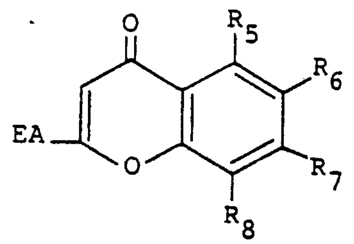

I

où une paire de radicaux adjacents parmi $R_5$, $R_6$, $R_7$ et $R_8$ représente la chaîne —X—$CR_{12}R_{13}$—$CR_{14}R_{15}$—Y— où la chaîne est substitué par —$A_1E_1$,

X représente $CR_{10}R_{11}$ ou une liaison simple,

Y représente O, S, $NR_{20}$ ou $CH_2$, et (a) $R_{14}$ et $R_{20}$ forment ensemble une liaison simple et éventuellement $R_{10}$ et $R_{12}$ forment ensemble une liaison simple, ou (b) $R_{12}$ et $R_{14}$ forment ensemble une liaison simple, ou (b) $R_{12}$ et $R_{14}$ forment ensemble une liaison simple, ou (c) $R_{10}$, $R_{12}$ et $R_{14}$ représentent chacun un atome d'hydrogène, et

ceux restants parmi $R_{10}$, $R_{11}$, $R_{12}$, $R_{13}$, $R_{14}$ et $R_{15}$, qui peuvent être identiques ou différents, représentent indépendamment des atomes d'hydrogène ou radicaux alcoyle en C1 à 6, $NR_{21}COR_{22}$, CN ou $C_nH_xF_{(2n+1-x)}$; en outre, $R_{10}$ et $R_{11}$, conjointement avec l'atome de carbone auquel ils sont unis, peuvent représenter C=O ou C=N—$OR_{23}$; $R_{13}$ et $R_{15}$ aussi peuvent former la chaîne —CH=CH—CH=CH— qui est substituée par $A_1E_1$,

A et $A_1$, qui peuvent être identiques ou différents, représentent une liaison smiple ou un radical $(CH_2)_m$ ou phénylène,

n représente un entier de 1 à 5 inclusivement,

x représente 0 ou un entier de 1 à 2n inclusivement,

m représente un entier de 1 à 4 inclusivement,

lorsque $R_{10}$ ne forme pas une liaison simple avec $R_{14}$, $R_{20}$ peut représenter un atome d'hydrogène ou radical alcoyle en C1 à 6 ou alcoyle en C1 à 6 substitué par phényle,

$R_{21}$, $R_{22}$ et $R_{23}$, qui peuvent être identiques ou différents, représentent indépendamment des atomes d'hydrogène ou radicaux alcoyle en C1 à 6;

ceux restants parmi $R_5$, $R_6$, $R_7$ et $R_8$, qui peuvent être identiques ou différents, représentent chacun indépendamment un atome d'hydrogène ou radical hydroxyle ou alcoyle en C1 à 6,.

E et $E_1$, qui peuvent être identiques ou différents, représentent indépendamment des radicaux —COOH ou 5H-tétrazolyle,

avec la restriction que lorsque la chaîne —X—$CR_{12}R_{13}$—$CR_{14}R_{15}$—Y—

i) représente une chaîne —CO—$CR_{13}$=$CR_{15}$—Y— dans laquelle Y représente O ou S et A et $A_1$ représentent chacun une liaison simple, alors Y est uni à $R_7$, $R_5$ et $R_{13}$ représentent chacun un atome d'hydrogène, $R_8$ représente un radical propyle et $R_{15}$ représente $E_1$, où $E_1$, au lieu d'être tel que défini ci-dessus, représente un radical 5H-tétrazolyle ou $CONR_{24}R_{25}$, où $R_{24}$ et $R_{25}$, qui peuvent être identiques ou différents, représentent des atomes d'hydrogène ou radicaux alcoyle en C1 à 6;

ii) représente une chaîne —CO—$CR_{13}$=$CR_{15}$—$NR_{20}$—, où l'un d'entre $R_{13}$ et $R_{15}$ représente $E_1$, alors $E_1$, au lieu d'être tel que défini ci-dessus, représente un atome d'hydrogène, et

iii) représente une chaîne —$CR_{11}$=$CR_{13}$—$CR_{15}$=N—, alors au moins l'un d'entre $R_{11}$, $R_{13}$ et $R_{15}$ qui peuvent être identiques ou différents, représente un radical $NR_{21}COR_{22}$, CN ou $C_nH_xF_{(2n+1-x)}$, et les sels pharmaceutiquement acceptables de celui-ci et lorsque l'un quelconque d'entre E et $E_1$ représente un radical acide carboxylique, les esters ou amides pharmaceutiquement acceptables des radicaux acide carboxylique.

2. Benzopyranne suivant la revendication 1, dans lequel la chaîne —X—$CR_{12}R_{13}$—$CR_{14}R_{15}$—Y— est formée entre $R_6$ et $R_7$.

3. Benzopyranne suivant la revendication 1 ou 2, dans lequel Y représente —O—.

4. Benzopyranne suivant l'une quelconque des revendications précédentes, dans lequel

une paire de radicaux parmi $R_5$, $R_6$, $R_7$ et $R_8$ représente la chaîne —CO—$CR_{12}R_{13}CR_{14}R_{15}$—O—,

$R_{12}$ et $R_{14}$ forment ensemble une liaison simple,

l'une d'entre $R_{13}$ et $R_{15}$ représente $A_1E_1$ et l'autre représente un atome d'hydrogène, ou bien $R_{13}$ et $R_{15}$ forment conjointement une chaîne —CH=CH—CH=CH—, la chaîne étant substituée par $A_1E_1$,

A représente une liaison simple,

$A_1$ représente une liaison simple ou un radical phénylène ou $(CH_2)_m$, et

E, $E_1$ et ceux restants parmi $R_5$, $R_6$, $R_7$, $R_8$, m et les restrictions sont tels que définis dans la revendication 1.

5. Benzopyranne suivant la revendication 1 ou 2, dans lequel

une paire de radicaux adjacents parmi $R_5$, $R_6$, $R_7$ et $R_8$ représente une chaîne —$CR_{10}R_{11}$—$CR_{12}R_{13}$—$CR_{14}R_{15}$—$NR_{20}$—, où la chaîne est substituée par —$A_1E_1$,

(a) $R_{10}$ et $R_{12}$ forment ensemble une liaison simple et $R_{14}$ et $R_{20}$ forment ensemble une liaison simple, ou bien

(b) $R_{12}$ et $R_{14}$ forment ensemble une liaison simple, ceux restants parmi $R_{10}$, $R_{11}$, $R_{12}$, $R_{13}$, $R_{14}$ et $R_{15}$, qui peuvent être identiques ou différents, représentent indépendamment des atomes d'hydrogène ou radicaux alcoyle en C1 à 6, $NHCOR_{22}$, CN ou $C_nH_xF_{(2n+1-x)}$; en outre, $R_{10}$ et $R_{11}$, conjointement avec l'atome de carbone auxquels ils sont unis, peuvent représenter un radical C=O ou $C{=}NOR_{23}$,

A et $A_1$ représentent chacun une liaison simple,

lorsque $R_{20}$ ne forme pas une liaison simple avec $R_{14}$, $R_{20}$ peut représenter un atome d'hydrogène ou radical alcoyle en C1 à 6 ou alcoyle en C1 à 6 substitué par phényle,

n, x, $R_{21}$ et $R_{22}$ sont tels que définis dans la revendication 1,

ceux restants parmi $R_5$, $R_6$, $R_7$ et $R_8$, qui peuvent être identiques ou différents, représentent chacun indépendamment un atome d'hydrogène ou radical hydroxyle,

E et $E_1$ représetent —COOH, sauf que lorsque la chaîne représente $—COCR_{13}{=}CR_{15}—NR_{20}—$, où l'une d'entre $R_{13}$ et $R_{15}$ représente $E_1$ et l'autre représente un atome d'hydrogènew ou racical alcoyle en C1 à 6, alors $E_1$ représente un atome d'hydrogène, et

avec le restriction que lorsque la chaîne représente $—CR_{11}{=}CR_{13}—CR_{15}{=}N—$, alors au moins l'un d'entre $R_{11}$, $R_{13}$ et $R_{15}$, qui peuvent être identiques ou différents, représente un radical $NHCOR_{22}$, CN ou $C_nH_xF_{(2n+1-x)}$.

6. Benzopyranne suivant la revendication 1, qui est
la 10-propyl-2,8-bis-(1H-tétrazol-5-yl)-4H,6H-benzo[1,2-b:5,4-b']dipyranne-4,6-dione
ou un sel pharmaceutiquement acceptable de celle-ci.

7. Benzopyranne de formule I tel que défini dans la revendication 1, qui est
l'acide 4-oxo-10-propyl-6-(trifluorométhyl)-4H-pyrano[3,2-g]quinoléine-2,8-dicarboxylique ou
l'acide 8-(4-carboxyphényl)-4,6-dioxo-10-propyl-4H,6H-benzo-[1,2-b(5,4-b']dipyranne-2-carboxylique
ou un sel, ester ou amide pharmaceutique acceptable de l'un de ceux-ci.

8. Benzopyranne de formule I tel que défini dans la revendication 1, qui est
l'acide 4,6-dioxo-10-propyl-6-(1H-tétrazol-5-yl)-4H,6H-benzo-[1,2-b:5,4-b']dipyranne-2-carboxylique
l'acide 8-aminocarbonyl-4,5-dioxo-10-propyl-4H,6H-benzo[1,2-b:5,4-b']dipyranne-8-carboxylique
l'acide 8-(N,N-diméthylcarbonylamino)-4,6-dioxo-10-propyl-4H,6H-benzo[1,2-b:5,4-b']dipyranne-2-carboxylique
l'acide 3-(8-carboxy-4,6-dioxo-10-propyl-4H,6H-benzo[1,2-b:5,4-b']dipyranne-2-)-propanoïque
l'acide 4,6-dioxo-12-propyl-4H,6H-[1]-benzopyranno[3,2-g][1]-benzopyranne-2,8-dicarboxylique
l'acide 6,7,8,9-tétrahydro-4-oxo-10-propyl-4H-naphto[2,3-b]pyranne-2,7-dicarboxylique
l'acide 6,7,8,9-tétrahydro-5-hydroxy-4-oxo-10-propyl-4H-naphto-[2,3-b]pyranne-2,7-dicarboxylique
l'acide 6,7,8,9-tétrahydro-5-hydroxy-4-oxo-10-propyl-4H-naphto-[2,3-b]pyranne-2,8-dicarboxylique
l'acide 7,8,9,10-tétrahydro-1-oxo-5-propyl-1H-naphto[2,1-b]pyranne-3,8-dicarboxylique
l'acide 6-(heptafluoropropyl)-4-oxo-10-propyl-4H-pyranno[3,2-g]quinoléine-2,8-dicarboxylique
l'acide 4-oxo-6-(pentafluoroéthyl)-10-propyl-4H-pyranno[3,2-g]quinoléine-2,8-dicarboxylique
l'acide 6-(difluorométhyl)-4-oxo-10-propyl-4H-pyranno[3,2-g]quinoléine-28-dicarboxylique
l'acide 6,9-dihydro-4,6-dioxo-10-propyl-4H,6H-pyranno[3,2-g]quinoléine-2-carboxylique
l'acide 6,9-dihydro-4,6-dioxo-9-éthyl-10-propyl-4H,6H-pyranno-[3,2-g]quinoléine-2-carboxylique
l'acide 6,9-dihydro-4,6-9-phénylméthyl-10-propyl-4H-pyranno-[3,2-g]quinoléine-2-carboxylique
l'acide 9-éthyl-7,9-dihydro-6-méthoxyimino-4-oxo-10-propyl-4H,6H-pyranno-[3,2-g]quinoléine-2,8-dicarboxylique
l'acide 6-méthoxyimino-4-oxo-10-propyl-4H,6H-benzo[1,2-b:5,4-b']dipyranne-2,8-dicarboxylique
l'acide 6-(N-méthyl-N-acéthylamino)-4-oxo-10-propyl-4H-pyranno-[3,2-q]quinoléine-2,8-dicarboxylique
l'acide 6-cyano-4-oxo-10-propyl-4H-pyranno-[3,2-g]quinoléine-2,8-dicarboxylique
l'acide 5-oxo-9-propyl-5H-furo-[3,2-g[1]-benzopyranne-2,7-dicarboxylique
l'acide 5-oxo-9-propyl-5H-thiéno-[3,2-g[1]-benzopyranne-2,7-dicarboxylique
l'acide 4-oxo-9-propyl-4H,6H-pyranno-[2,3-f]indole-2,7-dicarboxylique
l'acide 8-oxo-4-propyl-8H-furo-[2,3-g][1]benzopyranne-2,6-dicarboxylique
l'acide 6-éthyl-4-oxo-9-propyl-4H-pyranno-[2,3-f]indole-2,7-dicarboxylique
l'acide 4-oxo-9-propyl-4H,8H-pyranno-[3,2-f]indole-2,7-dicarboxylique
l'acide 5-oxo-9-propyl-5H-thiéno-[3,2-g][1]benzopyranne-3,7-dicarboxylique
ou un sel, ester ou amide pharmaceutiquement acceptable de l'un quelconque de ceux-ci.

9. Composition pharmaceutique, qui comprend un composé suivant l'une quelconque des revendications précédentes, en mélange avec un adjuvant, diluant ou excipient pharmaceutiquement acceptable.

10. Procédé de préparation d'un benzopyranne suivant l'une quelconque des revendications 1 à 8, qui comprend

(a) la cyclisation d'un composé de formule II ou d'un dérivé approprié de cellui-ci,

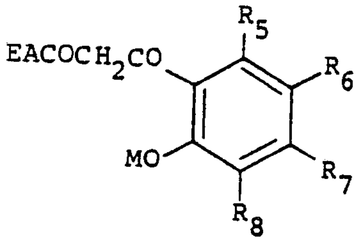

II

où M représente un atome d'hydrogène ou un métal alcalin,

et E, A, $R_5$, $R_6$, $R_7$ et $R_8$ sont tels que définis dans la revendication 1,

(b) la préparation d'un composé de formule I, où au moins l'un d'entre E et $E_1$ représente un radical —COOH, par hydrolyse d'un composé de formule I où le E ou $E_1$ correspondant représente un radical hydrolysable en un radical —COOH,

(c) la préparation d'un composé de formule I, où au moins l'une d'entre E et E 07 représente un radical 5-tétrazolyle, par réaction d'un composé de formule I, où le E ou $E_1$ correspondant représente —CN, avec un azide dans un solvant qui est inerte dans les conditions de réaction,

(d) la préparation d'un composé de formule I, où $E_1$ représente un radical $—CONR_{24}R_{25}$, par réaction d'un composé de formule I où $E_1$ représente un radical —COL, où L est un bon radical partant, avec un composé de formule III,

$$R_{24}R_{25}NH \qquad III$$

où $R_{24}$ et $R_{25}$ sont tels que définis dans la revendication 1, ou

(e) la préparation d'un composé de formule I, où au moins l'une d'entre $R_{10}$, $R_{11}$, $R_{12}$, $R_{13}$, $R_{14}$ et $R_{15}$ représente $C_nH_xF_{(2n+1-x)}$, par réaction d'un composé de formule I ou d'un dérivé de celui-ci, où le $R_{10}$, $R_{11}$, $R_{12}$, $R_{13}$, $R_{14}$ ou $R_{15}$ correspondant représente $L_1$, où $L_1$ est un atome d'halogène, avec un composé

$$C_nH_xF_{(2n+1-x)}L_2$$

où n et x sont tels que définis dans la revendication 1 et $L_2$ représente le chlore, le brome ou l'iode, et si la chose est nécessaire ou souhaitée, la conversion du composé de formule I en un sel pharmaceutiquement acceptable de celui-ci ou, lorsque l'une quelconque d'entre E et $E_1$ représente un radical acide carboxylique, en les esters ou amides pharmaceutiquement acceptables des radicaux acide carboxylique, ou réciproquement.

11. Utilisation d'un composé suivant l'une quelconque des revendications 1 à 8 pour la préparation d'une composition pharmaceutique en vue du traitement de l'asthme.

**Revendications pour l'Etats Contractants: AT**

1. Procédé de préparation d'un benzoyranne de formule I

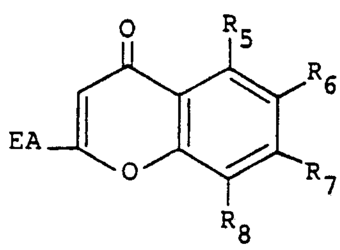

I

où une paire de radicaux adjacents parmi $R_5$, $R_6$, $R_7$ et $R_8$ représente la chaîne $—X—CR_{12}R_{13}—CR_{14}R_{15}—Y—$ où la chaîne est substitué par $—A_1E_1$,

X représente $CR_{10}R_{11}$ ou une liaison simple,

Y représente O, S, $NR_{20}$ ou $CH_2$, et (a) $R_{14}$ et $R_{20}$ forment ensemble une liaison simple et éventuellement $R_{10}$ et $R_{12}$ forment ensemble une liaison simple, ou (b) $R_{12}$ et $R_{14}$ forment ensemble une liaison simple et éventuellement $R_{10}$ et $R_{12}$ forment ensemble une liaison simple, ou (b) $R_{12}$ et $R_{14}$ forment ensemble une liaison simple, ou (c) $R_{10}$, $R_{12}$ et $R_{14}$ représentent chacun un atome d'hydrogène, et

ceux restants parmi $R_{10}$, $R_{11}$, $R_{12}$, $R_{13}$, $R_{14}$ et $R_{15}$, qui peuvent être identiques ou différents, représentent indépendamment des atomes d'hydrogène ou radicaux alcoyle en C1 à 6, $NR_{21}COR_{22}$, CN ou $C_nH_xF_{(2n+1-x)}$; en outre, $R_{10}$ et $R_{11}$, conjointement avec l'atome de carbone auquel ils sont unis, peuvent représenter C=O ou $C=N—OR_{23}$; $R_{13}$ et $R_{15}$ aussi peuvent former la chaîne —CH=CH—CH=CH— qui est substituée par $A_1E_1$,

A et $A_1$, qui peuvent être identiques ou différents, représentent une liaison simple ou un radical $(CH_2)_m$ ou phénylène,

n représente un entier de 1 à 5 inclusivement,

x représente 0 ou un entier de 1 à 2n inclusivement,

m représente un entier de 1 à 4 inclusivement,

lorsque $R_{10}$ ne forme pas une liaison simple avec $R_{14}$, $R_{20}$ peut représenter un atome d'hydrogène ou radical alcoyle en C1 à 6 ou alcoyle en C1 à 6 substitué par phényle,

$R_{21}$, $R_{22}$ et $R_{23}$, qui peuvent être identiques ou différents, représentent indépendamment des atomes d'hydrogène ou radicaux alcoyle en C1 à 6;

ceux restants parmi $R_5$, $R_6$, $R_7$ et $R_8$, qui peuvent être identiques ou différents, représentent chacun indépendamment un atome d'hydrogène ou radical hydroxyle ou alcoyle en C1 à 6,.

E et $E_1$, qui peuvent être identiques ou différents, représentent indépendamment des radicaux —COOH ou 5H-tétrazolyle,

avec la restriction que lorsque la chaîne $—X—CR_{12}R_{13}—CR_{14}R_{15}—Y—$

i) représente une chaîne $—CO—CR_{13}{=}CR_{15}—Y—$ dans laquelle Y représente O ou S et A et $A_1$ représentent chacun une liaison simple, alors Y est uni à $R_7$, $R_5$ et $R_{13}$ représentent chacun un atome d'hydrogène, $R_8$ représente un radical propyle et $R_{15}$ représente $E_1$, où $E_1$, au lieu d'être tel que défini ci-dessus, représente un radical 5H-tétrazolyle ou $CONR_{24}R_{25}$, où $R_{24}$ et $R_{25}$, qui peuvent être identiques ou différents, représentent des atomes d'hydrogène ou radicaux alcoyle en C1 à 6;

ii) représente une chaîne $—CO—CR_{13}{=}CR_{15}—NR_{20}—$, où l'un d'entre $R_{13}$ et $R_{15}$ représente $E_1$, alors $E_1$, au lieu d'être tel que défini ci-dessus, représente un atome d'hydrogène, et

iii) représente une chaîne $—CR_{11}{=}CR_{13}—CR_{15}{=}N—$, alors au moins l'un d'entre $R_{11}$, $R_{13}$ et $R_{15}$ qui peuvent être identiques ou différents, représente un radical $NR_{21}COR_{22}$, CN ou $C_nH_xF_{(2n+1-x)}$, et des sels pharmaceutiquement acceptables de celui-ci et lorsque l'un quelconque d'entre E et $E_1$ représente un radical acide carboxylique, les esters ou amides pharmaceutiquement acceptables des radicaux acide carboxylique.

qui comprend

(a) la cyclisation d'un composé de formule II ou d'un dérivé approprié de celui-ci,

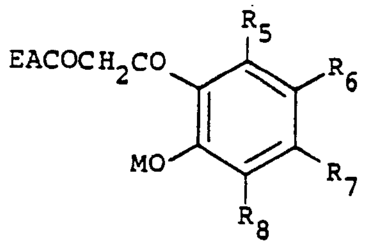

II

où M représente un atome d'hydrogène ou un métal alcalin,

et E, A, $R_5$, $R_6$, $R_7$ et $R_8$ sont tels que définis ci-dessus,

(b) la préparation d'un composé de formule I, où au moins l'un d'entre E et $E_1$ représente un radical —COOH, par hydrolyse d'un composé de formule I où le E ou $E_1$ correspondant représente un radical hydrolysable en un radical —COOH,

(c) la préparation d'un composé de formule I, où au moins l'une d'entre E et E 07 représente un radical 5-tétrazolyle, par réaction d'un composé de formule I, où le E ou $E_1$ correspondant représente —CN, avec un azide dans un solvant qui est inerte dans les conditions de réaction,

(d) la préparation d'un composé de formule I, où $E_1$ représente un radical $—CONR_{24}R_{25}$, par réaction d'un composé de formule I où $E_1$ représente un radical —COL, où L est un bon radical partant, avec un composé de formule III,

$$R_{24}R_{25}NH \qquad III$$

où $R_{24}$ et $R_{25}$ sont tels que définis ci-dessus, ou

(e) la préparation d'un composé de formule I, où au moins l'une d'entre $R_{10}$, $R_{11}$, $R_{12}$, $R_{13}$, $R_{14}$ et $R_{15}$ représente $C_nH_xF_{(2n+1-x)}$, par réaction d'un composé de formule I ou d'un dérivé de celui-ci, où le $R_{10}$, $R_{11}$, $R_{12}$, $R_{13}$, $R_{14}$ ou $R_{15}$ correspondant représente $L_1$, où $L_1$ est un atome d'halogène, avec un composé

$$C_nH_xF_{(2n+1-x)}L_2$$

où n et x sont tels que définis dans ci-dessus 1 et $L_2$ représente le chlore, le brome ou l'iode, et si la chose est nécessaire ou souhaitée, la conversion du composé de formule I en un sel pharmaceutiquement acceptable de celui-ci ou, lorsque l'un quelconque d'entre E et $E_1$ représente un radical acide carboxylique, en les esters ou amides pharmaceutiquement acceptables des radicaux acide carboxylique, ou réciproquement.

2. Procédé de préparation d'un benzopyranne suivant la revendication 1, dans lequel la chaîne $—X—CR_{12}R_{13}—CR_{14}R_{15}—Y—$ est formée entre $R_6$ et $R_7$.

3. Procédé de préparation d'un benzopyranne suivant la revendication 1 ou 2, dans lequel Y représente —O—.

4. Procédé de préparation d'un benzopyranne suivant l'une quelconque des revendications précédentes, dans lequel

une paire de radicaux parmi $R_5$, $R_6$, $R_7$ et $R_8$ représente la chaîne $—CO—CR_{12}R_{13}CR_{14}R_{15}—O—$,

$R_{12}$ et $R_{14}$ forment ensemble une liaison simple,

l'une d'entre $R_{13}$ et $R_{15}$ représente $A_1E_1$ et l'autre représente un atome d'hydrogène, ou bien $R_{13}$ et $R_{15}$ forment conjointement une chaîne —CH=CH—CH=CH—, la chaîne étant substituée par $A_1E_1$,

A représente une liaison simple,

$A_1$ représente une liaison simple ou un radical phénylène ou $(CH_2)_m$, et

E, $E_1$ et ceux restants parmi $R_5$, $R_6$, $R_7$, $R_8$, m et les restrictions sont tels que définis dans la revendication 1.

5. Procédé de préparation d'un benzopyranne suivant la revendication 1 ou 2, dans lequel

une paire de radicaux adjacents parmi $R_5$, $R_6$, $R_7$ et $R_8$ représente une chaîne $—CR_{10}R_{11}—CR_{12}R_{13}—CR_{14}R_{15}—NR_{20}—$, où la chaîne est substituée par $—A_1E_1$,

(a) $R_{10}$ et $R_{12}$ forment ensemble une liaison simple et $R_{14}$ et $R_{20}$ forment ensemble une liaison simple, ou bien

(b) $R_{12}$ et $R_{14}$ forment ensemble une liaison simple, ceux restants parmi $R_{10}$, $R_{11}$, $R_{12}$, $R_{13}$, $R_{14}$ et $R_{15}$, qui peuvent être identiques ou différents, représentent indépendamment des atomes d'hydrogène ou radicaux alcoyle en C1 à 6, $NHCOR_{22}$, CN ou $C_nH_xF_{(2n+1-x)}$; en outre, $R_{10}$ et $R_{11}$, conjointement avec l'atome de carbone auxquels ils sont unis, peuvent représenter un radical C=O ou $C=NOR_{23}$,

A et $A_1$ représentent chacun une liaison simple,

lorsque $R_{20}$ ne forme pas une liaison simple avec $R_{14}$, $R_{20}$ peut représenter un atome d'hydrogène ou radical alcoyle en C1 à 6 ou alcoyle en C1 à 6 substitué par phényle,

n, x, $R_{21}$ et $R_{22}$ sont tels que définis dans la revendication 1,

ceux restants parmi $R_5$, $R_6$, $R_7$ et $R_8$, qui peuvent être identiques ou différents, représentent chacun indépendamment un atome d'hydrogène ou radical hydroxyle,

E et $E_1$ représentent —COOH, sauf que lorsque la chaîne représente $—COCR_{13}=CR_{15}—NR_{20}—$, où l'une d'entre $R_{13}$ et $R_{15}$ représente $E_1$ et l'autre représente un atome d'hydrogène ou radical alcoyle en C1 à 6, alors $E_1$ représente un atome d'hydrogène, et

avec le restriction que lorsque la chaîne représente $—CR_{11}=CR_{13}—CR_{15}=N—$, alors au moins l'un d'entre $R_{11}$, $R_{13}$ et $R_{15}$, qui peuvent être identiques ou différents, représente un radical $NHCOR_{22}$, CN ou $C_nH_xF_{(2n+1-x)}$.

6. Procédé de preparation d'un benzopyranne suivant la revendication 1, dans lequel le composé de formule I est

la 10-propyl-2,8-bis-(1H-tétrazol-5-yl)-4H,6H-benzo[1,2-b:5,4-b']dipyranne-4,6-dione

ou un sel pharmaceutiquement acceptable de celle-ci.

7. Procédé de préparation d'un benzopyranne suivant la revendication 1, dans lequelle composé de formule I est

l'acide 4-oxo-10-propyl-6-(trifluorométhyl)-4H-pyranno[3,2-g]quinoléine-2,8-dicarboxylique ou

l'acide 8-(4-carboxyphényl)-4,6-dioxo-10-propyl-4H,6H-benzo-[1,2-b(5,4-b']dipyranne-2-carboxylique

ou un sel, ester ou amide pharmaceutique acceptable de l'un de ceux-ci.

8. Procédé de préparation d'un benzopyranne suivant la revendication 1, dans lequelle composé de formule I est

l'acide 4,6-dioxo-10-propyl-6-(1H-tétrazol-5-yl)-4H,6H-benzo-[1,2-b:5,4-b']dipyranne-2-carboxylique

l'acide 8-aminocarbonyl-4,5-dioxo-10-propyl-4H,6H-benzo[1,2-b:5,4-b']dipyranne-8-carboxylique

l'acide 8-(N,N-diméthylcarbonylamino)-4,6-dioxo-10-propyl-4H,6H-benzo[1,2-b:5,4-b']dipyranne-2-carboxylique

l'acide 3-(8-carboxy-4,6-dioxo-10-propyl-4H,6H-benzo[1,2-b:5,4-b']dipyranne-2-)-propanoïque

l'acide 4,6-dioxo-12-propyl-4H,6H-[1]-benzopyranno[3,2-g][1]-benzopyranne-2,8-dicarboxylique

l'acide 6,7,8,9-tétrahydro-4-oxo-10-propyl-4H-naphto[2,3-b]pyranne-2,7-dicarboxylique

l'acide 6,7,8,9-tétrahydro-5-hydroxy-4-oxo-10-propyl-4H-naphto-[2,3-b]pyranne-2,7-dicarboxylique

l'acide 6,7,8,9-tétrahydro-5-hydroxy-4-oxo-10-propyl-4H-naphto-[2,3-b]pyranne-2,8-dicarboxylique

l'acide 7,8,9,10-tétrahydro-1-oxo-5-propyl-1H-naphto[2,1-b]pyranne-3,8-dicarboxylique

l'acide 6-(heptafluoropropyl)-4-oxo-10-propyl-4H-pyranno[3,2-g]quinoléine-2,8-dicarboxylique

l'acide 4-oxo-6-(pentafluoroéthyl)-10-propyl-4H-pyranno[3,2-g]quinoléine-2,8-dicarboxylique

l'acide 6-(difluorométhyl)-4-oxo-10-propyl-4H-pyranno[3,2-g]quinoléine-28-dicarboxylique

l'acide 6,9-dihydro-4,6-dioxo-10-propyl-4H,6H-pyranno[3,2-g]quinoléine-2-carboxylique

l'acide 6,9-dihydro-4,6-dioxo-9-éthyl-10-propyl-4H,6H-pyranno-[3,2-g]quinoléine-2-carboxylique

l'acide 6,9-dihydro-4,6-9-phénylméthyl-10-propyl-4H-pyranno-[3,2-g]quinoléine-2-carboxylique

l'acide 9-éthyl-7,9-dihydro-6-méthoxyimino-4-oxo-10-propyl-4H,6H-pyranno-[3,2-g]quinoléine-2,8-dicarboxylique

l'acide 6-méthoxyimino-4-oxo-10-propyl-4H,6H-benzo[1,2-b:5,4-b']dipyranne-2,8-dicarboxylique

l'acide 6-(N-méthyl-N-acéthylamino)-4-oxo-10-propyl-4H-pyranno-[3,2-q]quinoléine-2,8-dicarboxylique

l'acide 6-cyano-4-oxo-10-propyl-4H-pyranno-[3,2-g]quinoléine-2,8-dicarboxylique

l'acide 5-oxo-9-propyl-5H-furo-[3,2-g[1]-benzopyranne-2,7-dicarboxylique
l'acide 5-oxo-9-propyl-5H-thiéno-[3,2-g[1]-benzopyranne-2,7-dicarboxylique
l'acide 4-oxo-9-propyl-4H,6H-pyranno-[2,3-f]indole-2,7-dicarboxylique
l'acide 8-oxo-4-propyl-8H-furo-[2,3-g][1]benzopyranne-2,6-dicarboxylique
l'acide 6-éthyl-4-oxo-9-propyl-4H-pyranno-[2,3-f]indole-2,7-dicarboxylique
l'acide 4-oxo-9-propyl-4H,8H-pyranno-[3,2-f]indole-2,7-dicarboxylique
l'acide 5-oxo-9-propyl-5H-thiéno-[3,2-g][1]benzopyranne-3,7-dicarboxylique
ou un sel, ester ou amide pharmaceutiquement acceptable de l'un quelconque de ceux-ci.